(19) Europäisches Patentamt / European Patent Office / Office européen des brevets

(11) **EP 2 253 633 A1**

(12) **EUROPEAN PATENT APPLICATION**
published in accordance with Art. 153(4) EPC

(43) Date of publication:
**24.11.2010 Bulletin 2010/47**

(21) Application number: **09719847.7**

(22) Date of filing: **11.03.2009**

(51) Int Cl.:
***C07D 487/04*** *(2006.01)* ***A61K 31/519*** *(2006.01)*
***A61P 9/00*** *(2006.01)* ***A61P 9/10*** *(2006.01)*
***A61P 31/12*** *(2006.01)* ***A61P 31/18*** *(2006.01)*
***A61P 35/00*** *(2006.01)* ***A61P 35/02*** *(2006.01)*
***A61P 43/00*** *(2006.01)* ***C07D 487/16*** *(2006.01)*

(86) International application number:
**PCT/JP2009/054603**

(87) International publication number:
**WO 2009/113560 (17.09.2009 Gazette 2009/38)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **12.03.2008 JP 2008063363**

(71) Applicant: **Takeda Pharmaceutical Company Limited**
**Osaka 541-0045 (JP)**

(72) Inventors:
- **ISHIKAWA, Tomoyasu**
  **Tsukuba-shi**
  **Ibaraki 300-4293 (JP)**
- **KAWAKITA, Youichi**
  **Tsukuba-shi**
  **Ibaraki 300-4293 (JP)**

(74) Representative: **Held, Stephan**
**Meissner, Bolte & Partner GbR**
**Widenmayerstraße 48**
**D-80538 München (DE)**

(54) **FUSED HETEROCYCLIC COMPOUND**

(57) The present invention provides a fused heterocyclic compound having a tyrosine kinase inhibitory action, which is represented by the formula:

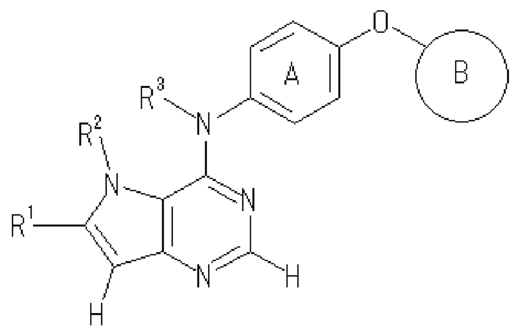

wherein
ring A is an optionally substituted benzene ring;
ring B is an optionally substituted benzoisothiazole ring;
$R^1$ is a hydrogen atom, a halogen atom, or an optionally substituted group bonded via a carbon atom, a nitrogen atom or an oxygen atom;
$R^2$ is a hydrogen atom, or an optionally substituted group bonded via a carbon atom or a sulfur atom;
$R^3$ is a hydrogen atom or an optionally substituted aliphatic hydrocarbon group;
or
$R^1$ and $R^2$, or $R^2$ and $R^3$ are optionally bonded to each other to form an optionally substituted ring structure;
or
$R^3$ is optionally bonded to the carbon atom on ring A to form an optionally substituted ring structure;
or a salt thereof.

EP 2 253 633 A1

## Description

### Technical Field

The present invention relates to a fused pyrimidine compound having a growth factor receptor tyrosine kinase inhibitory activity, which is useful for the prophylaxis or treatment of cancer, a production method thereof and use thereof.

### Background of the Invention

The gene of cell growth factor and growth factor receptor is called a protooncogene and plays a key role in the pathology of human tumor. The epithelial cell growth factor receptor family (erbB) includes EGFR, HER2, HER3 and HER4, which are type I receptor type tyrosine kinases. These erbB family express in various cell groups, and are deeply involved in the control of the growth and differentiation of cells and the control of suppression of cell death (apoptosis suppression). For example, high expression of EGFR and HER2, and homeostatic activation of receptors are empirically known to transform cells.

It is also known that high expression and simultaneous expression of each of these receptors are poor prognostic factors in various cancer patients.

These receptors are bound with many peptide ligands such as EGF, TGFα and the like, and binding of the ligand promotes homo- or heterodimerization of the receptors. This induces increase of kinase activity from self-phosphorylation or transphosphorylation of the receptors, and causes activation of downstream signaling pathway (MAPK, Akt) via a protein bound with a particular phosphorylated tyrosine residue. This is the mechanism of the receptor activity of the above-mentioned cell proliferation, differentiation, cell death suppression and the like, which is considered to be responsible for the high expression of receptor in cancer and malignant degeneration of cancer due to topical increase in the ligand concentration.

Many cancers are associated with the high expression of EGFR or HER2. For example, breast cancer (20-30%), ovarian cancer (20-40%), non-small cell lung cancer (30-60%), colorectal cancer (40-80%), prostate cancer (10-60%), urinary bladder cancer (30-60%), kidney cancer (20-40%) and the like can be mentioned. Moreover, receptor expression and prognosis are correlated, and receptor expression is a poor prognostic factor in breast cancer, non-small cell lung cancer and the like.

In recent years, clinical use of a humanized anti-HER2 antibody (Trastuzumab) against HER2 highly expressing breast cancer, clinical trial of anti-EGFR antibody and clinical trials of several low molecular weight receptor enzyme inhibitors have demonstrated a potential of these drugs against HER2 or EGFR for therapeutic drugs for cancer. While these drugs show a tumor growth inhibitory action in clinical and non-clinical trials, they are known to induce inhibition of receptor enzyme activity and suppression of downstream signaling pathway. Therefore, a compound inhibiting EGFR or HER2 kinase, or inhibiting activation of EGFR or HER2 kinase is effective as a therapeutic drug for cancer.

As a compound that inhibits receptor type tyrosine kinases represented by HER2/EGFR kinase, fused heterocyclic compounds (e.g., patent reference 1 (WO97/13771), patent reference 2 (WO98/02437), and patent reference 3 (WO00/44728)), quinazoline derivatives (e.g., patent reference 4 (WO02/02552), patent reference 5 (WO01/98277), patent reference 6 (WO03/049740) and patent reference 7 (WO03/050108)), thienopyrimidine derivatives (e.g., patent reference (WO03/053446)), aromatic azole derivatives (e.g., patent reference 9 (WO98/03648), patent reference 10 (WO01/77107) and patent reference 11 (WO03/031442)), condensed pyrimidine derivatives (e.g., patent reference 12 (WO2005/118588)) and the like are known.

As to pyrrolo[3,2-d]pyrimidine derivatives, the following compounds are known as compounds having a cell proliferation inhibitory activity (non-patent reference 1 (Khim.-Farm. Zh., 1982, 16, 1338-1343); non-patent reference 2 (Collect. Czech. Chem. Commun., 2003, 68, 779-791)).

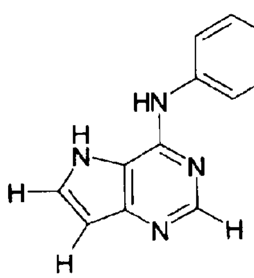

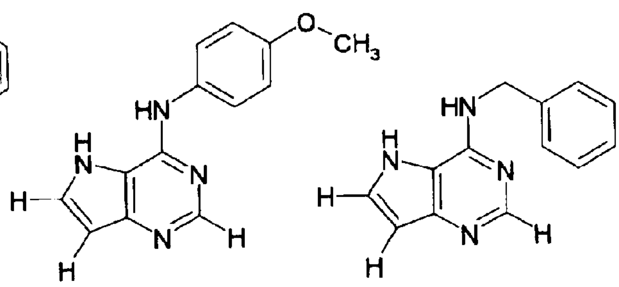

As a compound having a receptor type tyrosine kinase inhibitory activity, the following pyrrolo[3,2-d]pyrimidine derivative is known (patent reference 13 (WO96/40142) and patent reference 14 (WO98/23613)).
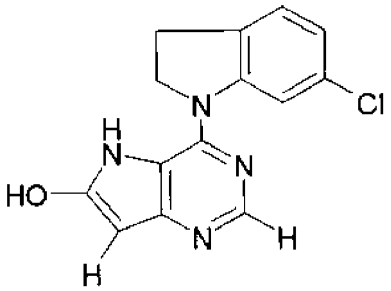

[patent reference 1] WO97/13771
[patent reference 2] WO98/02437
[patent reference 3] WO00/44728
[patent reference 4] WO02/02552
[patent reference 5] WO01/98277
[patent reference 6] WO03/049740
[patent reference 7] WO03/050108
[patent reference 8] WO03/053446
[patent reference 9] WO98/03648
[patent reference 10] WO01/77107
[patent reference 11] WO03/031442
[patent reference 12] WO2005/118588
[patent reference 13] WO96/40142
[patent reference 14] WO98/23613
[non-patent reference 1] Khim. -Farm.Zh., 1982, 16, 1338-1343
[non-patent reference 2] Collect. Czech. Chem. Commun., 2003, 68, 779-791

**Disclosure of the Invention**

**Problems to be Solved by the Invention**

The present invention aims at providing a compound having a superior tyrosine kinase inhibitory action, which is highly safe and sufficiently satisfactory as a pharmaceutical product.

**Means of Solving the Problems**

The present inventors have conducted intensive studies in an attempt to solve the aforementioned problems and found that the compounds represented by the following formula (I) and salts thereof (hereinafter to be sometimes abbreviated as compound (I)) have a superior tyrosine kinase inhibitory action. Further studies have resulted in the completion of the present invention.
Accordingly, the present invention relates to

[1] a compound represented by the formula:

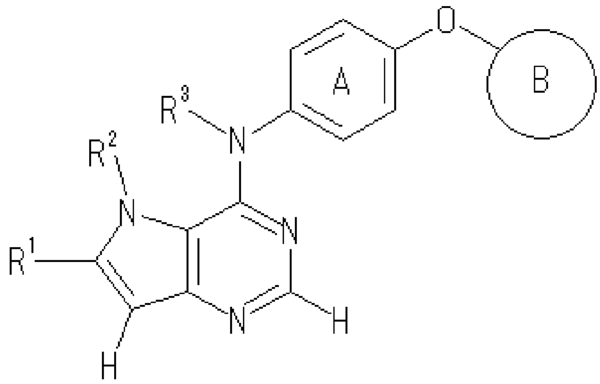

(I)

wherein

ring A is an optionally substituted benzene ring;
ring B is an optionally substituted benzoisothiazole ring;
$R^1$ is a hydrogen atom, a halogen atom, or an optionally substituted group bonded via a carbon atom, a nitrogen atom or an oxygen atom;
$R^2$ is a hydrogen atom, or an optionally substituted group bonded via a carbon atom or a sulfur atom;
$R^3$ is a hydrogen atom or an optionally substituted aliphatic hydrocarbon group; or
$R^1$ and $R^2$, or $R^2$ and $R^3$ are optionally bonded to each other to form an optionally substituted ring structure; or
$R^3$ is optionally bonded to the carbon atom on ring A to form an optionally substituted ring structure;
or a salt thereof;
[2] the compound of the above-mentioned [1], wherein $R^1$ is a hydrogen atom or a halogen atom;
[3] the compound of the above-mentioned [1], wherein $R^2$ is a hydrogen atom or an optionally substituted alkyl group;
[4] the compound of the above-mentioned [1], wherein $R^3$ is a hydrogen atom;
[5] the compound of the above-mentioned [1], wherein ring A is a benzene ring optionally substituted by 1 or 2 substituents selected from the group consisting of (1) a halogen atom, (2) $C_{1-4}$ alkyl optionally having 1 to 3 halogen atoms and (3) $C_{1-4}$ alkoxy;
[6] the compound of the above-mentioned [1], wherein ring B is a benzoisothiazole ring optionally having one $C_{1-6}$ alkyl;
[7] the compound of the above-mentioned [1], wherein $R^1$ is a hydrogen atom or a chlorine atom;
$R^2$ is a $C_{1-6}$ alkyl group optionally having one substituent selected from

(1) hydroxy,
(2) $C_{1-6}$ alkyl-carbonylamino optionally having 1 to 3 substituents selected from (1') halogen atom, (2') amino, (3') $C_{1-6}$ alkylamino, (4') hydroxy and (5') $C_{1-6}$ alkylsulfonyl,
(3) $C_{3-6}$ cycloalkyl-carbonylamino optionally having one substituent selected from (1') cyano, (2') amino and (3') hydroxy,
(4) $C_{2-6}$ alkynyl-carbonylamino,
(5) heterocyclyl-carbonylamino,
(6) $C_{1-6}$ alkyl-aminocarbonyl-amino,
(7) $C_{1-6}$ alkyl-carbonyloxy optionally having one carboxy and
(8) $C_{1-6}$ alkylsulfonyl;

$R^3$ is a hydrogen atom;
ring A is a benzene ring optionally substituted by 1 or 2 substituents selected from the group consisting of a fluorine atom, a chlorine atom, a bromine atom, methyl, trifluoromethyl and methoxy; and
ring B is a benzoisothiazole ring optionally having one methyl, which is bonded to oxygen atom at the 4- or 6-position;
[8] 2-(4-{[4-(1,2-benzoisothiazol-4-yloxy)-3-chlorophenyl]amino}-5H-pyrrolo[3,2-d]pyrimidin-5-yl)ethanol or a salt thereof;
[9] N-[2-(4-{[4-(1,2-benzoisothiazol-4-yloxy)-3-chlorophenyl]amino}-5H-pyrrolo[3,2-d]pyrimidin-5-yl)ethyl]acetamide or a salt thereof;
[10] N-[2-(4-{[4-(1,2-benzoisothiazol-4-yloxy)-3-chlorophenyl]amino}-5H-pyrrolo[3,2-d]pyrimidin-5-yl)ethyl]pyrrolidine-3-carboxamide or a salt thereof;

[11] N-[2-(4-{[4-(1,2-benzoisothiazol-4-yloxy)-3-methylphenyl]amino}-5H-pyrrolo[3,2-d]pyrimidin-5-yl)ethyl]-2,2-dimethylpropanamide or a salt thereof;
[12] N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-2,2-dimethylpropanamide or a salt thereof;
[13] 1-[2-(4-{[4-(1,2-benzoisothiazol-4-yloxy)-3-chlorophenyl]amino}-5H-pyrrolo[3,2-d]pyrimidin-5-yl)ethyl]-3-ethylurea or a salt thereof;
[14] 1-[2-(4-{[4-(1,2-benzoisothiazol-4-yloxy)-3-chlorophenyl]amino}-5H-pyrrolo[3,2-d]pyrimidin-5-yl)ethyl]-3-methylurea or a salt thereof;
[15] N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide or a salt thereof;
[16] N-[2-(4-{[4-(1,2-benzoisothiazol-4-yloxy)-3-chlorophenyl]amino}-5H-pyrrolo[3,2-d]pyrimidin-5-yl)ethyl]-$N^2$-tert-butylglycinamide or a salt thereof;
[17] a prodrug of the compound of the above-mentioned [1];
[18] a pharmaceutical agent comprising the compound of the above-mentioned [1] or a salt thereof, or a prodrug thereof;
[19] the pharmaceutical agent of the above-mentioned [18], which is a tyrosine kinase inhibitor;
[20] the pharmaceutical agent of the above-mentioned [18], which is an agent for the prophylaxis or treatment of cancer;
[21] the pharmaceutical agent of the above-mentioned [18], which is an agent for the prophylaxis or treatment of breast cancer, ovarian cancer, colorectal cancer, small intestinal cancer, gastric cancer, esophagus cancer, prostate cancer, lung cancer, pancreatic cancer or kidney cancer;
[22] a method for the prophylaxis or treatment of cancer in a mammal, which comprises administering an effective amount of the compound of the above-mentioned [1] or a salt thereof, or a prodrug thereof, to the mammal;
[23] use of the compound of the above-mentioned [1] or a salt thereof, or a prodrug thereof, for the production of an agent for the prophylaxis or treatment of cancer;

and the like.

## Effects of the Invention

According to the present invention, a fused pyrimidine compound having a superior tyrosine kinase inhibitory action, which is low toxic and sufficiently satisfactory as a pharmaceutical product, a production method thereof and use thereof are provided.

## Detailed Description of the Invention

$R^1$ is a hydrogen atom, a halogen atom, or an optionally substituted group bonded via a carbon atom, a nitrogen atom or an oxygen atom.
Examples of the "halogen atom" for $R^1$ include a fluorine atom, a chlorine atom, a bromine atom and an iodine atom.
Of the "optionally substituted group bonded via a carbon atom, a nitrogen atom or an oxygen atom" for $R^1$, examples of the "optionally substituted group bonded via a carbon atom" include cyano, optionally substituted $C_{1-8}$ alkyl, optionally substituted $C_{2-8}$ alkenyl, optionally substituted $C_{2-8}$ alkynyl, optionally substituted carbamoyl, optionally substituted $C_{1-8}$ alkyl-carbonyl, optionally substituted $C_{3-8}$ cycloalkyl, optionally substituted $C_{6-18}$ aryl, optionally substituted $C_{6-18}$ aryl-$C_{1-4}$ alkyl, optionally substituted $C_{6-18}$ aryl-carbonyl, optionally substituted $C_{6-18}$ aryl-$C_{1-4}$ alkyl-carbonyl, an optionally substituted heterocyclic group, optionally substituted heterocyclyl-$C_{1-4}$ alkyl, optionally substituted heterocyclyl-carbonyl and optionally substituted heterocyclyl-$C_{1-4}$ alkyl-carbonyl.
Examples of the "$C_{1-8}$ alkyl" of the above-mentioned "optionally substituted $C_{1-8}$ alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl and the like.
The "$C_{1-8}$ alkyl" of the above-mentioned "optionally substituted $C_{1-8}$ alkyl" may have one or more (preferably 1 to 5, more preferably 1 to 3) substituents at the substitutable positions. Such substituent is selected from the group consisting of

(a) a halogen atom,
(b) oxo,
(c) optionally-halogenated $C_{1-4}$ alkyl (e.g., methyl, ethyl, propyl, isopropyl, butyl, isobutyl, sec-butyl, tert-butyl),
(d) $C_{3-8}$ cycloalkyl (e.g., cyclopropyl, cyclobutyl, cyclopentyl, cyclohexyl, cycloheptyl, cyclooctyl etc.),
(e) $-(CH_2)_m-Q$ group,

(f) $-(CH_2)_m-Z^1-(C_{1-4}$ alkyl (e.g., methyl, ethyl, propyl, isopropyl, butyl, isobutyl, sec-butyl, tert-butyl) optionally substituted by substituent(s) selected from hydroxy, a halogen atom, cyano, $C_{1-4}$ alkoxy, amino and di-$C_{1-4}$ alkylamino),
(g) $-(CH_2)_m-Z^1-(C_{3-8}$ cycloalkyl (e.g., cyclopropyl, cyclobutyl, cyclopentyl, cyclohexyl, cycloheptyl, cyclooctyl etc.) optionally substituted by substituent(s) selected from hydroxy and cyano),
(h) $-(CH_2)_m-Z^1-(C_{6-10}$ aryl (e.g., phenyl etc.) optionally substituted by $C_{1-4}$ alkyl optionally substituted by halogen atom(s)),
(i) $-(CH_2)_m-Z^2-(CH_2)_n$-Q group,
(j) $-(CH_2)_m-Z^2-(CH2)_n-Z^1-C_{1-4}$ alkyl (e.g., methyl, ethyl, propyl, isopropyl, butyl, isobutyl, sec-butyl, tert-butyl),
(k) $-(CH_2)_m-Z^2-(CH_2)_n-Z^1-C_{3-8}$ cycloalkyl (e.g., cyclopropyl, cyclobutyl, cyclopentyl, cyclohexyl, cycloheptyl, cyclooctyl etc.),
(l) $-(CH_2)_m-Z^1$-(a heterocyclic group optionally substituted by substituent(s) selected from $C_{1-4}$ alkyl, hydroxy and amino (preferably a 5- to 8-membered heterocyclic group having 1 to 3 heteroatoms selected from a nitrogen atom, an oxygen atom and an optionally oxidized sulfur atom)),
(m) $-(CH_2)_m-Z^2-(CH_2)_n$-(a heterocyclic group optionally substituted by $C_{1-4}$ alkyl (preferably a 5- to 8-membered heterocyclic group having 1 to 3 heteroatoms selected from a nitrogen atom, an oxygen atom and an optionally oxidized sulfur atom)),
(n) $-(CH_2)_m-Z^2-C_{1-4}$ alkoxy (e.g., methoxy, ethoxy, propoxy, isopropoxy, butoxy, isobutoxy, sec-butoxy, tert-butoxy),
(o) $-(CH_2)_m-Z^2-(CH_2)_n-Z^1-(CH_2)_n-Z^1-C_{1-4}$ alkyl (e.g., methyl, ethyl, propyl, isopropyl, butyl, isobutyl, sec-butyl, tert-butyl), and
(p) 3- to 6-membered cyclic amino optionally substituted by substituent(s) selected from $C_{1-4}$ alkyl and oxo (hereinafter sometimes to be referred to as substituent group X). When the number of the substituents is 2 or more, the respective substituents may be the same or different.

In the above-mentioned formulas,

m is an integer of 0 to 4;
n is an integer of 1 to 4;
Q is hydroxy, carboxy, cyano, nitro, $-NR^{11}R^{12}$, $-CONR^{11}R^{12}$ or - $SO_2NR^{11}R^{12}$;
$Z^1$ is -O-, -CO-, $-C(OH)R^{13}-$, $-C(=N-OR^{13})-$, -S-, -SO-, $-SO_2-$, - $N(COR^{13})-$, $-N(CO_2R^{14})-$, $-N(SO_2R^{14})-$, -CO-O-, -O-CO-, $-CO-NR^{13}-$, - $NR^{13}-CO-$, $-NR^{13}-CO_2-$, $-NR^{13}-CO-NH-$, $-NR^{13}-SO_2-$ or $-NR^{13}-C(=NH)-NH-$; and
$Z^2$ is -O-, -CO-, $-C(OH)R^{13}-$, $-C(=N-OR^{13})-$, -S-, -SO-, $-SO_2-$,$-NR^{13}-$, $-N(COR^{13})-$, $-N(CO_2R^{14})-$, $-N(SO_2R^{14})-$, -CO-O-, -O-CO-, $-CO-NR^{13}-$, $-NR^{13}-CO-$, $-NR^{13}-CO_2-$, $-NR^{13}-CO-NH-$, $-NR^{13}-C(=NH)-NH-$, - $NR^{13}-SO_2-$ or $-SO_2NR^{13}-$.

In addition, $-(CH_{2)m}-$ and $-(CH_2)_n-$ in the above-mentioned formulas are optionally substituted by, for example, one or more (preferably 1 to 5, more preferably 1 to 3) substituents selected from halogen, optionally halogenated $C_{1-4}$ alkyl and hydroxy. When m or n is not less than 2, a subset $-CH_2CH_2-$ of $-(CH_2)_m-$ and $-(CH_2)_n-$ may be replaced by -CH=CH- or -C≡C-.
In the above-mentioned formulas, $R^{11}$ and $R^{12}$ are the same or different and each is a hydrogen atom or $C_{1-4}$ alkyl (e.g., methyl, ethyl, propyl, isopropyl, butyl, isobutyl, sec-butyl, tert-butyl), or $R^{11}$ and $R^{12}$ may be bonded to form a ring together with the nitrogen atom.
In addition, in the above-mentioned formulas, $R^{13}$ is a hydrogen atom or $C_{1-4}$ alkyl (e.g., methyl, ethyl, propyl, isopropyl, butyl, isobutyl, sec-butyl, tert-butyl), and $R^{14}$ is $C_{1-4}$ alkyl (e.g., methyl, ethyl, propyl, isopropyl, butyl, isobutyl, sec-butyl, tert-butyl).
When $R^{11}$ and $R^{12}$ are bonded to form a ring together with the nitrogen atom, Examples of the nitrogen-containing heterocyclic group include a 3- to 8-membered (preferably 5-or 6-membered) saturated or unsaturated (preferably saturated) aliphatic heterocycle such as azetidine, pyrrolidine, piperidine, homopiperidine, heptamethylenimine, morpholine, thiomorpholine, piperazine, homopiperazine and the like.
Examples of the "$C_{2-8}$ alkenyl" of the above-mentioned "optionally substituted $C_{2-8}$ alkenyl" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
The "$C_{2-8}$ alkenyl" of the above-mentioned "optionally substituted $C_{2-8}$ alkenyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.
Examples of the "$C_{2-8}$ alkynyl" of the above-mentioned "optionally substituted $C_{2-8}$ alkynyl" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.
The "$C_{2-8}$ alkynyl" of the above-mentioned "optionally substituted $C_{2-8}$ alkynyl" may have one or more (preferably

1 to 5, more preferably 1 to 3) substituents at the substitutable positions. Examples of such substituent include substituents selected from substituent group X. When the number of the substituents is not less than 2, the respective substituents may be the same or different.

The "carbamoyl" of the above-mentioned "optionally substituted carbamoyl" may have 1 or 2 substituents at the substitutable positions. Examples of such substituent include substituents selected from substituent group X. When the number of the substituents is not less than 2, the respective substituents may be the same or different.

Examples of the "$C_{1-8}$ alkyl-carbonyl" of the above-mentioned "optionally substituted $C_{1-8}$ alkyl-carbonyl" include acetyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, neopentylcarbonyl, 1-ethylpropylcarbonyl, hexylcarbonyl, isohexylcarbonyl, 1,1-dimethylbutylcarbonyl, 2,2-dimethylbutylcarbonyl, 3,3-dimethylbutylcarbonyl, 2-ethylbutylcarbonyl, heptylcarbonyl, octylcarbonyl and the like.

The "$C_{1-8}$ alkyl-carbonyl" of the above-mentioned "optionally substituted $C_{1-8}$ alkyl-carbonyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

Examples of the "$C_{3-8}$ cycloalkyl" of the above-mentioned "optionally substituted $C_{3-8}$ cycloalkyl" include cyclopropyl, cyclobutyl, cyclopentyl, cyclohexyl, cycloheptyl, cyclooctyl and the like.

The "$C_{3-8}$ cycloalkyl" of the above-mentioned "optionally substituted $C_{3-8}$ cycloalkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl" of the above-mentioned "optionally substituted $C_{6-18}$ aryl" include phenyl, naphthyl, anthryl, phenanthryl, acenaphthylenyl, biphenylyl and the like.

The "$C_{6-18}$ aryl" of the above-mentioned "optionally substituted $C_{6-18}$ aryl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-$C_{1-4}$ alkyl" of the above-mentioned "optionally substituted $C_{6-18}$ aryl-$C_{1-4}$ alkyl" include benzyl, phenethyl, phenylpropyl, naphthylmethyl, biphenylylmethyl and the like.

The "$C_{6-18}$ aryl-$C_{1-4}$ alkyl" of the above-mentioned "optionally substituted $C_{6-18}$ aryl-$C_{1-4}$ alkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-carbonyl" of the above-mentioned "optionally substituted $C_{6-18}$ aryl-carbonyl" include phenylcarbonyl, naphthylcarbonyl, anthrylcarbonyl, phenanthrylcarbonyl, acenaphthylenylcarbonyl, biphenylylcarbonyl and the like.

The "$C_{6-18}$ aryl-carbonyl" of the above-mentioned "optionally substituted $C_{6-18}$ aryl-carbonyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-$C_{1-4}$ alkyl-carbonyl" of the above-mentioned "optionally substituted $C_{6-18}$ aryl-$C_{1-4}$ alkyl-carbonyl" include benzylcarbonyl, phenethylcarbonyl, phenylpropylcarbonyl, naphthylmethylcarbonyl, biphenylylmethylcarbonyl and the like.

The "$C_{6-18}$ aryl-$C_{1-4}$ alkyl-carbonyl" of the above-mentioned "optionally substituted $C_{6-18}$ aryl-$C_{1-4}$ alkyl-carbonyl" may have one or more (preferably 1 to 5, more preferably 1 to 3) substituents at the substitutable positions. Examples of such substituent include substituents selected from the below-mentioned substituent V. When the number of the substituents is not less than 2, the respective substituents may be the same or different.

Examples of the "heterocyclic group" of the above-mentioned "optionally substituted heterocyclic group" include an aromatic heterocyclic group and a non-aromatic heterocyclic group.

Examples of the "aromatic heterocyclic group" include a 4- to 7-membered (preferably 5- or 6-membered) monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group. Examples of the fused aromatic heterocyclic group include a group derived from a fused ring wherein a ring corresponding to such 4- to 7-membered monocyclic aromatic heterocyclic group, and 1 or 2 rings selected from a 5- or 6-membered aromatic heterocycle containing 1 or 2 nitrogen atoms, a 5-membered aromatic heterocycle containing one sulfur atom and a benzene ring and the like are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl

(e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo [2,3-b]pyrazin-6-yl), pyrrolopyrimidinyl (e.g., 1H-pyrrolo[2,3-d]pyrimidin-2-yl, 1H-pyrrolo[2,3-d]pyrimidin-6-yl), imidazopyridinyl (e.g., 1H-imidazo[4,5-b]pyridin-2-yl, 1H-imidazo[4,5-C]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the "non-aromatic heterocyclic group" include a 4- to 7-membered (preferably 5- or 6-membered) monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group. Examples of the fused non-aromatic heterocyclic group include a group derived from a fused ring wherein a ring corresponding to such 4- to 7-membered monocyclic non-aromatic heterocyclic group, and 1 or 2 rings selected from a 5- or 6-membered heterocycle containing 1 or 2 nitrogen atoms, a 5-membered heterocycle containing one sulfur atom and a benzene ring and the like are condensed, and the like.

Preferable examples of the non-aromatic heterocyclic group include monocyclic non-aromatic heterocyclic groups such as oxetanyl (e.g., 2-oxetanyl, 3-oxetanyl), pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like; fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like;
and the like.

The "heterocyclic group" of the above-mentioned "optionally substituted heterocyclic group" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the above-mentioned "optionally substituted heterocyclyl-$C_{1-4}$ alkyl" include a group wherein $C_{1-4}$ alkyl (e.g., methyl, ethyl, propyl, isopropyl, butyl, isobutyl, sec-butyl, tert-butyl) is substituted by the above-mentioned "optionally substituted heterocyclic group".

Examples of the above-mentioned "optionally substituted heterocyclyl-carbonyl" include a group wherein the above-mentioned "optionally substituted heterocyclic group" is bonded to carbonyl.

Examples of the above-mentioned "optionally substituted heterocyclyl-$C_{1-4}$ alkyl-carbonyl" include a group wherein the above-mentioned "optionally substituted heterocyclyl-$C_{1-4}$ alkyl" is bonded to carbonyl.

Of the "optionally substituted group bonded via a carbon atom, a nitrogen atom or an oxygen atom" for $R^1$,

examples of the "optionally substituted group bonded via a nitrogen atom" include

(i) amino,
(ii) amino mono-substituted by the above-mentioned "optionally substituted group bonded via a carbon atom", and
(iii) amino di-substituted by the above-mentioned "optionally substituted group bonded via a carbon atom" and $C_{1-6}$ alkyl (e.g., methyl, ethyl, propyl, isopropyl, butyl, isobutyl, sec-butyl, tert-butyl, pentyl, propyl etc.).

Of the "optionally substituted group bonded via a carbon atom, a nitrogen atom or an oxygen atom" for $R^1$, examples of the "optionally substituted group bonded via an oxygen atom" include hydroxy optionally substituted by the above-mentioned "optionally substituted group bonded via a carbon atom".
As $R^1$, a hydrogen atom or a halogen atom is preferable, and a hydrogen atom or a chlorine atom is particularly preferable.
$R^2$ is a hydrogen atom, or an optionally substituted group bonded via a carbon atom or a sulfur atom.
Of the "optionally substituted group bonded via a carbon atom or a sulfur atom" for $R^2$, examples of the "optionally substituted group bonded via a carbon atom" include those similar to the "optionally substituted group bonded via a carbon atom" for $R^1$.
Of the "optionally substituted group bonded via a carbon atom or a sulfur atom" for $R^2$, examples of the "optionally substituted group bonded via a sulfur atom" include mercapto optionally substituted by the above-mentioned "optionally substituted group bonded via a carbon atom" wherein the sulfur atom may be oxidized.
As $R^2$, a hydrogen atom or optionally substituted alkyl is preferable. Of these, $C_{1-6}$ alkyl (e.g., methyl, ethyl) optionally having one substituent selected from

(1) hydroxy,
(2) $C_{1-6}$ alkyl-carbonylamino (e.g., acetylamino, 1-methylethylcarbonylamino, 1,1-dimethylethylcarbonylamino, 2-methylpropylcarbonylamino, tert-butylcarbonylamino) optionally having 1 to 3 substituents selected from (1') a halogen atom (e.g., fluorine atom), (2') amino, (3') $C_{1-6}$ alkylamino (e.g., tert-butylamino), (4') hydroxy and (5') $C_{1-6}$ alkylsulfonyl (e.g., methylsulfonyl),
(3) $C_{3-6}$ cycloalkyl-carbonylamino (e.g., cyclopropylcarbonylamino) optionally having one substituent selected from (1') cyano, (2') amino and (3') hydroxy,
(4) $C_{2-6}$ alkynyl-carbonylamino (e.g., 1-propynylcarbonylamino),
(5) heterocyclyl-carbonylamino (e.g., 2-pyrrolidinylcarbonylamino, 3-pyrrolidinylcarbonylamino),
(6) $C_{1-6}$ alkyl-aminocarbonyl-amino (e.g., methylaminocarbonylamino, ethylaminocarbonylamino, isopropylaminocarbonylamino),
(7) $C_{1-6}$ alkyl-carbonyloxy (e.g., acetoxy, ethylcarbonyloxy) optionally having one carboxy, and
(8) $C_{1-6}$ alkylsulfonyl (e.g., methylsulfonyl),

is preferable.
$R^3$ is a hydrogen atom or an optionally substituted aliphatic hydrocarbon group.
Examples of the "aliphatic hydrocarbon group" of the "optionally substituted aliphatic hydrocarbon group" for $R^3$ include those similar to the "optionally substituted $C_{1-8}$ alkyl", "optionally substituted $C_{2-8}$ alkenyl", "optionally substituted $C_{2-8}$ alkynyl" and "optionally substituted $C_{3-8}$ cycloalkyl" exemplified as the "optionally substituted group bonded via a carbon atom" for $R^1$.
As $R^3$, a hydrogen atom is preferable.
Ring A is an optionally substituted benzene ring.
The "benzene ring" of the "optionally substituted benzene ring" for ring A is optionally substituted by 1 to 5 substituents selected from the group consisting of

(1) $C_{3-10}$ cycloalkyl (e.g., cyclopropyl, cyclohexyl) optionally substituted by 1 to 3 substituents selected from the group consisting of

(a) halogen;
(b) hydroxy;
(c) carboxyl;
(d) sulfo;
(e) cyano;
(f) azido;
(g) nitro;
(h) nitroso;

(i) optionally halogenated $C_{1-4}$ alkyl (e.g., methyl, ethyl, propyl, isopropyl, butyl, isobutyl, sec-butyl, tert-butyl);
(j) optionally halogenated $C_{2-4}$ alkenyl (e.g., ethenyl, 1-propenyl, 2-propenyl, 2-methyl-1-propenyl, 1-butenyl, 2-butenyl, 3-butenyl);
(k) optionally halogenated $C_{2-4}$ alkynyl (e.g., ethynyl, 1-propynyl, 2-propynyl, 1-butynyl, 2-butynyl, 3-butynyl);
(1) $C_{3-7}$ cycloalkyl (e.g., cyclopropyl, cyclobutyl, cyclopentyl, cyclohexyl, cycloheptyl);
(m) $C_{6-14}$ aryl (e.g., phenyl, naphthyl, anthryl, phenanthryl, acenaphthylenyl, biphenylyl);
(n) $C_{7-16}$ aralkyl (e.g., benzyl, phenethyl, phenylpropyl, naphthylmethyl, biphenylylmethyl);
(o) formyl;
(p) optionally halogenated $C_{1-6}$ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl);
(q) optionally halogenated $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(r) optionally halogenated $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl);
(s) carbamoyl;
(t) mono- or di-substituted carbamoyl by optionally halogenated $C_{1-6}$ alkyl (e.g., methyl, ethyl, propyl, isopropyl, butyl, isobutyl, sec-butyl, tert-butyl);
(u) mono- or di-$C_{6-14}$ aryl-carbamoyl (e.g., phenylcarbamoyl, naphthylcarbamoyl, anthrylcarbamoyl, phenanthrylcarbamoyl, acenaphthylenylcarbamoyl, biphenylylcarbamoyl);
(v) mono- or di-optionally substituted thiocarbamoyl by optionally halogenated $C_{1-6}$ alkyl (e.g., methyl, ethyl, propyl, isopropyl, butyl, isobutyl, sec-butyl, tert-butyl);
(w) mono- or di-optionally substituted ureido by optionally halogenated $C_{1-6}$ alkyl (e.g., methyl, ethyl, propyl, isopropyl, butyl, isobutyl, sec-butyl, tert-butyl);
(x) mono- or di-$C_{6-14}$ aryl-ureido (e.g., phenylureido, naphthylureido, anthrylureido, phenanthrylureido, acenaphthylenylureido, biphenylylureido);
(y) mono- or di-optionally substituted sulfamoyl by optionally halogenated $C_{1-6}$ alkyl (e.g., methyl, ethyl, propyl, isopropyl, butyl, isobutyl, sec-butyl, tert-butyl);
(z) optionally halogenated $C_{1-6}$ alkoxy (e.g., methoxy, ethoxy, propoxy, isopropoxy, butoxy, tert-butoxy, pentyloxy, hexyloxy);
(aa) optionally halogenated $C_{2-6}$ alkenyloxy (e.g., ethenyloxy, 1-propenyloxy, 2-propenyloxy, 2-methyl-1-propenyloxy, 1-butenyloxy, 2-butenyloxy, 3-butenyloxy);
(bb) $C_{3-10}$ cycloalkyloxy (e.g., cyclopropyloxy, cyclobutyloxy, cyclopentyloxy, cyclohexyloxy, cycloheptyloxy);
(cc) $C_{7-13}$ aralkyloxy (e.g., benzyloxy, phenethyloxy, phenylpropyloxy, naphthylmethyloxy, biphenylylmethyloxy);
(dd) $C_{6-14}$ aryloxy (e.g., phenyloxy, naphthyloxy, anthryloxy, phenanthryloxy, acenaphthylenyloxy, biphenylyloxy);
(ee) $C_{1-6}$ alkyl-carbonyloxy (e.g., acetyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy);
(ff) $C_{3-10}$ cycloalkyl (e.g., cyclopropyl, cyclobutyl, cyclopentyl, cyclohexyl, cycloheptyl)-$C_{1-6}$ alkyloxy (e.g., methoxy, ethoxy, propoxy, isopropoxy, butoxy, tert-butoxy, pentyloxy, hexyloxy);
(gg) $C_{1-6}$ alkylsulfonyloxy (e.g., methylsulfonyloxy, ethylsulfonyloxy, propylsulfonyloxy, isopropylsulfonyloxy, butylsulfonyloxy, isobutylsulfonyloxy, sec-butylsulfonyloxy, tert-butylsulfonyloxy);
(hh) mercapto;
(ii) optionally halogenated $C_{1-6}$ alkylthio (e.g., methylthio, ethylthio, propylthio, isopropylthio, butylthio, isobutylthio, sec-butylthio, tert-butylthio);
(jj) $C_{7-13}$ aralkylthio (e.g., benzylthio, phenethylthio, phenylpropylthio, naphthylmethylthio, biphenylylmethylthio);
(kk) $C_{6-14}$ arylthio (e.g., phenylthio, naphthylthio, anthrylthio, phenanthrylthio, acenaphthylenylthio, biphenylylthio);
(11) $C_{1-6}$ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl);
(mm) oxo;
(nn) $C_{1-3}$ alkylenedioxy (e.g., methylenedioxy, ethylenedioxy, propylenedioxy); and
(oo) hydroxyimino optionally substituted by $C_{1-6}$ alkyl (e.g., methyl, ethyl, propyl, isopropyl, butyl, isobutyl, sec-butyl, tert-butyl); (hereinafter sometimes to be referred to as substituent group A),

(2) $C_{6-14}$ aryl (e.g., phenyl, naphthyl) optionally substituted by 1 to 3 substituents selected from substituent group A;
(3) a heterocyclic group (the heterocyclic group is similar to the "heterocyclic group" of the "optionally substituted heterocyclic group" exemplified as the "optionally substituted group bonded via a carbon atom" for $R^1$) optionally

substituted by 1 to 3 substituents selected from substituent group A;
(4) amino optionally substituted by 1 or 2 substituents selected from the group consisting of

(a) $C_{1-6}$ alkyl (e.g., methyl, ethyl, propyl, isopropyl, butyl, isobutyl, sec-butyl, tert-butyl) optionally substituted by substituent(s) selected from the group consisting of halogen, hydroxy, $C_{3-7}$ cycloalkyl (e.g., cyclopropyl, cyclobutyl, cyclopentyl, cyclohexyl, cycloheptyl), $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and $C_{1-6}$ alkoxy (e.g., methoxy, ethoxy, propoxy, isopropoxy, butoxy, tert-butoxy, pentyloxy, hexyloxy);
(b) optionally halogenated $C_{2-4}$ alkenyl (e.g., ethenyl, 1-propenyl, 2-propenyl, 2-methyl-1-propenyl, 1-butenyl, 2-butenyl, 3-butenyl);
(c) optionally halogenated $C_{2-4}$ alkynyl (e.g., ethynyl, 1-propynyl, 2-propynyl, 1-butynyl, 2-butynyl, 3-butynyl);
(d) $C_{3-7}$ cycloalkyl (e.g., cyclopropyl, cyclobutyl, cyclopentyl, cyclohexyl, cycloheptyl);
(e) $C_{6-14}$ aryl (e.g., phenyl, naphthyl, anthryl, phenanthryl, acenaphthylenyl, biphenylyl);
(f) $C_{7-16}$ aralkyl (e.g., benzyl, phenethyl, phenylpropyl, naphthylmethyl, biphenylylmethyl);
(g) a 4- to 7-membered (preferably 5- or 6-membered) heterocyclic group (e.g., a non-aromatic heterocyclic group such as morpholinyl etc.) containing, as a ring-constituting atom besides carbon atoms, 1 to 4 heteroatoms selected from an oxygen atom, a sulfur atom and a nitrogen atom;
(h) formyl;
(i) $C_{1-6}$ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl) optionally substituted by substituent(s) selected from the group consisting of halogen, hydroxy, $C_{3-7}$ cycloalkyl (e.g., cyclopropyl, cyclobutyl, cyclopentyl, cyclohexyl, cycloheptyl), $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and $C_{1-6}$ alkoxy (e.g., methoxy, ethoxy, propoxy, isopropoxy, butoxy, tert-butoxy, pentyloxy, hexyloxy);
(j) $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(k) $C_{6-14}$ aryl-carbonyl (e.g., benzoyl);
(l) $C_{7-13}$ aralkyl-carbonyl (e.g., benzylcarbonyl, phenethylcarbonyl);
(m) $C_{3-7}$ cycloalkyl-carbonyl (e.g., cyclopropylcarbonyl, cyclobutylcarbonyl, cyclopentylcarbonyl, cyclohexylcarbonyl, cycloheptylcarbonyl);
(n) $C_{1-6}$ alkyl-carbamoyl (e.g., methylcarbamoyl, ethylcarbamoyl);
(o) $C_{6-14}$ aryl-carbamoyl (e.g., phenylcarbamoyl, 1-naphthylcarbamoyl, 2-naphthylcarbamoyl);
(p) $C_{7-13}$ aralkyl-carbamoyl (e.g., benzylcarbamoyl);
(q) $C_{1-6}$ alkylsulfonyl (e.g., methylsulfonyl, ethylsulfonyl, isopropylsulfonyl);
(r) $C_{6-14}$ arylsulfonyl (e.g., benzenesulfonyl, toluenesulfonyl, 1-naphthalenesulfonyl, 2-naphthalenesulfonyl); and
(s) $C_{7-13}$ aralkylsulfonyl (e.g., benzylsulfonyl) (hereinafter sometimes to be referred to as substituent group B);

(5) amidino;
(6) optionally formylated or halogenated $C_{1-6}$ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl);
(7) optionally halogenated $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(8) optionally halogenated $C_{1-6}$ alkylsulfonyl (e.g., methylsulfonyl);
(9) carbamoyl optionally substituted by 1 or 2 substituents selected from substituent group B;
(10) thiocarbamoyl optionally mono- or di-substituted by optionally halogenated $C_{1-6}$ alkyl (e.g., methyl, ethyl, propyl, isopropyl, butyl, isobutyl, sec-butyl, tert-butyl);
(11) ureido optionally substituted by 1 or 2 substituents selected from substituent group B;
(12) sulfamoyl optionally substituted by 1 or 2 substituents selected from substituent group B;
(13) carboxyl;
(14) hydroxy;
(15) $C_{1-6}$ alkoxy (e.g., methoxy, ethoxy, propoxy, isopropoxy, butoxy, tert-butoxy, pentyloxy, hexyloxy) optionally substituted by 1 to 3 substituents selected from the group consisting of halogen, carboxyl, $C_{1-6}$ alkoxy (e.g., methoxy, ethoxy, propoxy, isopropoxy, butoxy, tert-butoxy, pentyloxy, hexyloxy) and $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(16) optionally halogenated $C_{2-6}$ alkenyloxy (e.g., ethenyloxy);
(17) $C_{3-10}$ cycloalkyloxy (e.g., cyclohexyloxy);

(18) $C_{7-13}$ aralkyloxy (e.g., benzyloxy);
(19) $C_{6-14}$ aryloxy (e.g., phenyloxy, naphthyloxy);
(20) $C_{1-6}$ alkyl-carbonyloxy (e.g., acetyloxy, tert-butylcarbonyloxy);
(21) mercapto;
(22) optionally halogenated $C_{1-6}$ alkylthio (e.g., methylthio, ethylthio);
(23) $C_{7-13}$ aralkylthio (e.g., benzylthio);
(24) $C_{6-14}$ arylthio (e.g., phenylthio, naphthylthio);
(25) sulfo;
(26) cyano;
(27) azido;
(28) nitro;
(29) nitroso;
(30) a halogen atom;
(31) $C_{1-6}$ alkylsulfinyl (e.g., methylsulfinyl);
(32) oxo;
(33) $C_{3-10}$ cycloalkyl-$C_{1-6}$ alkyloxy (e.g., cyclopropylmethyloxy);
(34) $C_{1-3}$ alkylenedioxy (e.g., methylenedioxy, ethylenedioxy);
(35) hydroxyimino optionally substituted by $C_{1-6}$ alkyl;
(36) $C_{1-10}$ alkyl (e.g., methyl, ethyl, propyl, isopropyl, butyl, isobutyl, sec-butyl, tert-butyl) optionally substituted by 1 to 3 substituents selected from the above-mentioned (1) - (35);
(37) $C_{2-10}$ alkenyl (e.g., ethenyl, 1-propenyl) optionally substituted by 1 to 3 substituents selected from the above-mentioned (1) - (35), and
(38) $C_{7-13}$ aralkyl (e.g., benzyl) optionally substituted by 1 to 3 substituents selected from the above-mentioned (1) - (35) (hereinafter sometimes to be referred to as substituent group V). When the number of the substituents is not less than 2, the respective substituents may be the same or different.

As ring A, a benzene ring optionally substituted by 1 or 2 substituents selected from the group consisting of (1) a halogen atom, (2) $C_{1-4}$ alkyl optionally having 1 to 3 halogen atoms and (3) $C_{1-4}$ alkoxy is preferable. Particularly, a benzene ring optionally substituted by 1 or 2 substituents selected from the group consisting of a fluorine atom, a chlorine atom, a bromine atom, methyl, trifluoromethyl and methoxy is preferable.
Ring B is an optionally substituted benzoisothiazole ring.
The "benzoisothiazole ring" of the "optionally substituted benzoisothiazole ring" for ring B optionally has one or more (preferably 1 to 4, more preferably 1 to 3) substituents at substitutable position(s). Examples of the substituent include those selected from substituent group V.
As ring B, a benzoisothiazole ring optionally having one $C_{1-6}$ alkyl (e.g., methyl) is preferable, and a benzoisothiazole ring optionally having one $C_{1-6}$ alkyl (e.g., methyl), which is bonded to oxygen atom at the 4- or 6-position is particularly preferable. Especially, a benzoisothiazole ring bonded to oxygen atom at the 4-position is preferable.
$R^1$ and $R^2$ are optionally bonded to each other to form an optionally substituted ring structure. Examples of the "ring structure" include a saturated or unsaturated (preferably saturated) 4- to 8-membered (preferably 5- to 7-membered) heterocycle.
Examples of the "ring structure" of the "optionally substituted ring structure" formed by $R^1$ and $R^2$ bonded to each other include
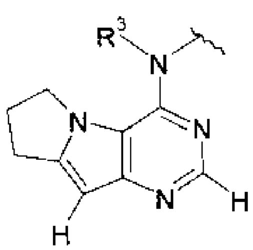
,
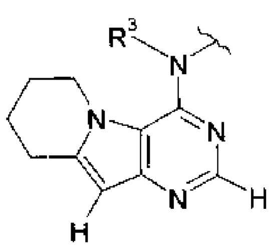
,
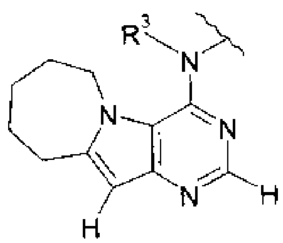
,
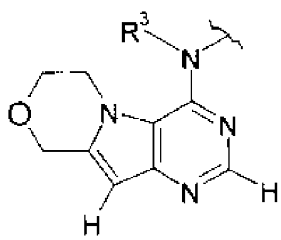

wherein each symbol is as defined above,
and the like.
$R^2$ and $R^3$ are optionally bonded to each other to form an optionally substituted ring structure. Examples of the

"ring structure" include a saturated or unsaturated (preferably saturated) 4- to 8-membered (preferably 5- to 7-membered) heterocycle.

Examples of the "ring structure" of the "optionally substituted ring structure" formed by $R^2$ and $R^3$ bonded to each other include

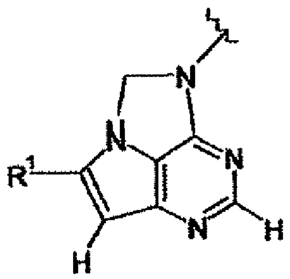

,

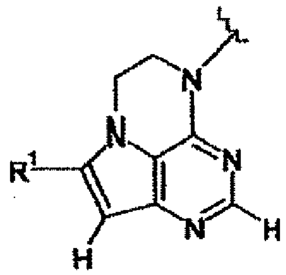

,

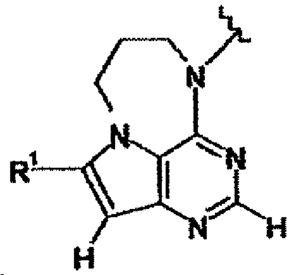

,

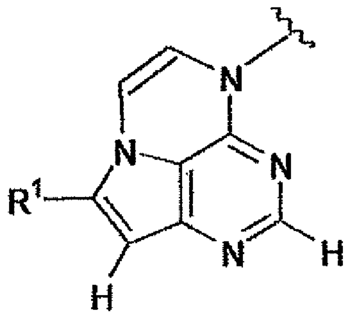

wherein each symbol is as defined above,
and the like.

The "ring structure" of the "optionally substituted ring structure" formed by $R^1$ and $R^2$, or $R^2$ and $R^3$, optionally has 1 to 5 (preferably 1 to 3, more preferably 1 or 2), the same or different substituents at any substitutable positions. Examples of the substituent include substituents (e.g., $C_{1-6}$ alkyl (e.g., methyl)) selected from substituent group V. When the number of the substituents is not less than 2, the respective substituents may be the same or different.

Examples of the "ring structure" of the "optionally substituted ring structure" formed by $R^3$ bonded to the carbon atom on the adjacent benzene ring (ring A) include a saturated or unsaturated (preferably saturated) 4- to 8-membered (preferably 5- or 6-membered) nitrogen-containing heterocycle.

Specifically, the moiety of

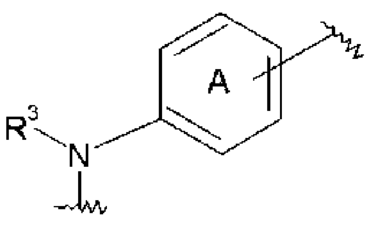

wherein each symbol is as defined above, is, for example,

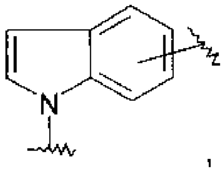 , 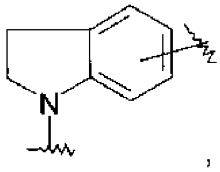 , 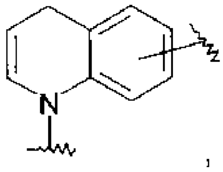 , 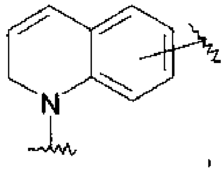 , 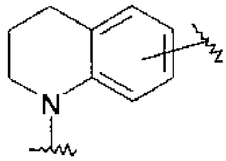

and the like.

The "ring structure" optionally has 1 to 5 (preferably 1 to 3, more preferably 1 or 2), the same or different substituents at any substitutable positions. Examples of the substituent include substituents selected from substituent group V. When the number of the substituents is not less than 2, the respective substituents may be the same or different.

Preferable compounds of compound (I) are as follows.

[Compound A]

Compound (I) wherein

$R^1$ is a hydrogen atom or a halogen atom;
$R^2$ is a hydrogen atom or optionally substituted alkyl;
$R^3$ is a hydrogen atom;
ring A is a benzene ring optionally substituted by 1 or 2 substituents selected from the group consisting of (1) a halogen atom, (2) $C_{1-4}$ alkyl optionally having 1 to 3 halogen atoms and (3) $C_{1-4}$ alkoxy;
ring B is a benzoisothiazole ring optionally having one $C_{1-6}$ alkyl.

[Compound B]

Compound (I) wherein

$R^1$ is a hydrogen atom or a chlorine atom;
$R^2$ is $C_{1-6}$ alkyl (e.g., methyl, ethyl) optionally having one substituent selected from

(1) hydroxy,
(2) $C_{1-6}$ alkyl-carbonylamino (e.g., acetylamino, 1-methylethylcarbonylamino, 1,1-dimethylethylcarbonylamino, 2-methylpropylcarbonylamino, tert-butylcarbonylamino) optionally having 1 to 3 substituents selected from (1') a halogen atom (e.g., fluorine atom), (2') amino, (3') $C_{1-6}$ alkylamino (e.g., tert-butylamino), (4') hydroxy and (5') $C_{1-6}$ alkylsulfonyl (e.g., methylsulfonyl),
(3) $C_{3-6}$ cycloalkyl-carbonylamino optionally having one substituent selected from (1') cyano, (2') amino and (3') hydroxy (e.g., cyclopropylcarbonylamino),
(4) $C_{2-6}$ alkynyl-carbonylamino (e.g., 1-propynylcarbonylamino),
(5) heterocyclyl-carbonylamino (e.g., 2-pyrrolidinylcarbonylamino, 3-pyrrolidinylcarbonylamino),
(6) $C_{1-6}$ alkyl-aminocarbonyl-amino (e.g., methylaminocarbonylamino, ethylaminocarbonylamino, isopropylaminocarbonylamino, t-butylaminocarbonylamino),
(7) $C_{1-6}$ alkyl-carbonyloxy (e.g., acetoxy, ethylcarbonyloxy) optionally having one carboxy, and
(8) $C_{1-6}$ alkylsulfonyl (e.g., methylsulfonyl);

$R^3$ is a hydrogen atom;
ring A is a benzene ring optionally substituted by 1 or 2 substituents selected from the group consisting of a fluorine atom, a chlorine atom, a bromine atom, methyl, trifluoromethyl and methoxy; and
ring B is a benzoisothiazole ring optionally having one methyl, which is bonded to oxygen atom at the 4- or 6-position.

[Compound C]

2-(4-{[4-(1,2-benzoisothiazol-4-yloxy)-3-chlorophenyl]amino}-5H-pyrrolo[3,2-d]pyrimidin-5-yl)ethanol (Example 3);
N-[2-(4-{[4-(1,2-benzoisothiazol-4-yloxy)-3-chlorophenyl]amino}-5H-pyrrolo[3,2-d]pyrimidin-5-yl)ethyl]acetamide

(Example 4);
N-[2-(4-{[4-(1,2-benzoisothiazol-4-yloxy)-3-chlorophenyl]amino}-5H-pyrrolo[3,2-d]pyrimidin-5-yl)ethyl]pyrrolidine-3-carboxamide (Example 33);
N-[2-(4-{[4-(1,2-benzoisothiazol-4-yloxy)-3-methylphenyl]amino}-5H-pyrrolo[3,2-d]pyrimidin-5-yl)ethyl]-2,2-dimethylpropanamide (Example 38);
N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-2,2-dimethylpropanamide (Example 41);
1-[2-(4-{[4-(1,2-benzoisothiazol-4-yloxy)-3-chlorophenyl]amino}-5H-pyrrolo[3,2-d]pyrimidin-5-yl)ethyl]-3-ethylurea (Example 51);
1-[2-(4-{[4-(1,2-benzoisothiazol-4-yloxy)-3-chlorophenyl]amino}-5H-pyrrolo[3,2-d]pyrimidin-5-yl)ethyl]-3-methylurea (Example 56);
N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide (Example 60);
N-[2-(4-{[4-(1,2-benzoisothiazol-4-yloxy)-3-chlorophenyl]amino}-5H-pyrrolo[3,2-d]pyrimidin-5-yl)ethyl]-$N^2$-tert-butylglycineamide (Example 61);

or a salt thereof.

Examples of the salts of compound (I) include metal salts, ammonium salts, salts with organic base, salts with inorganic acid, salts with organic acid, salts with basic or acidic amino acid and the like.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salts with organic base include salts with trimethylamine, triethylamine, pyridine, picoline, 2,6-lutidine, ethanolamine, diethanolamine, triethanolamine, tromethamine[tris(hydroxymethyl)methylamine], t-butylamine, cyclohexylamine, dicyclohexylamine, N,N'-dibenzylethylenediamine and the like.

Preferable examples of salts with inorganic acid include salts with hydrochloric acid, hydrobromic acid, nitric acid, sulfuric acid, phosphoric acid and the like.

Preferable examples of the salts with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acid include salts with arginine, lysine, ornithine and the like.

Preferable examples of the salts with acidic amino acid include salts with aspartic acid, glutamic acid and the like.

Of these, pharmaceutically acceptable salts are preferable. When a compound contains an acidic functional group, for example, inorganic salts such as alkali metal salts (e.g., sodium salt, potassium salt etc.), alkaline earth metal salts (e.g., calcium salt, magnesium salt, barium salt etc.) and the like, ammonium salt and the like can be mentioned. When a compound contains a basic functional group, for example, salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

A production method of compound (I) is described below.

The production intermediate includes salts, and as such salts, for example, those similar to the salts of compound (I) and the like can be used.

The compound obtained in each step can be used as a reaction mixture or as a crude product in the next reaction. In addition, the compound can be isolated from a reaction mixture according to a conventional method, and can be easily purified by a separation means such as recrystallization, distillation, chromatography and the like.

Schematic reaction formulas are shown in the following, wherein each symbol in the compounds is as defined above.

Compound (I) can be produced, for example, by reacting a compound represented by the formula:

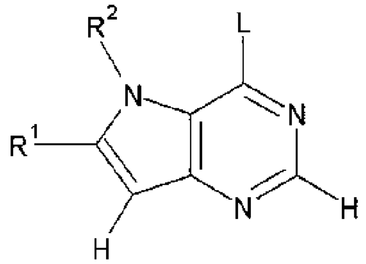

(II)

wherein L is a leaving group, and other symbols are as defined above (hereinafter sometimes to be abbreviated as compound (II)), or a salt thereof with a compound represented by the formula:

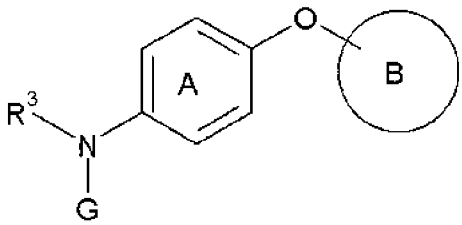

(III)

wherein G is a hydrogen atom or a metal atom, and other symbols are as defined above (hereinafter sometimes to be abbreviated as compound (III)), or a salt thereof.

G is mainly a hydrogen atom, but may be an alkali metal such as lithium, sodium, potassium, cesium and the like, or an alkaline earth metal such as magnesium, calcium and the like.

Compound (III) or a salt thereof is preferably used in an amount of 1 to 5 equivalents, preferably 1 to 2 equivalents, relative to compound (II) and the reaction is preferably carried out in a solvent. In addition, a base or an ammonium salt may be used in an amount of about 0.01 to 10 equivalents, preferably 0.1 to 2 equivalents.

In the aforementioned formula, as the leaving group for L, a halogen atom such as chlorine, bromine, iodine and the like, a group represented by the formula: $-S(O)_kP^z$ wherein k is an integer of 0, 1 or 2, and $R^z$ is a lower(e.g., $C_{1-4}$)alkyl such as methyl, ethyl, propyl and the like; a $C_{6-10}$ aryl such as phenyl and tolyl and the like; $C_{7-13}$ aralkyl such as benzyl and the like, and the like, or a group represented by the formula: $-OR^z$ wherein $R^z$ is as defined above, can be used.

As the solvent in the aforementioned reaction, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used.

As the base in the aforementioned reaction, an inorganic base, an organic base and the like can be used. Specifically, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used.

As the ammonium salt in the aforementioned reaction, pyridine hydrochloride, pyridine hydrobromide, pyridinium p-toluenesulfonate, quinoline hydrochloride, isoquinoline hydrochloride, pyrimidine hydrochloride, pyrazine hydrochloride, triazine hydrochloride, trimethylamine hydrochloride, triethylamine hydrochloride, N-ethyldiisopropylamine hydrochloride and the like can be used.

The aforementioned reaction can be carried out under cooling, at room temperature or under heating (about 40 to 200°C, preferably about 40 to 160°C), and the reaction time is generally about 1 to 30 hr, preferably about 1 to 20 hr, more preferably about 1 to 10 hr.

A compound within the scope of the present invention can be also produced by applying means known *per se* to the obtained compound (I) for introduction of substituents and conversion of functional groups. For conversion of

substituents, a known conventional method can be used. For example, conversion to carboxy by hydrolysis of ester, conversion to carbamoyl by amidation of carboxy, conversion to hydroxymethyl by reduction of carboxy, conversion to alcohol compound by reduction or alkylation of carbonyl, reductive amination of carbonyl, oximation of carbonyl, acylation of amino, alkylation of amino, substitution and amination of active halogen by amine, alkylation of hydroxy, substitution and amination of hydroxy and the like can be mentioned. When a reactive substituent that causes non-objective reaction is present during the introduction of substituents and conversion of functional groups, a protecting group is introduced in advance as necessary into the reactive substituent by a means known *per se,* and the protecting group is removed by a means known *per se* after the objective reaction, whereby the compound within the scope of the present invention can be also produced.

The compound (I), which is a product of the reaction, may be produced as a single compound or as a mixture.

The compound (I) thus obtained can be subjected to a means known *per se,* such as solvent extraction, concentration, neutralization, filtration, crystallization, recrystallization, column chromatography, high performance liquid chromatography and the like, whereby the objective compound can be isolated and purified at high purity from a reaction mixture.

The starting compound (III) of this production method is commercially available, or can be produced by a means known *per se.*

The starting compound (II) of this production method can be produced by, for example, a method shown by the following scheme. Here, compounds (IIa), (IIb), (IIc) and (IId) are encompassed in compound (II).

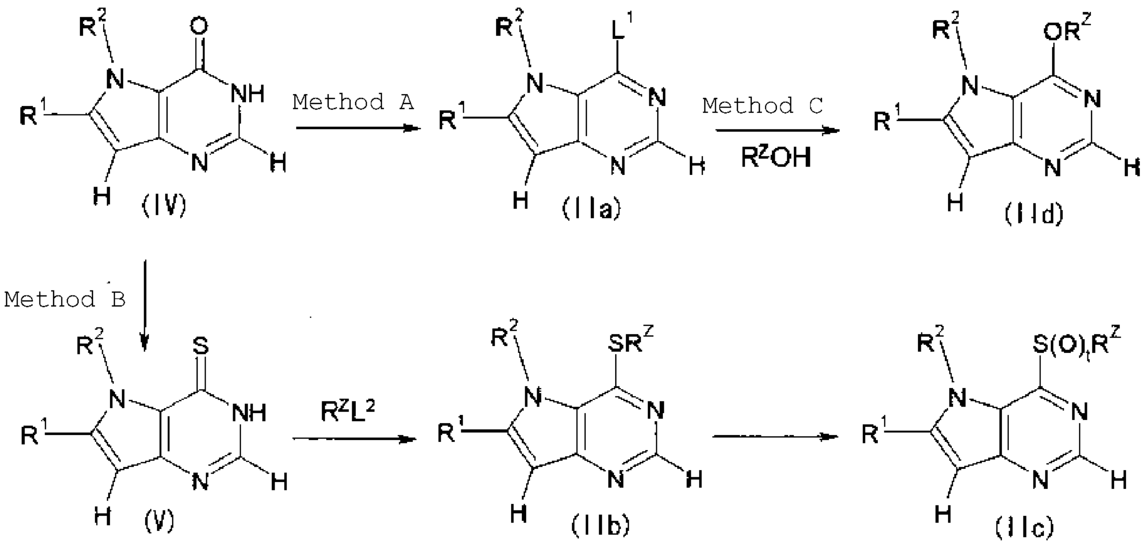


wherein $L^1$ and $L^2$ are halogen atoms, t is an integer of 1 or 2, and the other symbol is as defined above.

As Method A, compound (IIa) can be produced by reacting compound (IV) with a halogenating agent. As Method B, compound (IV) is reacted with a thionating agent to give compound (V), which is then reacted with a compound represented by $R^zL^2$ in the presence of a base to give compound (IIb), which is further subjected to an oxidation reaction to give compound (IIc). As Method C, compound (IId) can be produced by reacting compound (IIa) with a compound represented by $P^zOH$ in the presence of a base.

As the halogenating agent in Method A, for example, about 1 to 100 equivalents of phosphorus oxychloride, phosphorus pentachloride, phosphorus trichloride, thionyl chloride, sulfuryl chloride, phosphorus tribromide and the like can be used. In this case, the reaction may be carried out in the presence of a base such as diethylaniline, dimethylaniline, pyridine and the like. While the reaction may be carried out without solvent, as a reaction solvent, for example, halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetonitrile, ethyl acetate and the like may be used. The reaction is carried out under cooling, at room temperature or under heating, and the reaction time is generally about 1 to 20 hr, preferably about 1 to 10 hr.

As the thionating agent used in the production step from compound (IV) to compound (V) in Method B, for example, about 1 to 5 equivalents of the Lawesson's reagent, phosphorus pentasulfide and the like can be used. As the reaction solvent, for example, halogenated hydrocarbons such as dichloromethane, chloroform, carbon tetrachloride,

1,2-dichloroethane and the like; aromatic hydrocarbons such as benzene, toluene, xylene and the like; ethers such as diethyl ether, tetrahydrofuran, dioxane and the like; and the like can be used. The reaction is carried out at room temperature or under heating, and the reaction time is generally about 1 to 20 hr, preferably about 1 to 10 hr.

As $R^zL^2$ in the production step from compound (V) to compound (IIb) in Method B, for example, about 1 to 5 equivalents of methyl iodide, benzyl chloride, benzyl bromide and the like can be used, and as the base, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

As the oxidizing agent in the production step from compound (IIb) to compound (IIc) in Method B, for example, m-chloroperbenzoic acid, hydrogen peroxide, peracetic acid, t-butyl hydroperoxide, potassium peroxysulfate, potassium permanganate, sodium perborate, sodium periodate, sodium hypochlorite, halogen and the like can be used. When compound (IIc) wherein t=1 is produced, the oxidizing agent is used in about 1 to 1.5 equivalents relative to compound (IIb), and when compound (IIc) wherein t=2 is produced, it is used in about 2 to 3 equivalents relative to compound (IIb). The reaction solvent is not particularly limited as long as it does not react with the oxidizing agent and, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; carboxylic acids such as acetic acid, trifluoroacetic acid and the like; acetonitrile, ethyl acetate, N,N-dimethylformamide, N,N-dimethylacetamide, 1-methyl-2-pyrrolidone, dimethyl sulfoxide, water or a mixed solvent thereof and the like can be used. The reaction is carried out under cooling, at room temperature or under heating, and the reaction time is generally about 1 to 20 hr, preferably about 1 to 10 hr.

As $R^zOH$ in the production step from compound (IIa) to compound (IId) in Method C, for example, about 1 to 10 equivalents of methanol, ethanol, phenol and the like can be used, and as the base, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used. As a reaction solvent, for example, halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

Furthermore, compound (IV) can be produced by, for example, a method shown by the following formula:

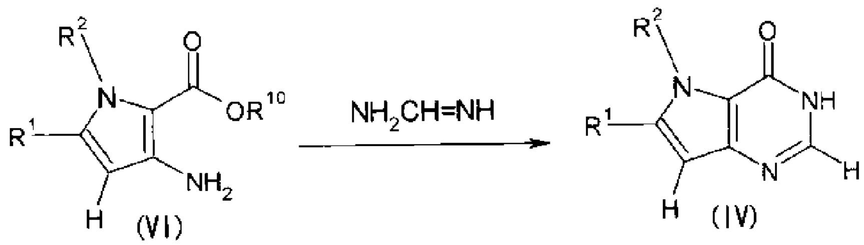


wherein $R^{10}$ is $C_{1-4}$ alkyl, and other symbols are as defined above.

That is, compound (VI) is reacted with about 1 to 4 equivalents of formamidine or a salt thereof to give compound (IV). As the reaction solvent, for example, alcohols such as methanol, ethanol, isopropanol, t-butanol and the like; halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

Compound (II) can be also produced by, for example, a method shown by the following formula:

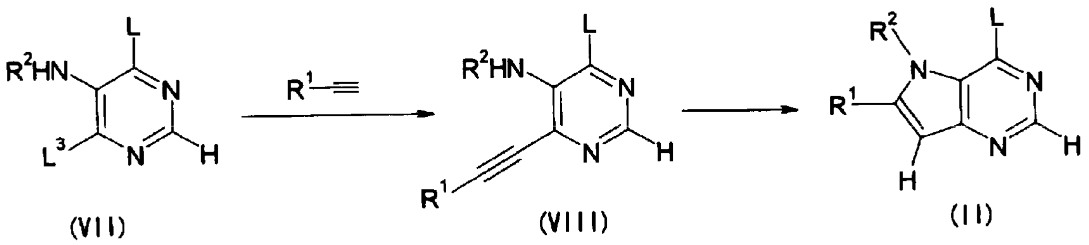

(VII) (VIII) (II)

wherein $L^3$ is a halogen atom, and other symbols are as defined above.

For the production step from compound (VII) to compound (VIII) in this method, a reaction generally known as a Sonogashira reaction or a reaction analogous thereto can be carried out, and generally, compound (VIII) can be produced by reacting compound (VII) with about 1 to 3 equivalents of a compound represented by the formula:

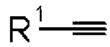

in the presence of a base, about 0.01-1 equivalent of a palladium catalyst and copper iodide. As the base, for example, triethylamine, N-ethyldiisopropylamine, diisopropylamine, pyridine, N,N-dimethylaminopyridine, diazabicycloundecene (DBU), sodium carbonate, potassium carbonate, sodium hydrogen carbonate, potassium hydrogen carbonate and the like can be used. As the palladium catalyst, for example, dichlorobis(triphenylphosphine)palladium(II), palladium on carbon, palladium(II) diacetate, bis(benzonitrile)dichloropalladium(II) and the like can be used. This reaction may be carried out in the co-presence of a tertiary phosphine compound such as triphenylphosphine, tributylphosphine and the like as a ligand. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane, 1,2-dimethoxyethane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. This reaction is carried out at room temperature or under heating, and the reaction time is generally about 1 to 50 hr, preferably about 1 to 20 hr.

For the production step from compound (VIII) to compound (II) in this method, a cyclization reaction is generally carried out in the presence of about 1 to 3 equivalents of a base or about 0.01-1 equivalent of copper iodide to give compound (II). As the base, for example, potassium t-butoxide, sodium t-butoxide, cesium t-butoxide, sodium ethoxide, potassium hydride, sodium hydride, cesium hydroxide, sodium hydroxide, potassium hydroxide, sodium carbonate, potassium carbonate, sodium hydrogen carbonate, potassium hydrogen carbonate, triethylamine, N-ethyldiisopropylamine, diisopropylamine, pyridine, N,N-dimethylaminopyridine, diazabicycloundecene (DBU) and the like can be used. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane, 1,2-dimethoxyethane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out at low temperature, at room temperature or under heating, and the reaction time is generally about 1 to 50 hr, preferably about 1 to 20 hr.

Depending on the kind of the substituent of starting compound (II), a starting compound (II) having a different substituent can be produced by substituent conversion from, as a starting material, a compound produced by the above-mentioned production method. For the substituent conversion, a known general method can be used. For example, conversion to carbamoyl by hydrolysis and amidation of ester, conversion to hydroxymethyl by reduction of carboxy,

conversion to alcohol compound by reduction or alkylation of carbonyl, reductive amination of carbonyl, oximation of carbonyl, acylation of amino, alkylation of amino, substitution and amination of active halogen by amine, alkylation of hydroxy, substitution and amination of hydroxy and the like can be mentioned. When a reactive substituent that causes non-objective reaction is present during the introduction of substituents and conversion of functional groups, a protecting group is introduced in advance as necessary into the reactive substituent by a means known *per se,* and the protecting group is removed by a means known *per se* after the objective reaction, whereby the starting compound (II) can be also produced.

In addition, a starting material, compound (II) can be produced by, for example, a method to be used compound (II') shown in the following formula.

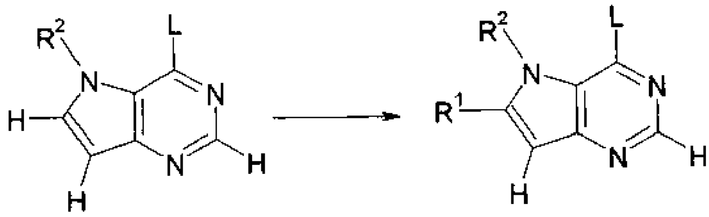

(II') (II)

wherein each symbol is as defined above.

A step for producing compound (II) from compound (II') in this method is generally completed by withdrawing a proton from compound (II') using a base to give an anion, and reacting the anion with a cation having $R^1$. As the base, for example, n-butyllithium, s-butyllithium, t-butyllithium, lithium t-butoxide, lithium diisopropylamide and the like can be used. As a reagent for generating the cation, for example, p-toluenesulfonyl chloride, benzenesulfonyl bromide, p-toluenesulfonyl cyanide, S-(trifluoromethyl)dibenzothiophenium trifluoromethanesulfonate, N,N-dimethylformamide and the like can be used. As the reaction solvent, for example, halogenated hydrocarbons such as dichloromethane, chloroform, carbon tetrachloride, 1,2-dichloroethane and the like; ethers such as diethyl ether, tetrahydrofuran, dioxane, 1,2-dimethoxyethane and the like, a mixed solvent thereof and the like can be used. The aforementioned reaction can be carried out under cooling, preferably about not more than -20°C, and the reaction time is generally about 15 min to 50 hr, preferably about 30 min to 4 hr.

Thus-obtained compound (I) can be isolated and purified by a separation means known *per se,* such as concentration, concentration under reduced pressure, solvent extraction, crystallization, recrystallization, phase transfer, chromatography and the like.

When compound (I) is obtained as a free form, it can be converted to a desired salt by a method known *per se* or a modification thereof; conversely, when compounds (I) is obtained as a salt, it can be converted to a free form or other desired salt by a method known *per se* or a modification thereof.

When compound (I) has isomers such as optical isomer, stereoisomer, positional isomer, rotational isomer and the like, any isomers and mixtures are encompassed in the compound (I). For example, when compound (I) has an optical isomer, an optical isomer separated from a racemate is also encompassed in the compound (I). These isomers can be obtained as independent products by a synthesis means or a separation means (concentration, solvent extraction, column chromatography, recrystallization and the like) known *per se.*

The compounds (I) may be a crystal, and both a single crystal and crystal mixtures are encompassed in the compound (I). Crystals can be produced by crystallization according to crystallization methods known *per se.*

The compounds (I) may be a solvate (e.g., hydrate etc.) or a non-solvate, both of which are encompassed in the compound (I).

A compound labeled with an isotope (e.g., $^{2}H$, $^{3}H$, $^{14}C$, $^{35}S$, $^{125}I$ and the like) is also encompassed in the compound (I).

A prodrug of the compounds (I) or salts thereof (hereinafter referred to as compound (I)) means a compound which is converted to the compounds (I) with a reaction due to an enzyme, an gastric acid, etc. under the physiological condition in the living body, that is, a compound which is converted to the compounds (I) with oxidation, reduction, hydrolysis, etc. due to an enzyme; a compound which is converted to the compounds (I) by hydrolysis etc. due to gastric acid, etc. A prodrug for compounds (I) may be a compound obtained by subjecting amino in compounds (I) to an acylation, alkylation or phosphorylation (e.g., a compound obtained by subjecting amino in compounds (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting hydroxy in compounds (I) to an acylation, alkylation, phosphorylation or boration (e.g., a compound obtained by subjecting hydroxy

in compounds (I) to an acetylation, palmitoylation, propanoylation, pivaloylation, succinylation, fumarylation, alanylation or dimethylaminomethylcarbonylation); a compound obtained by subjecting carboxyl in compounds (I) to an esterification or amidation (e.g., a compound obtained by subjecting carboxyl in compounds (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any one of these compounds can be produced from compounds (I) by a method known *per se.*

A prodrug for compounds (I) may also be one which is converted into compounds (I) under a physiological condition, such as those described in IYAKUHIN no KAIHATSU (Development of Pharmaceuticals), Vol. 7, Design of Molecules, p.163-198, Published by HIROKAWA SHOTEN (1990).

The compounds (I) of the present invention, or a salt thereof or a prodrug thereof (hereinafter referred to as the compound of the present invention) possess tyrosine kinase-inhibiting activity and can be used for the prophylaxis or treatment of tyrosine kinase-dependent diseases in mammals. Tyrosine kinase-dependent diseases include diseases characterized by increased cell proliferation due to abnormal tyrosine kinase enzyme activity.

Particularly, the compound of the present invention inhibits HER2 kinase and/or EGFR kinase and is therefore also useful as a therapeutic agent for suppressing the growth of HER2 and/or EGFR kinase-expressing cancer. Also, the compound of the present invention is useful as a preventive agent for preventing hormone-dependent cancer and the transition of hormone-dependent cancer to hormone-independent cancer. Since the compound of the present invention inhibits HER4 kinase, it is useful as an agent for the prophylaxis or treatment of the diseases caused by HER4. In addition, the compound of the present invention is useful as a pharmaceutical agent because it shows low toxicity (e.g., acute toxicity, chronic toxicity, genetic toxicity, reproductive toxicity, cardiotoxicity, drug interaction, carcinogenicity and the like), high water solubility, and is superior in stability, pharmacokinetics (absorption, distribution, metabolism, excretion and the like) and efficacy expression.

Accordingly, the compound of the present invention can be used as a safe agent for the prophylaxis or treatment of diseases due to abnormal cell proliferation such as various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colorectal cancer (e.g., familial colorectal cancer, hereditary nonpolyposis colorectal cancer, gastrointestinal stromal tumor etc.), small intestinal cancer, colon cancer (e.g., gastrointestinal stromal tumor etc.), rectal cancer (e.g., gastrointestinal stromal tumor etc.), esophagus cancer, duodenal cancer, cancer of the tongue, cancer of pharynx (e.g., nasopharyngeal carcinoma, orocancer of pharynx, hypocancer of pharynx etc.), salivary gland cancer, brain tumor (e.g., pineal astrocytoma, pilocytic astrocytoma, diffuse astrocytoma, anaplastic astrocytoma etc.), schwannoma, non-small cell lung cancer, small cell lung cancer, liver cancer (e.g., primary liver cancer, extrahepatic bile duct cancer etc.), kidney cancer (e.g., renal cell carcinoma, transitional cell cancer of renal pelvis and ureter etc.), biliary tract cancer, endometrial cancer, endometrial carcinoma, cancer of the uterine cervix, ovarian cancer (e.g., ovarian epithelial, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), Hemangioma, malignant lymphoma, malignant melanoma, thyroid cancer (e.g., medullary thyroid carcinoma etc.), parathyroid cancer, nasal cavity cancer, paranasal sinus cancer, bone tumor (e.g., osteosarcoma, Ewing's tumor, uterus sarcoma, soft tissue sarcoma etc.), vascular fibroma, retinoblastoma, penile cancer, solid cancer in childhood, Kaposi's sarcoma, Kaposi's sarcoma derived from AIDS, maxillary tumor, fibrous histiocytoma, leiomyosarcoma, rhabdomyosarcoma, leukemia (e.g., acute myeloid leukemia, acute lymphoblastic leukemia etc.) etc.), atherosclerosis, angiogenesis (e.g., angiogenesis associated with growth of solid cancer and sarcoma, angiogenesis associated with tumor metastasis, angiogenesis associated with diabetic retinopathy, etc.), viral diseases (HIV infection etc.) and the like.

Tyrosine kinase-dependent diseases further include cardiovascular diseases associated with abnormal tyrosine kinase enzyme activity. The compound of the present invention can therefore be used as an agent for prophylaxis or treatment of cardiovascular diseases such as restenosis.

The compound of the present invention is useful as an anticancer agent for the prophylaxis or treatment of cancer, especially breast cancer, ovarian cancer, colorectal cancer, gastric cancer, esophagus cancer, prostate cancer, lung cancer, pancreatic cancer and the like.

The compound of the present invention shows low toxicity and can be used as a pharmaceutical agent as it is, or as a pharmaceutical composition in admixture with a commonly known pharmaceutically acceptable carrier etc. in mammals (e.g., humans, horses, bovines, dogs, cats, rats, mice, rabbits, pigs, monkeys and the like).

In addition to the compound of the present invention, said pharmaceutical composition may contain other active ingredients, e.g., the following hormonal therapeutic agents, anticancer agents (e.g., chemotherapeutic agents, immunotherapeutic agents, or pharmaceutical agents inhibiting the action of cell growth factors or cell growth factor receptors,

etc.), and the like.

As a pharmaceutical agent for mammals such as humans, the compound of the present invention can be generally administered orally in the form of, for example, tablets, capsules (including soft capsules and microcapsules), powders, granules and the like, or parenterally in the form of injections, suppositories, pellets and the like. Examples of the "parenteral administration route" include intravenous, intramuscular, subcutaneous, intra-tissue, intranasal, intradermal, instillation, intracerebral, intrarectal, intravaginal, intraperitoneal, intratumoral, administration to juxtaposition and the like of tumor, or directly to the lesion.

The dose of the compound of the present invention varies depending on the route of administration, symptoms, etc. For example, when it is administered orally as an anticancer agent to a patient (body weight 40 to 80 kg) with breast cancer or prostate cancer, its dose is, for example, 0.5 to 100 mg/kg body weight per day, preferably 1 to 50 mg/kg body weight per day, and more preferably 1 to 25 mg/kg body weight per day. This amount may be administered once or in 2 to 3 divided portions daily.

The compound of the present invention can be safely administered orally or parenterally (e.g., topical, rectal, intravenous administrations etc.) as a single agent, or a pharmaceutical composition containing a pharmacologically acceptable carrier according to a conventional method (e.g., a method described in the Japanese Pharmacopoeia etc.), such as tablet (including sugar-coated tablet, film-coated tablet), powder, granule, capsule, liquid, emulsion, suspension, injection, suppository, sustained release preparation, plaster and the like.

A combination of (1) administering an effective amount of a compound of the present invention and (2) 1 to 3 selected from the group consisting of (i) administering an effective amount of other anticancer agents, (ii) administering an effective amount of hormonal therapeutic agents and (iii) non-drug therapy can prevent and/or treat cancer more effectively. As the non-drug therapy, for example, surgery, radiotherapy, gene therapy, thermotherapy, cryotherapy, laser cauterization and the like are exemplified and two or more of these may be combined.

For example, the compound of the present invention can be used in combination with other hormonal therapeutic agents, anti-cancer agents (e.g., chemotherapeutic agent, immunotherapeutic agent (including vaccine), antibody, gene therapeutic drug, pharmaceutical agent inhibiting action of cell growth factor and a receptor thereof, pharmaceutical agent inhibiting angiogenesis) and the like (hereinafter to be abbreviated as concomitant drug).

Although the compound of the present invention exhibits excellent anticancer action even when used as a simple agent, its effect can be enhanced by using it in combination with one or more of the concomitant drug(s) mentioned above (multi-agent co-administration).

As examples of said "hormonal therapeutic agents", there may be mentioned fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogens (e.g., tamoxifen citrate, toremifene citrate, and the like), ER down regulator (e.g., fulvestrant, and the like), human menopausal gonadotrophin, follicle stimulating hormone, pill preparations, mepitiostane, testrolactone, aminoglutethimide, LH-RH derivatives (LH-RH agonist (e.g., goserelin acetate, buserelin, leuprorelin, and the like), LH-RH antagonist), droloxifene, epitiostanol, ethinylestradiol sulfonate, aromatase inhibitors (e.g., fadrozole hydrochloride, anastrozole, retrozole, exemestane, vorozole, formestane, and the like), anti-androgens (e.g., flutamide, bicartamide, nilutamide, and the like), 5$\alpha$-reductase inhibitors (e.g., finasteride, dutasteride, epristeride, and the like), adrenocorticohormone drugs (e.g., dexamethasone, prednisolone, betamethasone, triamcinolone, and the like), androgen synthesis inhibitors (e.g., abiraterone, and the like), retinoid and drugs that retard retinoid metabolism (e.g., liarozole, and the like), etc. and LH-RH agonists (e.g., goserelin acetate, buserelin, leuprorelin) are preferable.

As examples of said "chemotherapeutic agents", there may be mentioned alkylating agents, antimetabolites, anticancer antibiotics, plant-derived anticancer agents, and the other chemotherapeutic agents.

As examples of the "alkylating agents", there may be mentioned nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and the like.

As examples of the "antimetabolites", there may be mentioned mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, pemetrexed sodium hydrate (Alimta (trade mark)) and the like.

As examples of the "anticancer antibiotics", there may be mentioned actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin,

mithramycin, sarcomycin, carzinophilin, mitotane, zorubicin hydrochloride, mitoxantrone hydrochloride, idarubicin hydrochloride, and the like.

As examples of the "plant-derived anticancer agents", there may be mentioned etoposide, etoposide phosphate, vinblastine sulfate, vincristine sulfate, vindesine sulfate, teniposide, paclitaxel (Taxol (trade mark)), docetaxel, vinorelbine, irinotecan, topotecan and the like.
As the "other chemotherapeutic agent", sobuzoxane and the like can be used.

As examples of said "immunotherapeutic agents (BRM)", there may be mentioned picibanil, krestin, sizofiran, lentinan, ubenimex, interferons, interleukins, macrophage colony-stimulating factor, granulocyte colony-stimulating factor, erythropoietin, lymphotoxin, *Corynebacterium parvum,* levamisole, polysaccharide K, procodazole, and the like. In addition, as the vaccine, BCG vaccine, PROVENGE, Onyvax-P, PROSTVAC-VF, GVAX, DCVax-Prostate, SAPOIMMUNE, VPM-4-001 and the like can be used.

As the "antibody", antibody to EpiCAM, antibody to PSCA, and antibody to PSMA can be used.

As examples of the "cell growth factor" in said "pharmaceutical agents inhibiting the action of cell growth factors or cell growth factor receptors", there may be mentioned any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin, TGF-$\alpha$, HB-EGF and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., acidic FGF, basic FGF, KGF (keratinocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGF$\beta$ (transforming growth factor $\beta$), HGF (hepatocyte growth factor), VEGF (vascular endothelial growth factor), and the like], and the like.

As examples of said "cell growth factor receptors", there may be mentioned any receptors capable of binding to the aforementioned cell growth factors, including EGF receptor, and a receptor belonging to the same family with that, HER2, HER3 and HER4, insulin receptor, IGF receptor, FGF receptor-1, FGF receptor-2 and the like.

As examples of said "pharmaceutical agents inhibiting the action of cell growth factor", there may be mentioned trastuzumab (Herceptin (trade mark) HER2 antibody), imatinib mesylate, ZD1839 or EGFR antibody (cetuximab (Erbitux (trade mark)) etc.), antibody against VEGF (e.g., bevacizumab (Avastin (trade mark))), VEGFR antibody, VEGFR inhibitor and EGFR inhibitor (gefitinib (Iressa (trade mark)), erlotinib (Tarceva (trade mark)) etc.).

In addition to the aforementioned drugs, L-asparaginase, aceglatone, procarbazine hydrochloride, protoporphyrin-cobalt complex salt, mercuric hematoporphyrin-sodium, topoisomerase I inhibitors (e.g., irinotecan, topotecan, and the like), topoisomerase II inhibitors (e.g., sobuzoxane, and the like), differentiation inducers (e.g., retinoid, vitamin D, and the like), angiogenesis inhibitors (e.g., thalidomide, SU11248, and the like), $\alpha$-blockers (e.g., tamsulosin hydrochloride, naftopidil, urapidil, alfuzosin, terazosin, prazosin, silodosin, and the like), serine/threonine kinase inhibitor, endothelin receptor antagonist (e.g., atrasentan, and the like), proteasome inhibitor (e.g., bortezomib, and the like), Hsp 90 inhibitor (e.g., 17-AAG, and the like), spironolactone, minoxidil, 11$\alpha$-hydroxyprogesterone, bone resorption inhibiting/metastasis suppressing agent (e.g., zoledronic acid, alendronic acid, pamidronic acid, etidronic acid, ibandronic acid, clodronic acid) and the like can be used.

Of those mentioned above, as the concomitant drug, LH-RH agonist (e.g., goserelin acetate, buserelin, leuprorelin, and the like), HER2 antibody (trastuzumab (Herceptin (trade mark))), EGFR antibody (cetuximab (Erbitux) (trade mark) etc.), EGFR inhibitor (erlotinib (Tarceva) (trade mark), gefitinib (Iressa (trade mark)) etc.), VEGFR inhibitor or chemotherapeutic agent (paclitaxel (Taxol (trade mark) etc.) are preferable.

Particularly, trastuzumab (Herceptin (trade mark)), cetuximab (Erbitux (trade mark)), erlotinib (Tarceva (trade mark)), gefitinib (Iressa (trade mark)), paclitaxel (Taxol (trade mark)) and the like are preferable.

In combination of the compound of the present invention and the concomitant drug, the administration time of the compound of the present invention and the concomitant drug is not restricted, and the compound of the present invention and the concomitant drug can be administered to the administration subject simultaneously, or may be administered at different times. The dosage of the concomitant drug may be determined according to the dose clinically used, and can be appropriately selected depending on the administration subject, administration route, disease, combination and the like.

The administration mode of the compound of the present invention and the concomitant drug is not particularly restricted, and it is sufficient that the compound of the present invention and the concomitant drug are combined in administration. Examples of such administration mode include the following methods:

(1) The compound of the present invention and the concomitant drug are simultaneously produced to give a single preparation which is administered. (2) The compound of the present invention and the concomitant drug are separately produced to give two kinds of preparations which are administered simultaneously by the same administration route. (3) The compound of the present invention and the concomitant drug are separately produced to give two

kinds of preparations which are administered by the same administration route at the different times. (4) The compound of the present invention and the concomitant drug are separately produced to give two kinds of preparations which are administered simultaneously by different administration routes. (5) The compound of the present invention and the concomitant drug are separately produced to give two kinds of preparations which are administered by different administration routes at different times (e.g., the compound of the present invention and the concomitant drug are administered in this order, or in the reverse order).

**Examples**

The present invention is explained in detail in the following by referring to Examples, Formulation Examples and Experimental Examples, which are not to be construed as limitative.

Example 1

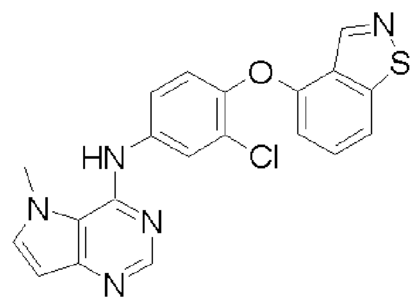

Production of N-[4-(1,2-benzoisothiazol-4-yloxy)-3-chlorophenyl]-5-methyl-5H-pyrrolo[3,2-d]pyrimidin-4-amine (i) Production of 2-(benzylsulfanyl)-6-methoxybenzaldehyde

To a suspension of sodium tert-butoxide (3.47 g) in tetrahydrofuran (50 mL) was added benzylmercaptan (4.24 mL) at 0°C, and the mixture was stirred for 30 min. A solution of 2-fluoro-6-methoxybenzaldehyde (4.64 g) in tetrahydrofuran (10 mL) was added dropwise to the reaction solution, and the mixture was stirred at room temperature for 1 hr. Water (150 mL) was added to the reaction solution, and the precipitated crystals were collected by filtration, washed with water and diethyl ether, and dried to give the title compound (7.08 g) as pale-yellow crystals.
$^1$H-NMR ($CDCl_3$) δ: 3.91 (3H, s), 4.15 (2H, s), 6.72 (1H, d, J = 8.3 Hz), 6.95 (1H, J = 8.3 Hz), 7.25 - 7.43 (6H, m), 10.59 (1H, s).

(ii) Production of 4-methoxy-1,2-benzoisothiazole

2-(Benzylsulfanyl)-6-methoxybenzaldehyde (18.8 g) and thioanisole (18 g) were dissolved in a mixed solvent of acetonitrile (175 mL) and water (175 mL), and the mixture was stirred at room temperature for 30 min. Hydroxylamine-O-sulfonic acid (12.4 g) was added, and the mixture was stirred at room temperature for 2 hr. Saturated aqueous sodium hydrogen carbonate (400 mL) was added to the reaction solution, and the mixture was extracted with ethyl acetate (500 mL). The organic layer was washed with water (250 mL) and dried over magnesium sulfate. The solvent was evaporated, and the obtained residue was purified by silica gel chromatography (eluent, hexane/ethyl acetate=100:0→30/70). The obtained fraction was concentrated under reduced pressure, and the precipitated crystals were collected by filtration to give the title compound (10.2 g) as white crystals.
$^1$H-NMR ($CDCl_3$) δ: 4.00 (3H, s), 6.75 (1H, dd, J = 7.4 Hz, 0.9 Hz), 7.35 - 7.62 (2H, m), 9.02 (1H, d, J = 0.8 Hz).

(iii) Production of 1,2-benzoisothiazol-4-ol

4-Methoxy-1,2-benzoisothiazole (22.0 g) and pyridine hydrochloride (140 g) were mixed in a solid state, and the mixture was stirred at 195°C for 2 hr. After cooling to room temperature, water (1 L), 1N hydrochloric acid (12 mL) and ethyl acetate (1 L) were added and the mixture was stirred. The organic layer was washed with water (500 mL) and saturated brine (200 mL) and dried over anhydrous magnesium sulfate. Anhydrous magnesium sulfate was filtered off,

and the filtrate was concentrated under reduced pressure. The obtained residue was purified by silica gel chromatography (eluent, hexane/ethyl acetate=100:0→0:100). The obtained fraction was concentrated under reduced pressure, and the precipitated crystals were collected by filtration to give the title compound (15.7 g) as white crystals.
$^1$H-NMR ($CDCl_3$) δ: 6.08 (1H, s), 6.74 (1H, dd, J = 8.0 Hz, 0.6 Hz), 7.38 (1H, t, J = 8.0 Hz), 7.51 (1H, dt, J = 8.0 Hz, 0.9 Hz), 9.06 (1H, d, J = 0.9 Hz).

(iv) Production of 4-(2-chloro-4-nitrophenoxy)-1,2-benzoisothiazole

To a solution of 2-chloro-1-fluoro-4-nitrobenzene (4.7 g) and 1,2-benzoisothiazol-4-ol (5.4 g) in N,N-dimethylformamide (50 mL) was added potassium carbonate (9.4 g), and the mixture was stirred at room temperature for 24 hr. Water (100 mL) was added to the reaction mixture, and the mixture was extracted with ethyl acetate (150 mL). The organic layer was washed with water (250 mL) and saturated brine (100 mL), and dried over anhydrous magnesium sulfate. Anhydrous magnesium sulfate was filtered off, and the filtrate was concentrated under reduced pressure. Crystallization from hexane and diethyl ether gave the title compound (9.6 g) as pale-yellow crystals.
$^1$H-NMR ($CDCl_3$) δ: 6.89 (1H, dd, J = 8.0, 0.6 Hz), 7.04 (1H, d, J = 9.1 Hz), 7.53 (1H, t, J = 8.0 Hz), 7.83 (1H, dd, J = 8.0, 0.8 Hz), 8.11 (1H, dd, J = 9.1, 2.6 Hz), 8.46 (1H, d, J = 2.6 Hz), 8.95 (1H, d, J = 0.9 Hz).

(v) Production of 4-(1,2-benzoisothiazol-4-yloxy)-3-chloroaniline

4-(2-Chloro-4-nitrophenoxy)-1,2-benzoisothiazole (9.6 g) was suspended in ethanol (150 mL), reduced iron (20 g) was added, and the mixture was heated at 80°. 1N Hydrochloric acid (5 mL) was added, and the mixture was heated under reflux for 5 hr. The reaction mixture was cooled to room temperature, 1N aqueous sodium hydroxide solution (15 mL) was added, and the reaction mixture was filtered through celite. The filtrate was concentrated under reduced pressure. The residue was diluted with ethyl acetate (400 mL), washed with water (200 mL), saturated aqueous sodium hydrogen carbonate (200 mL), 1N aqueous sodium hydroxide solution (100 mL) and saturated brine (100 mL), and dried over anhydrous magnesium sulfate. Anhydrous magnesium sulfate was filtered off, and the filtrate was concentrated under reduced pressure. The obtained residue was crystallized from hexane/diisopropyl ether to give the title compound (8.5 g) as pale-purple crystals.
$^1$H-NMR ($CDCl_3$) δ: 3.74 (2H, br s), 6.49 (1H, dd, J = 8.0, 0.6 Hz), 6.62 (1H, dd, J = 8.6, 2.6 Hz), 6.83 (1H, d, J = 2.6 Hz), 7.01 (1H, d, J = 8.6 Hz), 7.35 (1H, t, J = 8.0 Hz), 7.57 (1H, dt, J = 8.0, 0.8 Hz), 9.15 (1H, d, J = 0.9 Hz).

(vi) Production of N-[4-(1,2-benzoisothiazol-4-yloxy)-3-chlorophenyl]-5-methyl-5H-pyrrolo[3,2-d]pyrimidin-4-amine

A mixture of 4-chloro-5-methyl-5H-pyrrolo[3,2-d]pyrimidine (60 mg), 4-(1,2-benzoisothiazol-4-yloxy)-3-chloroaniline (100 mg) and isopropyl alcohol (5 mL) was stirred at 80°C overnight. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure and the residue was crystallized from ethyl acetate/diisopropyl ether to give the title compound (72 mg) as a white powder.
$^1$H-NMR (DMSO-$d_6$) δ: 4.21 (3H, s), 6.53 (1H, d, J = 3.0 Hz), 6.62 (1H, d, J = 8.0 Hz), 7.41 (1H, d, J = 8.6 Hz), 7.55 (1H, t, J = 8.0 Hz), 7.69-7.76 (2H, m), 7.94 (1H, d, J = 8.6 Hz), 8.01 (1H, d, J = 3.0 Hz), 8.46 (1H, s), 9.04 (1H, br s), 9.24 (1H, d, J = 0.9 Hz).
melting point: 229-231°C

Example 2

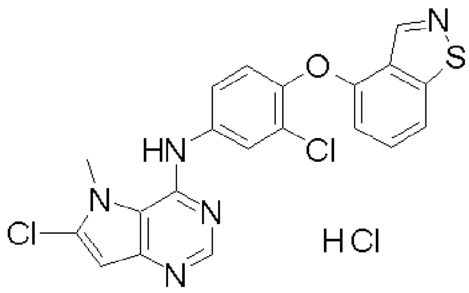

Production of N-[4-(1,2-benzoisothiazol-4-yloxy)-3-chlorophenyl]-6-chloro-5-methyl-5H-pyrrolo[3,2-d]pyrimidin-4-amine hydrochloride

In the same manner as in Example 1(vi), and using 4,6-dichloro-5-methyl-5H-pyrrolo[3,2-d]pyrimidine (68 mg), 4-(1,2-benzoisothiazol-4-yloxy)-3-chloroaniline (93 mg) and isopropyl alcohol (5 mL) and treating with 4N hydrochloric acid/ethyl acetate (1 mL), the title compound (41 mg) was obtained as a white powder.
$^1$H-NMR (DMSO-$d_6$) δ: 4.17 (3H, s), 6.66 (1H, d, J = 7.3 Hz), 6.90 (1H, s), 7.45 (1H, d, J = 8.8 Hz), 7.57 (1H, t, J = 8.0 Hz), 7.68 (1H, dd, J = 2.5, 8.8 Hz), 7.90-8.01 (2H, m), 8.69 (1H, s), 9.22 (1H, d, J = 0.9 Hz), 9.90 (1H, br s).

Example 3

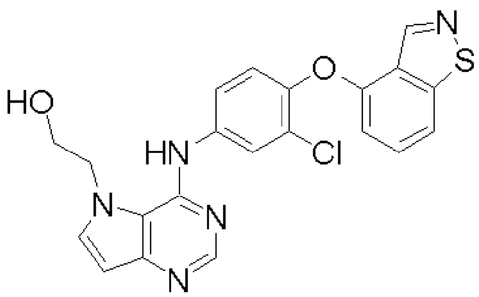

Production of 2-(4-{[4-(1,2-benzoisothiazol-4-yloxy)-3-chlorophenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (100 mg), 4-(1,2-benzoisothiazol-4-yloxy)-3-chloroaniline (92 mg) and isopropyl alcohol (7 mL) was stirred at 80°C overnight. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate: hexane=50:50→100:0→methanol:ethyl acetate=15:85). The object fraction was concentrated under reduced pressure. Methanol (5 mL) and 1N aqueous sodium hydroxide solution (1.0 mL) were added to the residue and the mixture was stirred overnight at room temperature. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure, and the residue was crystallized from ethyl acetate/diisopropyl ether to give the title compound (97 mg) as a white powder.
$^1$H-NMR (DMSO-$d_6$) δ: 3.89 (2H, br s), 4.56 (2H, t, J = 4.5 Hz), 6.34 (1H, br s), 6.48-6.63 (2H, m), 7.42 (1H, d, J = 8.8 Hz), 7.53 (1H, t, J = 8.0 Hz), 7.60-7.76 (2H, m), 7.92 (1H, d, J = 8.0 Hz), 8.05 (1H, d, J = 2.4 Hz), 8.38 (1H, s), 9.25 (1H, s), 9.99 (1H, s).
melting point: 218-220°C

Example 4

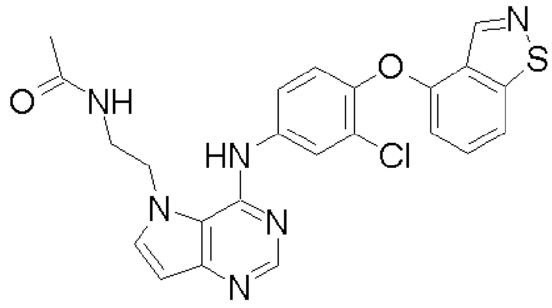

Production of N-[2-(4-{[4-(1,2-benzoisothiazol-4-yloxy)-3-chlorophenyl]amino}-5H-pyrrolo[3,2-d]pyrimidin-5-yl)ethyl] acetamide

(i) Production of 5-(2-aminoethyl)-N-[4-(1,2-benzoisothiazol-4-yloxy)-3-chlorophenyl]-5H-pyrrolo[3,2-d]pyrimidin-4-amine dihydrochloride

A mixture of tert-butyl [2-(4-chloro-5H-pyrrolo[3,2-d]pyrimidin-5-yl)ethyl]carbamate (2.9 g), 4-(1,2-benzoisothiazol-4-yloxy)-3-chloroaniline (2.7 g) and isopropyl alcohol (50 mL) was stirred at 80°C overnight. An aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was subjected to silica gel column chromatography (eluent, methanol: ethyl acetate=0:100→10:90). The object fraction was concentrated under reduced pressure. 4N Hydrochloric acid/ethyl acetate (15 mL) and ethanol (15 mL) were added to the residue, and the mixture was stirred at 80°C for 4 hr. Ethyl acetate was added to the reaction mixture, and the precipitate was collected by filtration to give the title compound (4.0 g) as a brown powder.
$^1$H-NMR (DMSO-$d_6$) δ: 3.27-3.37 (2H, m), 4.97-5.10 (2H, m), 6.67 (1H, d, J = 7.7 Hz), 6.75 (1H, d, J = 3.0 Hz), 7.44 (1H, d, J = 8.9 Hz), 7.58 (1H, t, J = 7.7 Hz), 7.63-7.74 (1H, m), 7.88-8.03 (2H, m), 8.06 (1H, d, J = 3.0 Hz), 8.29 (3H, br s), 8.74 (1H, s), 9.22 (1H, d, J = 0.8 Hz), 10.10 (1H, br s).

(ii) Production of N-[2-(4-{[4-(1,2-benzoisothiazol-4-yloxy)-3-chlorophenyl]amino}-5H-pyrrolo[3,2-d]pyrimidin-5-yl)ethyl] acetamide

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (560 mg), acetic acid (94 mg), triethylamine (1.5 mL), 1-hydroxybenzotriazole (200 mg), 1-ethyl-3-(3-dimethylaminopropyl)carbodiimide hydrochloride (1.0 g) and N,N-dimethylformamide (50 mL) was stirred overnight at room temperature. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure, and the residue was crystallized from ethyl acetate/diisopropyl ether to give the title compound (450 mg) as a white powder.
$^1$H-NMR (DMSO-$d_6$) δ: 1.80 (3H, s), 3.35-3.43 (2H, m), 4.52 (2H, br s), 6.52 (1H, d, J = 2.3 Hz), 6.60 (1H, d, J = 8.0 Hz), 7.40 (1H, d, J = 8.7 Hz), 7.54 (1H, t, J = 8.0 Hz), 7.66 (1H, d, J = 2.3 Hz), 7.82 (1H, dd, J = 2.3, 8.7 Hz), 7. 92 (1H, d, J = 8.0 Hz), 8.10 (1H, d, J = 2.3 Hz), 8.27 (1H, br s), 8.36 (1H, s), 8.84 (1H, s), 9.25 (1H, s).
melting point: 222-224°C

Example 5

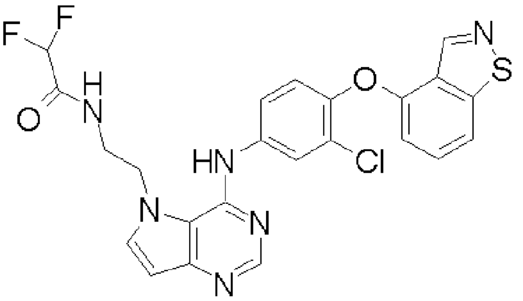

Production of N-[2-(4-{[4-(1,2-benzoisothiazol-4-yloxy)-3-chlorophenyl]amino}-5H-pyrrolo[3,2-d]pyrimidin-5-yl)ethyl]-2,2-difluoroacetamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (200 mg), difluoroacetic acid (240 mg), N,N-dimethylformamide (20 mL), triethylamine (2.0 mL), 1-hydroxybenzotriazole (20 mg) and 1-ethyl-3-(3-dimethylaminopropyl)carbodiimide hydrochloride (450 mg), and by a reaction in the same manner as in Example 4(ii), the title compound (143 mg) was obtained as a white powder.
$^1$H-NMR (DMSO-$d_6$) δ: 3.44-3.56 (2H, m), 4.56-4.73 (2H, m), 6.12 (1H, t, J = 53.6 Hz), 6.52 (1H, d, J = 2.7 Hz), 6.61 (1H, d, J = 8.0 Hz), 7.40 (1H, d, J = 9.1 Hz), 7.47-7.64 (2H, m), 7.67-7.77 (1H, m), 7.86-8.06 (2H, m), 8.38 (1H, s), 8.68 (1H, s), 8.91 (1H, br s), 9.25 (1H, s).

Example 6

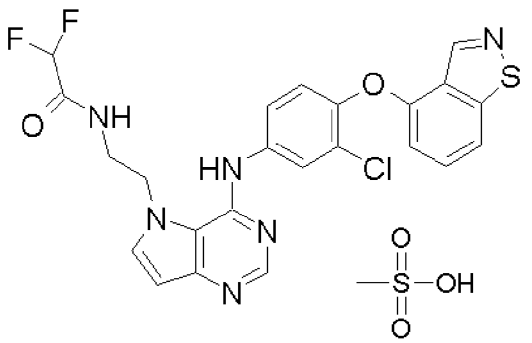

Production of N-[2-(4-{[4-(1,2-benzoisothiazol-4-yloxy)-3-chlorophenyl]amino}-5H-pyrrolo[3,2-d]pyrimidin-5-yl)ethyl]-2,2-difluoroacetamido methanesulfonate

N-[2-(4-{[4-(1,2-Benzoisothiazol-4-yloxy)-3-chlorophenyl]amino}-5H-pyrrolo[3,2-d]pyrimidin-5-yl)ethyl]-2,2-difluoroacetamide (150 mg) was dissolved in ethyl acetate (10 mL), and methanesulfonic acid (29 mg) was added. Diethyl ether (1 mL) was added, and the precipitated powder was collected by filtration to give the title compound (155 mg) as a white powder.
$^1$H-NMR (DMSO-$d_6$) δ: 2.32 (3H, s), 3.53-3.60 (2H, m), 4.77 (2H, br s), 6.14 (1H, t, J = 54.0 Hz), 6.63-6.71 (2H, m), 7.47 (1H, d, J = 8.7 Hz), 7.50-7.75 (2H, m), 7.91-8.03 (3H, m), 8.83-8.99 (1H, m), 9.15-9.26 (1H, m), 9.77 (1H, br s).

Example 7

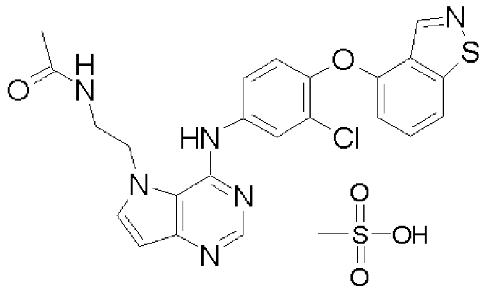

Production of N-[2-(4-{[4-(1,2-benzoisothiazol-4-yloxy)-3-chlorophenyl]amino}-5H-pyrrolo[3,2-d]pyrimidin-5-yl)ethyl] acetamide methanesulfonate

N-[2-(4-{[4-(l,2-Benzoisothiazol-4-yloxy)-3-chlorophenyl]amino}-5H-pyrrolo[3,2-d]pyrimidin-5-yl)ethyl]acetamide (50 mg) was dissolved in ethyl acetate (5 mL), and methanesulfonic acid (10 mg) was added. Diethyl ether (1 mL) was added, and the precipitated powder was collected by filtration to give the title compound (52 mg) as a white powder.
$^1$H-NMR (DMSO-$d_6$) δ: 1.81 (3H, s), 2.29 (3H, s), 3.40-3.48 (2H, m), 4.47-4.70 (2H, m), 6.60-6.72 (2H, m), 7.44 (1H, d, J = 8.7 Hz), 7.58 (1H, t, J = 7.9 Hz), 7.67-7.75 (1H, m), 7.88-8.06 (3H, m), 8.37 (1H, s), 8.68 (1H, s), 9.23 (1H, d, J = 0.9 Hz), 9.89 (1H, br s).

Example 8

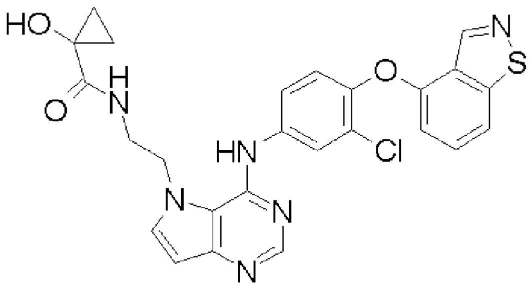

Production of N-[2-(4-{[4-(1,2-benzoisothiazol-4-yloxy)-3-chlorophenyl]amino}-5H-pyrrolo[3,2-d]pyrimidin-5-yl)ethyl]-1-hydroxycyclopropanecarboxamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 1-hydroxycyclopropanecarboxylic acid (50 mg), N,N-dimethylformamide (10 mL), triethylamine (0.8 mL), 1-hydroxybenzotriazole (10 mg) and 1-ethyl-3-(3-dimethylaminopropyl)carbodiimide hydrochloride (300 mg), and by a reaction in the same manner as in Example 4(ii), the title compound (61 mg) was obtained as a white powder.
$^1$H-NMR (DMSO-$d_6$) δ: 0.78-0.90 (2H, m), 0.95-1.04 (2H, m), 3.45-3.60 (2H, m), 4.56 (2H, br s), 6.28 (1H, br s), 6.51 (1H, d, J = 3.0 Hz), 6.60 (1H, d, J = 8.0 Hz), 7.38 (1H, d, J = 8.9 Hz), 7.54 (1H, t, J = 8.0 Hz), 7.63 (1H, d, J = 3.0 Hz), 7.85 (1H, dd, J = 2.5, 8. 9 Hz), 7. 92 (1H, d, J = 8.0 Hz), 8.06 (1H, d, J = 2.5 Hz), 8.35 (2H, br s), 8.85 (1H, s), 9.25 (1H, d, J = 0.8 Hz).

Example 9

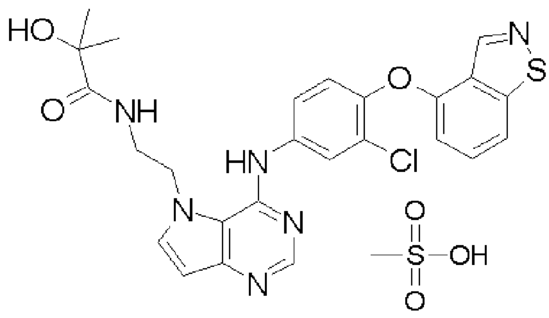

Production of N-[2-(4-{[4-(1,2-benzoisothiazol-4-yloxy)-3-chlorophenyl]amino}-5H-pyrrolo[3,2-d]pyrimidin-5-yl)ethyl]-2-hydroxy-2-methylpropanamide methanesulfonate

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 2-hydroxy-2-methylpropanoic acid (50 mg), N,N-dimethylformamide (10 mL), triethylamine (0.5 mL), 1-hydroxybenzotriazole (10 mg) and 1-ethyl-3-(3-dimethylaminopropyl)carbodiimide hydrochloride (300 mg), a reaction in the same manner as in Example 4(ii) was performed. Then, in the same manner as in Example 7 and by treating with methanesulfonic acid (20 mg), the title compound (55 mg) was obtained as a white powder.
$^1$H-NMR (DMSO-$d_6$) δ: 1.12 (6H, s), 2.31 (3H, s), 3.47-3.58 (2H, m), 4.59-4.77 (2H, m), 6.61-6.77 (2H, m), 7.47 (1H, d, J = 8.9 Hz), 7.59 (1H, t, J = 8.0 Hz), 7.71 (1H, dd, J = 2.5, 8.9 Hz), 7.89-8.14 (4H, m), 8.76 (1H, s), 9.22 (1H, d, J = 0.9 Hz), 10.00 (1H, s).

Example 10

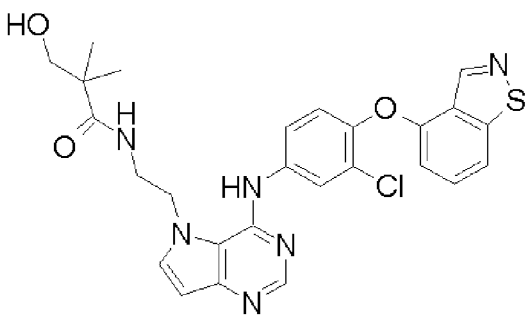

Production of N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-2,2-dimethylpropanamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 3-hydroxy-2,2-dimethylpropanoic acid (50 mg), N,N-dimethylformamide (10 mL), triethylamine (0.8 mL), 1-hydroxybenzotriazole (10 mg) and 1-ethyl-3-(3-dimethylaminopropyl)carbodiimide hydrochloride (200 mg), and by a reaction in the same manner as in Example 4(ii), the title compound (55 mg) was obtained as a pale-yellow powder. $^1$H-NMR (DMSO-$d_6$) δ: 0.98 (6H, s), 3.33 (2H, s), 3.39-3.53 (2H, m), 4.53 (2H, br s), 4.85 (1H, br s), 6.50 (1H, br s), 6.61
(1H, d, J = 7.7 Hz), 7.39 (1H, d, J = 8.9 Hz), 7.44-7.70 (2H, m), 7.81-8.02 (3H, m), 8.16 (1H, br s), 8.36 (1H, s), 8.96 (1H, br s), 9.25 (1H, d, J = 0.9 Hz).

Example 11

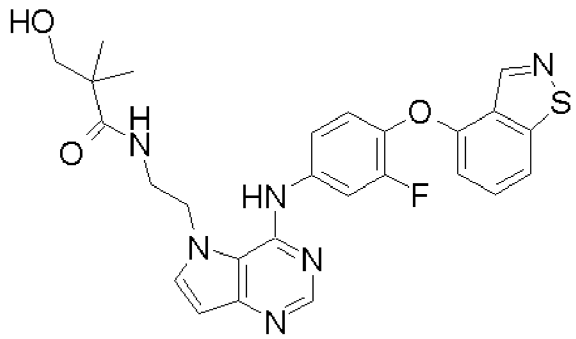

Production of N-[2-(4-{[4-(1,2-benzoisothiazol-4-yloxy)-3-fluorophenyl]amino}-5H-pyrrolo[3,2-d]pyrimidin-5-yl)ethyl]-3-hydroxy-2,2-dimethylpropanamide

(i) Production of 4-(1,2-benzoisothiazol-4-yloxy)-3-fluoroaniline

To a solution of 1,2-difluoro-4-nitrobenzene (1.5 g) and 1,2-benzoisothiazol-4-ol (1.5 g) in N,N-dimethylformamide (50 mL) was added potassium carbonate (1.5 g), and the mixture was stirred at room temperature for 12 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=19:1→4:1). The obtained residue was dissolved in ethanol (70 mL), reduced iron (4.0 g) and 1N hydrochloric acid (5.0 mL) was added, and the mixture was heated under reflux at 80°C for 1 hr. The reaction mixture was cooled to room temperature, 1N aqueous sodium hydroxide solution (8 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane:ethyl acetate=49:1→1:1) to give the title compound (2.1 g) as a pale-purple powder. $^1$H-NMR (DMSO-$d_6$) δ: 5.45 (2H, s), 6.37-6.60 (3H, m), 6.99-7.16 (1H, m), 7.48 (1H, t, J = 7.9 Hz), 7.84 (1H, d, J = 8.1 Hz), 9.21 (1H, d, J = 0.9 Hz) .

(ii) Production of 5-(2-aminoethyl)-N-[4-(1,2-benzoisothiazol-4-yloxy)-3-fluorophenyl]-5H-pyrrolo[3,2-d]pyrimidin-4-amine dihydrochloride

Using tert-butyl [2-(4-chloro-5H-pyrrolo[3,2-d]pyrimidin-5-yl)ethyl]carbamate (300 mg), 4-(1,2-benzoisothiazol-4-yloxy)-3-fluoroaniline (265 mg), isopropyl alcohol (20 mL), ethanol (40 mL) and 4N hydrochloric acid/ethyl acetate (10 mL), by a reaction in the same manner as in Example 4(i), the title compound (380 mg) was obtained as a yellow powder. $^1$H-NMR (DMSO-$d_6$) δ: 3.23-3.35 (2H, m), 5.02 (2H, t, J = 6.3 Hz), 6.69-6.80 (2H, m), 7.47 (1H, t, J = 8.9 Hz), 7.52-7.60 (1H, m), 7.58 (1H, t, J = 8.0 Hz), 7.82 (1H, dd, J = 1.9 Hz, 12.3 Hz), 7.98 (1H, d, J = 8.0 Hz), 8.06 (1H, d, J = 3.0 Hz), 8.27 (3H, br s), 8.75 (1H, s), 9.25 (1H, d, J = 0.8 Hz), 10.12 (1H, br s).

(iii) Production of N-[2-(4-{[4-(1,2-benzoisothiazol-4-yloxy)-3-fluorophenyl]amino}-5H-pyrrolo[3,2-d]pyrimidin-5-yl)ethyl]-3-hydroxy-2,2-dimethylpropanamide

Using 5-(2-aminoethyl)-N-[4-(1,2-benzoisothiazol-4-yloxy)-3-fluorophenyl]-5H-pyrrolo[3,2-d]pyrimidin-4-amine dihydrochloride (100 mg), 3-hydroxy-2,2-dimethylpropanoic acid (50 mg), N,N-dimethylformamide (20 mL), triethylamine (0.8 mL), 1-hydroxybenzotriazole (10 mg) and 1-ethyl-3-(3-dimethylaminopropyl)carbodiimide hydrochloride (250 mg), and by a reaction in the same manner as in Example 4(ii), the title compound (70 mg) was obtained as a pale-yellow powder.
$^1$H-NMR (DMSO-$d_6$) δ: 0.97 (6H, s), 3.32 (2H, s), 3.41 (2H, q, J = 6.6 Hz), 4.52 (2H, t, J = 6.6 Hz), 4.84 (1H, t, J = 5.4 Hz), 6.50 (1H, d, J = 3.2 Hz), 6.70 (1H, d, J = 7.7 Hz), 7.40 (1H, t, J = 9.1 Hz), 7.54 (1H, t, J = 7.7 Hz), 7.62 (1H, d, J = 3.2 Hz), 7.66-7.79 (1H, m), 7.81-8.06 (3H, m), 8.37 (1H, s), 8.95 (1H, s), 9.27 (1H, d, J = 0.9 Hz).

Example 12

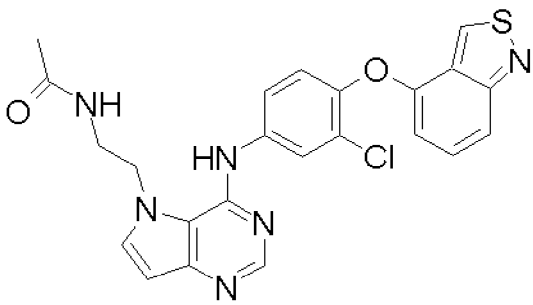

Production of N-[2-(4-{[4-(2,1-benzoisothiazol-4-yloxy)-3-chlorophenyl]amino}-5H-pyrrolo[3,2-d]pyrimidin-5-yl)ethyl] acetamide

(i) Production of 4-(2,1-benzoisothiazol-4-yloxy)-3-chloroaniline

To a solution of 2-chloro-1-fluoro-4-nitrobenzene (500 mg) and 2,1-benzoisothiazol-4-ol (300 mg) in N,N-dimethylformamide (20 mL) was added potassium carbonate (420 mg), and the mixture was stirred at room temperature for 12 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=19:1→4:1), and the obtained residue was dissolved in ethanol (25 mL). Reduced iron (1.5 g) and 1N hydrochloric acid (2.0 mL) were added and the mixture was heated under reflux at 80°C for 1 hr. The reaction mixture was cooled to room temperature, 1N aqueous sodium hydroxide solution (4 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane: ethyl acetate=49:1→1:1) to give the title compound (260 mg) as a pale-purple powder.
$^1$H-NMR ($CDCl_3$) δ: 3.70 (2H, br s), 6.19 (1H, d, J = 7.2 Hz), 6.62 (1H, dd, J = 2.7 Hz, 8.7 Hz), 6.83 (1H, d, J = 2.7 Hz), 7.01 (1H, d, J = 8.7 Hz), 7.26-7.31 (1H, m), 7.50 (1H, d, J = 8.7 Hz), 9.48 (1H, d, J = 0.9 Hz).

(ii) Production of N-[2-(4-{[4-(2,1-benzoisothiazol-4-yloxy)-3-chlorophenyl]amino}-5H-pyrrolo[3,2-d]pyrimidin-5-yl)ethyl] acetamide

Using tert-butyl [2-(4-chloro-5H-pyrrolo[3,2-d]pyrimidin-5-yl)ethyl]carbamate (100 mg), 4-(2,1-benzoisothiazol-4-yloxy)-3-chloroaniline (90 mg), isopropyl alcohol (7 mL), ethanol (5 mL) and 4N hydrochloric acid (3 mL), and by a reaction in the same manner as in Example 4(i), and using the obtained pale-yellow powder, acetic acid (34 mg), N,N-dimethylformamide (5 mL), triethylamine (0.5 mL), 1-hydroxybenzotriazole (2 mg) and 1-ethyl-3-(3-dimethylaminopropyl) carbodiimide hydrochloride (100 mg), and by a reaction in the same manner as in Example 4(ii), the title compound (16 mg) was obtained as a white powder.
$^1$H-NMR (DMSO-$d_6$) δ: 1.81 (3H, s), 3.32-3.45 (2H, m), 4.51 (2H, br s), 6.51 (1H, d, J = 2.7 Hz), 6.61 (1H, d, J = 8.0 Hz), 7.42 (1H, d, J = 8.7 Hz), 7.51 (1H, t, J = 8.0 Hz), 7.58-7.67 (1H, m), 7.80-8.29 (4H, m), 8.34 (1H, br s), 8.85 (1H, s), 9.23 (1H, br s).

Example 13

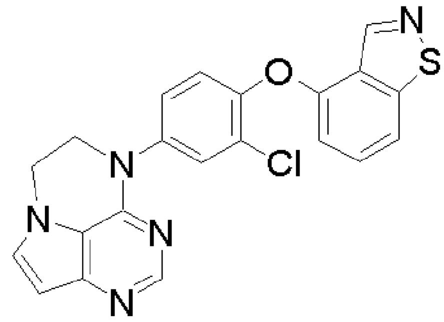

Production of 4-[4-(1,2-benzoisothiazol-4-yloxy)-3-chlorophenyl]-5,6-dihydro-4H-pyrrolo[3,2,1-de]pteridine

A mixture of 4-chloro-5-(2-chloroethyl)-5H-pyrrolo[3,2-d]pyrimidine (50 mg), 4-(1,2-benzoisothiazol-4-yloxy)-3-chloroaniline (70 mg) and isopropyl alcohol (5 mL) was stirred at 80°C for 2 hr. 1-Methyl-2-pyrrolidone (5 mL) and potassium carbonate (50 mg) were added to the reaction mixture, and the mixture was stirred at 100°C for 2 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, hexane:ethyl acetate=50:50→0:100), and the object fraction was concentrated under reduced pressure to give the title compound (37 mg) as a yellow powder.
$^1$H-NMR (DMSO-$d_6$) δ: 4.25-4.35 (2H, m), 4.36-4.53 (2H, m), 6.42-6.54 (1H, m), 6.65 (1H, d, J = 7.9 Hz), 7.40-7.71 (4H, m), 7.87-7.98 (2H, m), 8.31 (1H, s), 9.24 (1H, s).

Example 14

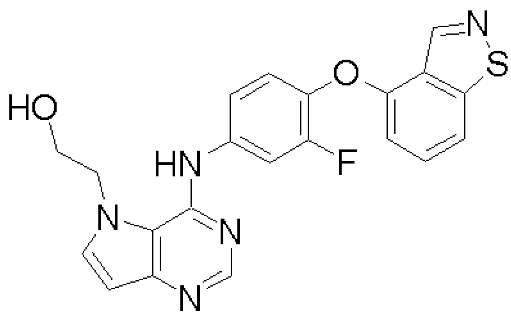

Production of 2-(4-{[4-(1,2-benzoisothiazol-4-yloxy)-3-fluorophenyl]amino}-5H-pyrrolo[3,2-d]pyrimidin-5-yl)ethanol

Using 2-(4-chloro-5H-pyrrolo[3,2-d]pyrimidin-5-yl)ethyl benzoate (100 mg), 4-(1,2-benzoisothiazol-4-yloxy)-3-fluoroaniline (86 mg), isopropyl alcohol (10 mL) and 1N aqueous sodium hydroxide solution (5.0 mL), and by a reaction in the same manner as in Example 3, the title compound (100 mg) was obtained as a white powder.
$^1$H-NMR (DMSO-$d_6$) δ: 3.89 (2H, t, J = 4.3 Hz), 4.46-4.64 (2H, m), 6.52 (1H, d, J = 3.2 Hz), 6.67 (1H, d, J = 7.7 Hz), 7.38-7.46 (2H, m), 7.53 (1H, t, J = 7.7 Hz), 7.67 (1H, d, J = 3.2 Hz), 7.82-8.03 (2H, m), 8.36 (1H, s), 9.27 (1H, s), 9.97 (1H, br s).

Example 15

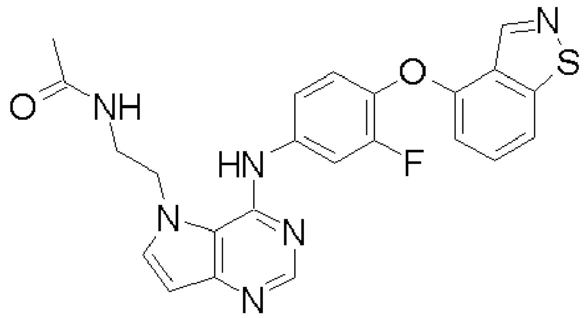

Production of N-[2-(4-{[4-(1,2-benzoisothiazol-4-yloxy)-3-fluorophenyl]amino}-5H-pyrrolo[3,2-d]pyrimidin-5-yl)ethyl] acetamide

Using 5-(2-aminoethyl)-N-[4-(1,2-benzoisothiazol-4-yloxy)-3-fluorophenyl]-5H-pyrrolo[3,2-d]pyrimidin-4-amine dihydrochloride (100 mg), acetic acid (0.4 mL), N,N-dimethylformamide (10 mL), triethylamine (0.5 mL), 1-hydroxybenzotriazole (10 mg) and 1-ethyl-3-(3-dimethylaminopropyl)carbodiimide hydrochloride (200 mg), and by a reaction in the same manner as in Example 4(ii), the title compound (71 mg) was obtained as a pale-yellow powder.
$^1$H-NMR (DMSO-$d_6$) δ: 1.81 (3H, s), 3.36-3.43 (2H, m), 4.52 (2H, t, J = 6.9 Hz), 6.52 (1H, d, J = 2.6 Hz), 6.69 (1H, d, J = 7.7 Hz), 7.40 (1H, t, J = 9.1 Hz), 7.55 (1H, t, J = 8.0 Hz), 7.66 (2H, br s), 7.88-8.03 (2H, m), 8.22-8.45 (2H, m), 8.86 (1H, br s), 9.27 (1H, s).

Example 16

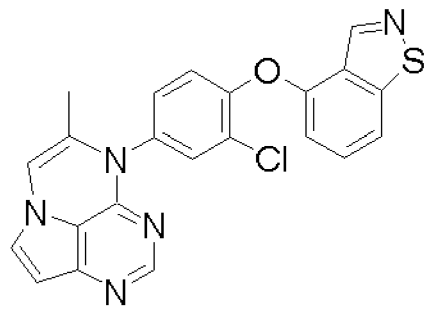

Production of 4-[4-(1,2-benzoisothiazol-4-yloxy)-3-chlorophenyl]-5-methyl-4H-pyrrolo[3,2,1-de]pteridine

A mixture of 1-(4-chloro-5H-pyrrolo[3,2-d]pyrimidin-5-yl)acetone (200 mg) and 4-(1,2-benzoisothiazol-4-yloxy)-3-chloroaniline (264 mg) was dissolved in isopropyl alcohol (10 mL), and the mixture was stirred at 80°C for 8 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane: ethyl acetate=50:50→0:100) to give the title compound (41 mg) as a pale-yellow powder.
$^1$H-NMR (DMSO-$d_6$) δ: 1.66 (3H, s), 6.28 (1H, d, J = 2.8 Hz), 6.74-6.85 (2H, m), 7.36-7.65 (4H, m), 7.79-7.91 (2H, m), 8.02 (1H, d, J = 7.7 Hz), 9.20 (1H, s).

Example 17

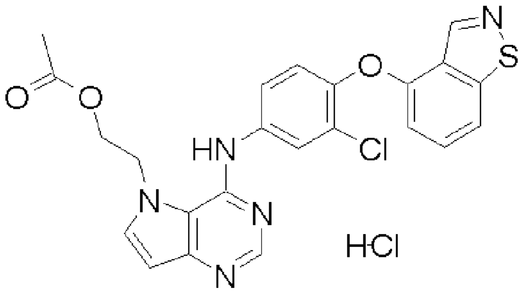

Production of 2-(4-{[4-(1,2-benzoisothiazol-4-yloxy)-3-chlorophenyl]amino}-5H-pyrrolo[3,2-d]pyrimidin-5-yl)ethylacetate hydrochloride

To a solution of 2-(4-{[4-(1,2-benzoisothiazol-4-yloxy)-3-chlorophenyl]amino}-5H-pyrrolo[3,2-d]pyrimidin-5-yl) ethanol (100 mg) in tetrahydrofuran (5 mL) were added acetic anhydride (0.2 mL) and triethylamine (1.6 mL), and the mixture was stirred for 1 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was dissolved in triethylamine (1 mL), and the mixture was stirred for 1 hr. Triethylamine was evaporated, and the residue was separated and purified by silica gel column chromatography (hexane:ethyl acetate=50:50→0:100) and the residue was treated with 4N hydrochloric acid/ ethyl acetate (1 mL) to give the title compound (81 mg) as a pale-yellow powder.
$^1$H-NMR (DMSO-$d_6$) δ: 1.88 (3H, s), 4.30-4.39 (2H, m), 4.91-5.03 (2H, m), 6.61-6.77 (2H, m), 7.41-7.73 (3H, m), 7.92-8.13 (3H, m), 8.67-8.81 (1H, m), 9.22 (1H, s), 9.85 (1H, br s).

Example 18

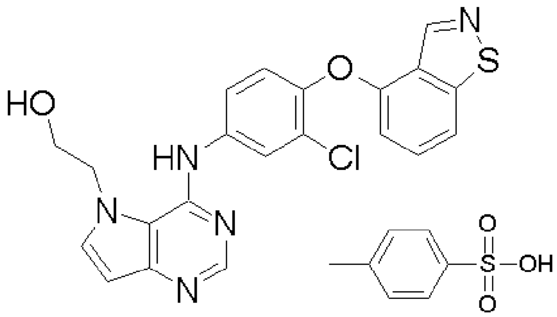

Production of 2-(4-{[4-(1,2-benzoisothiazol-4-yloxy)-3-chlorophenyl]amino}-5H-pyrrolo[3,2-d]pyrimidin-5-yl)ethanol p-toluenesulfonate

2-(4-{[4-(1,2-Benzoisothiazol-4-yloxy)-3-chlorophenyl]amino}-5H-pyrrolo[3,2-d]pyrimidin-5-yl)ethanol (700 mg) was dissolved in ethanol (10 mL), and p-toluenesulfonic acid monohydrate (310 mg) was added. Ethyl acetate (5 mL) was added, and the precipitated crystals were collected by filtration to give the title compound (892 mg) as a white powder.
$^1$H-NMR (DMSO-$d_6$) δ: 2.28 (3H, s), 3.92 (2H, t, J = 4.4 Hz), 4.61-4.82 (2H, m), 6.65 (1H, d, J = 7.7 Hz), 6.72 (1H, d, J = 3.0 Hz), 7.11 (2H, d, J = 7.9 Hz), 7.38-7.73 (5H, m), 7.92-8.07 (3H, m), 8.81 (1H, s), 9.23 (1H, d, J = 0.8 Hz), 10.88 (1H, s).

Example 19

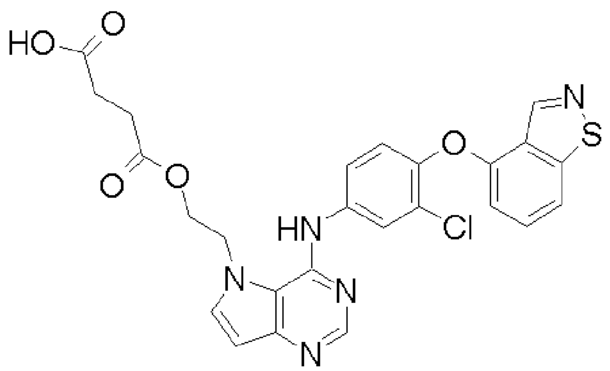

Production of 4-[2-(4-{[4-(1,2-benzoisothiazol-4-yloxy)-3-chlorophenyl]amino}-5H-pyrrolo[3,2-d]pyrimidin-5-yl)ethoxy]-4-oxobutanoic acid

To a solution of 2-(4-{[4-(1,2-benzoisothiazol-4-yloxy)-3-chlorophenyl]amino}-5H-pyrrolo[3,2-d]pyrimidin-5-yl) ethanol (100 mg) in 1-methyl-2-pyrrolidone (5 mL) were added dihydrofuran-2,5-dione (25 mg) and triethylamine (0.6 mL), and the mixture was stirred at 60°C for 1 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (ethyl acetate:methanol=100:0→90:10) to give the title compound (40 mg) as a pale-yellow powder.
$^1$H-NMR (DMSO-$d_6$) δ: 2.32 (4H, br s), 4.31 (2H, br s), 4.79 (2H, br s), 6.53 (1H, d, J = 2.5 Hz), 6.60 (1H, d, J = 7.9 Hz), 7.38 (1H, d, J = 8.7 Hz), 7.53 (1H, t, J = 7.9 Hz), 7.70 (2H, br s), 7.92 (1H, d, J = 8.7 Hz), 7.99 (1H, d, J = 2.5 Hz), 8.37 (1H, s), 8.69 (1H, br s), 9.25 (1H, d, J = 0.8 Hz).

Example 20

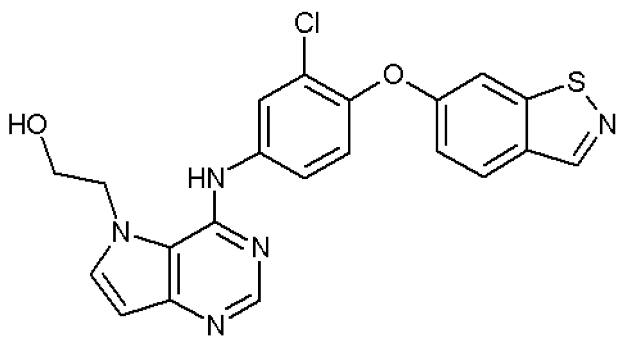

Production of 2-(4-{[4-(1,2-benzoisothiazol-6-yloxy)-3-chlorophenyl]amino}-5H-pyrrolo[3,2-d]pyrimidin-5-yl)ethanol (i) Production of 6-methoxy-1,2-benzoisothiazole

A mixture of 2-fluoro-4-methoxybenzaldehyde (5.0 g), sulfur (1.04 g), 28% aqueous ammonia solution (25 mL) and 2-methoxyethanol (25 mL) was heated in a sealed tube to 160°C, and the mixture was stirred for 4 hr. After cooling to room temperature, the reaction mixture was poured into water, and the mixture was extracted with ethyl acetate. The extract was dried over anhydrous magnesium sulfate and concentrated under reduced pressure. The residue was separated and purified by silica gel column chromatography (hexane:ethyl acetate=97:3→80:20) to give the title compound (1.0 g) as a yellow oil.
$^1$H-NMR ($CDCl_3$) δ: 3.92 (3H, s), 7.05 (1H, dd, J= 2.1 Hz, 8.7 Hz), 7.34 (1H, m), 7.92 (1H, d, J= 8.7 Hz), 8.77 (1H, m).

(ii) Production of 1,2-benzoisothiazol-6-ol

A mixture of 6-methoxy-1,2-benzoisothiazole (990 mg) and 47% hydrobromic acid (10 g) was heated to 100°C and the mixture was stirred for 4 hr. After cooling to room temperature, the reaction mixture was poured into water and extracted with ethyl acetate. The extract was dried over anhydrous magnesium sulfate and concentrated under reduced pressure. The residue was separated and purified by silica gel column chromatography (hexane:ethyl acetate=97:3→80:20) to give the title compound (204 mg) as a colorless solid.
$^1$H-NMR ($CDCl_3$) δ: 5.41 (1H, br s), 6.99 (1H, dd, J= 2.4 Hz, 8.7 Hz), 7.33 (1H, m), 7.93 (1H, d, J= 8.7 Hz), 8.77 (1H, m).

(iii) Production of 4-(1,2-benzoisothiazol-6-yloxy)-3-chloroaniline

A mixture of 1,2-benzoisothiazol-6-ol (203 mg), N,N-dimethylformamide (5 mL), potassium carbonate (185 mg) and 2-chloro-1-fluoro-4-nitrobenzene (236 mg) was stirred overnight at room temperature. The reaction mixture was poured into water, and the mixture was extracted with ethyl acetate. The extract was washed with aqueous sodium hydrogen carbonate, dried over anhydrous magnesium sulfate, and concentrated under reduced pressure. Reduced iron (420 mg), 1N hydrochloric acid (1.2 mL) and ethanol (15 mL) were added to the residue, and the mixture was heated to 80°C and stirred for 2 hr. The reaction mixture was cooled to room temperature, filtered through celite, and the celite was washed with ethanol. The filtrate was concentrated under reduced pressure, and ethyl acetate, water and a small amount of methanol were added to the residue to allow partitioning. The organic layer was dried over anhydrous magnesium sulfate and concentrated under reduced pressure. The residue was separated and purified by silica gel column chromatography (hexane:ethyl acetate=95:5→0:100) to give the title compound (294 mg) as a yellow oil.
$^1$H-NMR ($CDCl_3$) δ: 3.74 (2H, br s), 6.62 (1H, dd, J= 2.4 Hz, 8.4 Hz), 6.82 (1H, d, J= 2.4 Hz), 6.98 (1H, d, J= 8.4 Hz), 7.12-7.20 (2H, m), 7.97 (1H, d, J= 8.4 Hz), 8.79 (1H, d, J= 0.9 Hz).

(iv) Production of 2-(4-{[4-(1,2-benzoisothiazol-6-yloxy)-3-chlorophenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (151 mg), 4-(1,2-benzoisothiazol-6-yloxy)-3-chloroaniline (139 mg), isopropyl alcohol (5 mL) and pyridine hydrochloride (5 mg) was heated to 80°C, and the mixture was stirred overnight. The reaction mixture was poured into aqueous sodium hydrogen carbonate, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a coupling compound. Tetrahydrofuran (1 mL), methanol (1 mL) and 1N aqueous sodium hydroxide solution (1 mL) were added to the obtained coupling compound and the mixture was stirred over 30 min. The reaction mixture was poured into aqueous sodium hydrogen carbonate, and the mixture was extracted with ethyl acetate. The extract was dried over anhydrous magnesium sulfate, concentrated under reduced pressure, and crystallized from ethyl acetate to give the title compound (130 mg) as white crystals.
$^1$H-NMR (DMSO-$d_6$) δ: 3.86-3.90 (2H, m), 4.53-4.56 (2H, m), 6.33 (1H, br s), 6.52 (1H, d, J= 3.3 Hz), 7.23 (1H, dd, J= 2.1 Hz, 8.4 Hz), 7.35 (1H, d, J= 9.0 Hz), 7.63-7.68 (3H, m), 8.02 (1H, d, J= 2.4 Hz), 8.20 (1H, d, J= 8.7 Hz), 8.35 (1H, s), 9.03 (1H, s), 9.94 (1H, br s).

Example 21

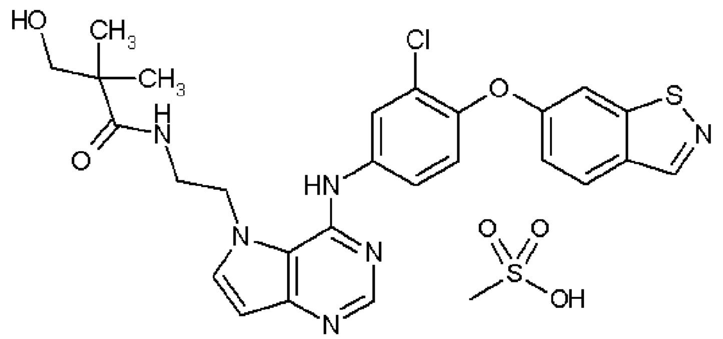

Production of N-[2-(4-{[4-(1,2-benzoisothiazol-6-yloxy)-3-chlorophenyl]amino}-5H-pyrrolo[3,2-d]pyrimidin-5-yl)ethyl]-3-hydroxy-2,2-dimethylpropanamide methanesulfonate

A mixture of tert-butyl [2-(4-chloro-5H-pyrrolo[3,2-d]pyrimidin-5-yl)ethyl]carbamate (168 mg), 4-(1,2-benzoisothiazol-6-yloxy)-3-chloroaniline (156 mg), isopropyl alcohol (5 mL) and pyridine hydrochloride (5 mg) was stirred at 80°C overnight. The reaction mixture was concentrated under reduced pressure, methanol (2 mL) and 4N hydrochloric acid/ethyl acetate solution (2 mL) were added to the residue, and the mixture was stirred at room temperature for 2 hr. The reaction mixture was concentrated under reduced pressure, ethanol/ethyl acetate was added, and the precipitate (156 mg) was collected by filtration. A mixture of the precipitate, triethylamine (95 μL) and N,N-dimethylformamide (5 mL) was stirred at room temperature for 5 min. 3-Hydroxy-2,2-dimethylpropanoic acid (43 mg), 1-hydroxybenzotriazole (50 mg) and 1-ethyl-3-(3-dimethylaminopropyl)carbodiimide hydrochloride (70 mg) were successively added, and the mixture was stirred at room temperature for 5 hr. The reaction mixture was poured into aqueous sodium hydrogen carbonate, and the mixture was extracted with ethyl acetate. The extract was dried over anhydrous magnesium sulfate and concentrated under reduced pressure. The residue was separated and purified by silica gel column chromatography (ethyl acetate:methanol=100:0→80:20) to give a purified compound (113 mg). The obtained purified compound was dissolved in ethanol (3 mL), and methanesulfonic acid (14 μL) was added. After concentration under reduced pressure, the residue was crystallized from ethanol/ethyl acetate to give the title compound (118 mg) as a white solid.
$^1$H-NMR (DMSO-$d_6$) δ: 0.96 (6H, s), 2.30 (3H, s), 3.30 (2H, m), 3.45-3.51 (2H, m), 4.61-4.67 (2H, m), 6.67 (1H, d, J= 3.0 Hz), 7.26 (1H, dd, J= 2.1 Hz, 8.4 Hz), 7.39 (1H, d, J= 8.7 Hz), 7.74-8.03 (5H, m), 8.24 (1H, d, J= 9.0 Hz), 8.75 (1H, s), 9.06 (1H, d, J= 1.2 Hz), 10.11 (1H, br s).

Example 22

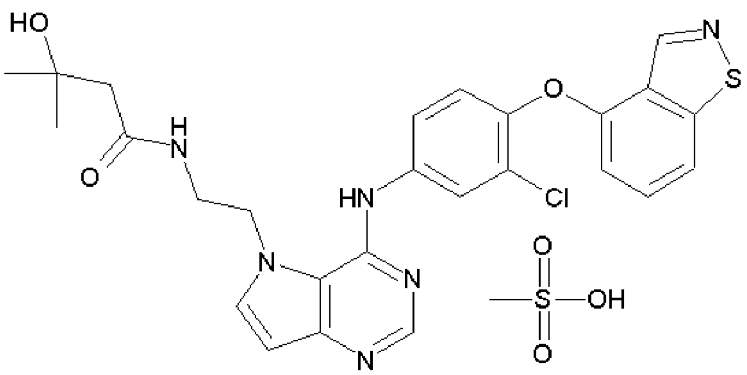

Production of N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-3-methylbutanamide methanesulfonate

(i) Production of tert-butyl [2-(4-{[4-(1,2-benzoisothiazol-4-yloxy)-3-chlorophenyl]amino}-5H-pyrrolo[3,2-d]pyrimidin-5-yl)ethyl]carbamate

A mixture of tert-butyl [2-(4-chloro-5H-pyrrolo[3,2-d]pyrimidin-5-yl)ethyl]carbamate (634 mg), 4-(1,2-benzoisothiazol-4-yloxy)-3-chloroaniline (626 mg) and isopropyl alcohol (8 mL) was stirred at 80°C for 14 hr. Saturated aqueous sodium hydrogen carbonate solution was added to the reaction mixture, and the mixture was extracted with a mixture of ethyl acetate/tetrahydrofuran=1/1. The organic layer was washed with saturated brine, dried over anhydrous magnesium sulfate, and concentrated under reduced pressure. The residue was separated and purified by silica gel column chromatography (eluent, hexane:ethyl acetate=2:1→ethyl acetate) to give the title compound (956 mg) as a pale-yellow powder.
$^1$H-NMR ($CDCl_3$) δ: 1.50 (9H, s), 3.43-3.58 (2H, m), 4.42-4.56 (2H, m), 5.09 (1H, t, J = 5.8 Hz), 6.58-6.70 (2H, m), 7.12-7.23 (2H, m), 7.39 (1H, t, J = 8.0 Hz), 7.62 (1H, d, J = 8.0 Hz), 7.96 (1H, dd, J = 2.6 Hz, 9.0 Hz), 8.08 (1H, d, J = 2.6 Hz), 8.53 (1H, s), 8.65 (1H, s), 9.17 (1H, d, J = 0.9 Hz).

(ii) Production of 5-(2-aminoethyl)-N-[4-(1,2-benzoisothiazol-4-yloxy)-3-chlorophenyl]-5H-pyrrolo[3,2-d]pyrimidin-4-amine dihydrochloride

tert-Butyl [2-(4-{[4-(1,2-benzoisothiazol-4-yloxy)-3-chlorophenyl]amino}-5H-pyrrolo[3,2-d]pyrimidin-5-yl)ethyl] carbamate (952 mg) was dissolved in ethanol (10 mL) and tetrahydrofuran (5 mL), 6N hydrochloric acid (1.5 mL) was added, and the mixture was stirred at 50°C for 14 hr. The reaction mixture was concentrated under reduced pressure, and the residue was dissolved in ethanol. The resulting solution was concentrated under reduced pressure, and the precipitate was collected by filtration to give the title compound (869 mg) as a white powder.
$^1$H-NMR (DMSO-$d_6$) δ: 3.23-3.39 (2H, m), 5.07 (2H, t, J = 6.2 Hz), 6.67 (1H, dd, J = 0.4 Hz, 7.7 Hz), 6.76 (1H, d, J = 3.2 Hz), 7.44 (1H, d, J = 8.9 Hz), 7.58 (1H, t, J = 7.9 Hz), 7.71 (1H, dd, J = 2.5 Hz, 8.9 Hz), 7.92-8.04 (2H, m), 8.09 (1H, d, J = 3.2 Hz), 8.37 (3H, br s), 8.75 (1H, s), 9.22 (1H, d, J = 0.9 Hz), 10.24 (1H, br s).

(iii) Production of N-[2-(4-{[4-(1,2-benzoisothiazol-4-yloxy)-3-chlorophenyl]amino}-5H-pyrrolo[3,2-d]pyrimidin-5-yl) ethyl]-3-hydroxy-3-methylbutanamide methanesulfonate

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (88.4 mg), 3-hydroxy-3-methylbutanoic acid (31.2 mg), 1-hydroxybenzotriazole (41.5 mg), 1-ethyl-3-(3-dimethylaminopropyl)carbodiimide hydrochloride (51.4 mg), triethylamine (0.25 mL), tetrahydrofuran (0.5 mL) and N,N-dimethylformamide (0.5 mL) was stirred at room temperature for 14 hr. Water was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, hexane:ethyl acetate=1:4→ethyl acetate→methyl acetate:methanol=9:1) to give N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-3-methylbutanamide. The obtained compound was dissolved in ethanol (2 mL), methanesulfonic acid (15 μL) was added, and the mixture was stirred at room temperature for 30 min. The reaction mixture was concentrated under reduced pressure and crystallized from ethyl acetate/ethanol to give the title compound (56.8 mg) as white crystals.
$^1$H-NMR (DMSO-$d_6$) δ: 1.13 (6H, s), 2.21 (2H, s), 2.30 (3H, s), 3.43-3.56 (2H, m), 4.64 (2H, t, J = 7.2 Hz), 6.61-6.71 (2H, m), 7.45 (1H, d, J = 8.9 Hz), 7.57 (1H, t, J = 7.9 Hz), 7.75 (1H, dd, J = 2.5 Hz, 8.9 Hz), 7.92-8.06 (3H, m), 8.36 (1H, t, J = 5.5 Hz), 8.74 (1H, s), 9.22 (1H, d, J = 0.8 Hz), 10.18 (1H, br s).

Example 23

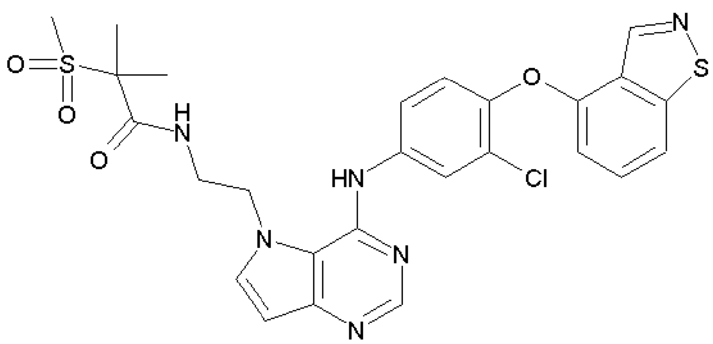

Production of N-[2-(4-{[4-(1,2-benzoisothiazol-4-yloxy)-3-chlorophenyl]amino}-5H-pyrrolo[3,2-d]pyrimidin-5-yl)ethyl]-2-methyl-2-(methylsulfonyl)propanamide

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (90.6 mg), 2-methyl-2-(methylsulfonyl)propanoic acid (44.5 mg), 1-hydroxybenzotriazole (35.0 mg), 1-ethyl-3-(3-dimethylaminopropyl)carbodiimide hydrochloride (49.9 mg), triethylamine (0.25 mL), tetrahydrofuran (0.5 mL) and N,N-dimethylformamide (0.5 mL) was stirred at room temperature for 14 hr. Water was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:4→ethyl acetate→ethyl acetate:methanol=9:1) and crystallized from diisopropyl ether/ethyl acetate to give the title compound (70.8 mg) as white crystals.

$^1$H-NMR (DMSO-$d_6$) δ: 1.41 (6H, s), 2.96 (3H, s), 3.41-3.56 (2H, m), 4.59 (2H, t, J = 6.6 Hz), 6.50 (1H, d, J = 2.6 Hz), 6.60 (1H, d, J = 8.0 Hz), 7.40 (1H, d, J = 9.0 Hz), 7.54 (1H, t, J = 8.0 Hz), 7.58 (1H, d, J = 2.6 Hz), 7.81 (1H, dd, J = 2.0 Hz, 9.0 Hz), 7.92 (1H, d, J = 8.0 Hz), 8.06 (1H, d, J = 2.0 Hz), 8.22 (1H, t, J = 5.9 Hz), 8.36 (1H, s), 8.74 (1H, s), 9.25 (1H, d, J = 0.8 Hz).

Example 24

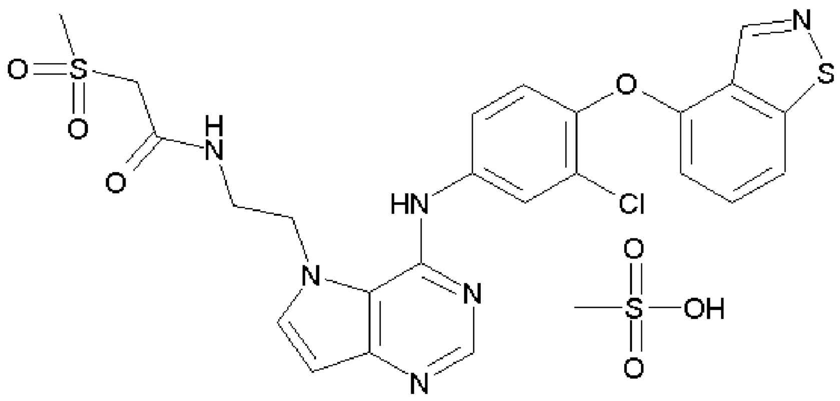

Production of N-[2-(4-{[4-(1,2-benzoisothiazol-4-yloxy)-3-chlorophenyl]amino}-5H-pyrrolo[3,2-d]pyrimidin-5-yl)ethyl]-2-(methylsulfonyl)acetamide methanesulfonate

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (89.9 mg), methanesulfonylacetic acid (38.5 mg), 1-hydroxybenzotriazole (36.1 mg), 1-ethyl-3-(3-dimethylaminopropyl)carbodiimide hydrochloride (51.5 mg), triethylamine (0.25 mL), tetrahydrofuran (0.5 mL), N,N-dimethylformamide (0.5 mL), methanesulfonic acid (5.6 μL), ethyl acetate (1 mL) and ethanol (0.5 mL), and by a reaction in the same manner as in Example 22(iii), the title compound (36.0 mg) was obtained as white crystals.
$^1$H-NMR (DMSO-$d_6$) δ: 2.30 (3H, s), 3.07 (3H, s), 3.51-3.62 (2H, m), 4.06 (2H, s), 4.69 (2H, t, J = 6.5 Hz), 6.62-6.72 (2H, m), 7.46 (1H, d, J = 8.8 Hz), 7.58 (1H, t, J = 8.0 Hz), 7.71 (1H, dd, J = 2.6 Hz, 8.8 Hz), 7.89-8.05 (3H, m), 8.71 (1H, t, J = 6.0 Hz), 8.75 (1H, s), 9.22 (1H, d, J = 0.9 Hz), 9.85 (1H, br s).

Example 25

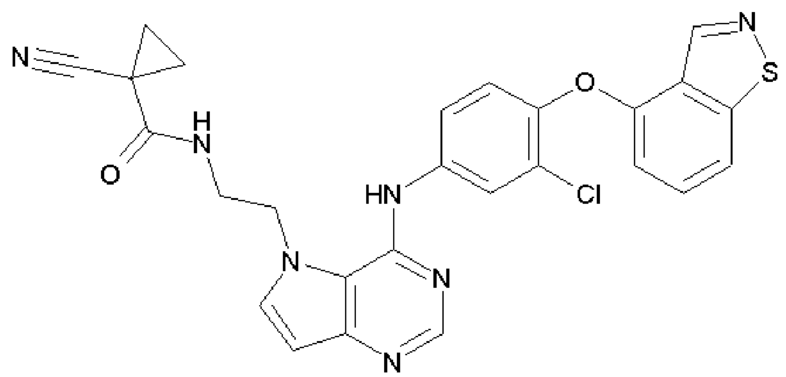

Production of N-[2-(4-{[4-(1,2-benzoisothiazol-4-yloxy)-3-chlorophenyl]amino}-5H-pyrrolo[3,2-d]pyrimidin-5-yl)ethyl]-1-cyanocyclopropanecarboxamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (90.2 mg), 1-cyanocyclopropanecarboxylic acid (31.1 mg), 1-hydroxybenzotriazole (36.7 mg), 1-ethyl-3-(3-dimethylaminopropyl)carbodiimide hydrochloride (60.5 mg), triethylamine (0.25 mL), tetrahydrofuran (0.5 mL) and N,N-dimethylformamide (0.5 mL), and by a reaction in the same manner as in Example 23, the title compound (60.1 mg) was obtained as white crystals.
$^1$H-NMR (DMSO-$d_6$) δ: 1.24-1.34 (2H, m), 1.41-1.51 (2H, m), 3.44 (2H, q, J = 5.5 Hz), 4.60 (2H, t, J = 5.5 Hz), 6.52 (1H, d, J = 3.0 Hz), 6.61 (1H, d, J = 8.0 Hz), 7.39 (1H, d, J = 8.9 Hz), 7.53 (1H, t, J = 8.0 Hz), 7.59 (1H, d, J = 3.0 Hz), 7.77 (1H, dd, J = 2.2 Hz, 8.9 Hz), 7.92 (1H, d, J = 8.0 Hz), 8.03 (1H, d, J = 2.2 Hz), 8.28 (1H, t, J = 5.5 Hz), 8.36 (1H, s), 8.63 (1H, s), 9.25 (1H, d, J = 0.9 Hz).

Example 26

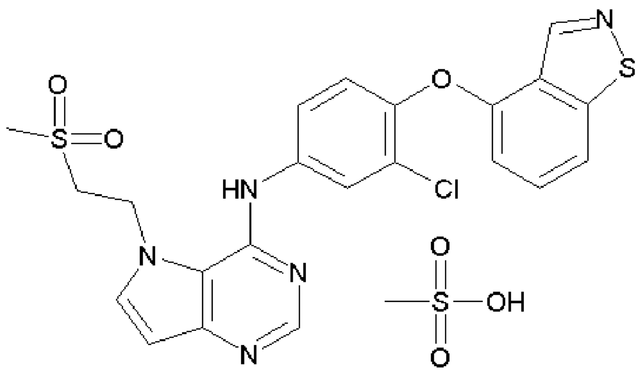

Production of N-[4-(1,2-benzoisothiazol-4-yloxy)-3-chlorophenyl]-5-[2-(methylsulfonyl)ethyl]-5H-pyrrolo[3,2-d]pyrimidin-4-amine methanesulfonate

(i) Production of 4-chloro-5-[2-(methylsulfonyl)ethyl]-5H-pyrrolo[3,2-d]pyrimidine

A mixture of 4-chloro-5H-pyrrolo[3,2-d]pyrimidine (497 mg), methylvinylsulfone (0.43 mL), cesium carbonate (1.63 g) and N,N-dimethylformamide (10 mL) was stirred at room temperature for 2 days. Water was added to the reaction mixture, and the mixture was extracted with ethyl acetate. The organic layer was washed successively with water and saturated brine, dried over anhydrous magnesium sulfate, and concentrated under reduced pressure. The residue was separated and purified by silica gel column chromatography (eluent, hexane:ethyl acetate=2:1→1:4) to give the title compound (185 mg) as a pale-yellow powder.
$^1$H-NMR ($CDCl_3$) δ: 2.79 (3H, s), 3.60 (2H, t, J = 6.5 Hz), 5.02 (2H, t, J = 6.5 Hz), 6.79 (1H, d, J = 3.2 Hz), 7.64 (1H, d, J = 3.2 Hz), 8.75 (1H, s).

(ii) Production of N-[4-(1,2-benzoisothiazol-4-yloxy)-3-chlorophenyl]-5-[2-(methylsulfonyl)ethyl]-5H-pyrrolo[3,2-d]pyrimidin-4-amine methanesulfonate

A mixture of 4-chloro-5-[2-(methylsulfonyl)ethyl]-5H-pyrrolo[3,2-d]pyrimidine (58.9 mg), 4-(1,2-benzoisothiazol-4-yloxy)-3-chloroaniline (68.8 mg) and isopropyl alcohol (1.5 mL) was stirred at 80°C for 17 hr. Aqueous sodium hydrogen carbonate solution was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:1→ethyl acetate) to give N-[4-(1,2-benzoisothiazol-4-yloxy)-3-chlorophenyl]-5-[2-(methylsulfonyl)ethyl]-5H-pyrrolo[3,2-d]pyrimidin-4-amine. The obtained compound was dissolved in ethyl acetate (3 mL), methanesulfonic acid (15 μL) was added, and the mixture was stirred at room temperature for 1 hr. The reaction mixture was concentrated under reduced pressure and crystallized from ethyl acetate/ethanol to give the title compound (53.3 mg) as pale-yellow crystals.
$^1$H-NMR (DMSO-$d_6$) δ: 2.30 (3H, s), 2.97 (3H, s), 3.75 (2H, t, J = 6.5 Hz), 5.14 (2H, t, J = 6.5 Hz), 6.67 (1H, d, J = 8.0

Hz), 6.70 (1H, d, J = 3.2 Hz), 7.46 (1H, d, J = 8.7 Hz), 7.58 (1H, t, J = 8.0 Hz), 7.65 (1H, dd, J = 2.3 Hz, 8.7 Hz), 7.93-8.07 (3H, m), 8.75 (1H, s), 9.22 (1H, d, J = 0.8 Hz), 9.74 (1H, br s).

Example 27

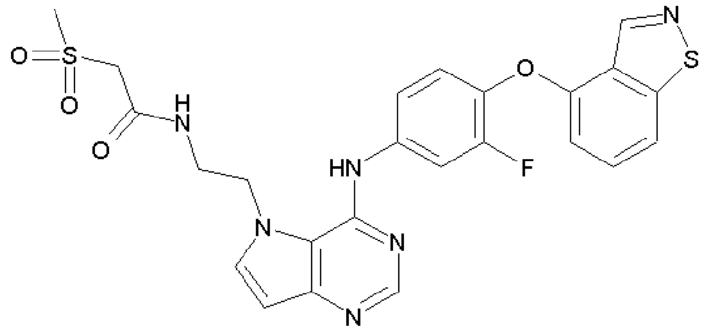

Production of N-[2-(4-{[4-(1,2-benzoisothiazol-4-yloxy)-3-fluorophenyl]amino}-5H-pyrrolo[3,2-d]pyrimidin-5-yl)ethyl]-2-(methylsulfonyl)acetamide

(i) Production of tert-butyl [2-(4-{[4-(1,2-benzoisothiazol-4-yloxy)-3-fluorophenyl]amino}-5H-pyrrolo[3,2-d]pyrimidin-5-yl) ethyl]carbamate

Using tert-butyl [2-(4-chloro-5H-pyrrolo[3,2-d]pyrimidin-5-yl)ethyl]carbamate (115 mg), 4-(1,2-benzoisothiazol-4-yloxy)-3-fluoroaniline (105 mg) and isopropyl alcohol (2.5 mL), and by a reaction in the same manner as in Example 22(i), the title compound (85.5 mg) was obtained as white crystals.
$^1$H-NMR ($CDCl_3$) δ: 1.49 (9H, s), 3.44-3.57 (2H, m), 4.44-4.56 (2H, m), 5.08 (1H, t, J = 5.8 Hz), 6.62 (1H, d, J = 3.0 Hz), 6.71 (1H, d, J = 8.0 Hz), 7.14-7.24 (2H, m), 7.39 (1H, t, J = 8.0 Hz), 7.58-7.64 (1H, m), 7.75 (1H, ddd, J = 1.4 Hz, 2.5 Hz, 9.0 Hz), 7.93 (1H, dd, J = 2.5 Hz, 12.9 Hz), 8.54 (1H, s), 8.60 (1H, br s), 9.17 (1H, d, J = 0.9 Hz).

(ii) Production of 5-(2-aminoethyl)-N-[4-(1,2-benzoisothiazol-4-yloxy)-3-fluorophenyl]-5H-pyrrolo[3,2-d]pyrimidin-4-amine dihydrochloride

Using tert-butyl [2-(4-{[4-(1,2-benzoisothiazol-4-yloxy)-3-fluorophenyl]amino}-5H-pyrrolo[3,2-d]pyrimidin-5-yl) ethyl]carbamate (85.0 mg), 6N hydrochloric acid (0.1 mL), ethanol (1 mL) and tetrahydrofuran (1 mL), and by a reaction in the same manner as in Example 22(ii), the title compound (64.6 mg) was obtained as a white solid.
$^1$H-NMR (DMSO-$d_6$) δ: 3.23-3.35 (2H, m), 5.02 (2H, t, J = 6.3 Hz), 6.69-6.80 (2H, m), 7.47 (1H, t, J = 8.9 Hz), 7.52-7.60 (1H, m), 7.58 (1H, t, J = 8.0 Hz), 7.82 (1H, dd, J = 1.9 Hz, 12.3 Hz), 7.98 (1H, d, J = 8.0 Hz), 8.06 (1H, d, J = 3.0 Hz), 8.27 (3H, br s), 8.75 (1H, s), 9.25 (1H, d, J = 0.8 Hz), 10.12 (1H, br s).

(iii) Production of N-[2-(4-{[4-(1,2-benzoisothiazol-4-yloxy)-3-fluorophenyl]amino}-5H-pyrrolo[3,2-d]pyrimidin-5-yl)ethyl]-2-(methylsulfonyl)acetamide

Using 5-(2-aminoethyl)-N-[4-(1,2-benzoisothiazol-4-yloxy)-3-fluorophenyl]-5H-pyrrolo[3,2-d]pyrimidin-4-amine dihydrochloride (61.3 mg), methylsulfonylacetic acid (26.7 mg), 1-hydroxybenzotriazole (33.4 mg), 1-ethyl-3-(3-dimethylaminopropyl)carbodiimide hydrochloride (46.7 mg), triethylamine (175 μL), tetrahydrofuran (0.3 mL) and N,N-dimethylformamide (0.3 mL), and by a reaction in the same manner as in Example 23, the title compound (32.9 mg) was obtained as white crystals.
$^1$H-NMR (DMSO-$d_6$) δ: 3.10 (3H, s), 3.41-3.54 (2H, m), 4.04 (2H, s), 4.58 (2H, t, J = 6.4 Hz), 6.52 (1H, d, J = 2.8 Hz), 6.69 (1H, d, J = 8.0 Hz), 7.40 (1H, t, J = 9.1 Hz), 7.54 (1H, t, J = 8.0 Hz), 7.58-7.68 (2H, m), 7.84-7.96 (2H, m), 8.38 (1H, s), 8.68 (1H, t, J = 5.7 Hz), 8.76 (1H, s), 9.27 (1H, d, J = 0.8 Hz).

Example 28

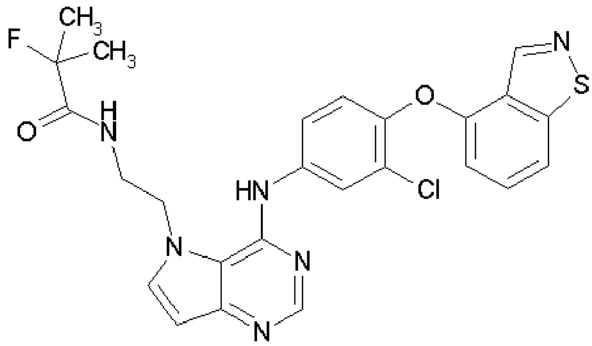

Production of N-[2-(4-{[4-(1,2-benzoisothiazol-4-yloxy)-3-chlorophenyl]amino}-5H-pyrrolo[3,2-d]pyrimidin-5-yl)ethyl]-2-fluoro-2-methylpropanamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 2-fluoro-2-methylpropanoic acid (42 mg), N,N-dimethylformamide (10 mL), triethylamine (0.4 mL), 1-hydroxybenzotriazole (10 mg) and 1-ethyl-3-(3-dimethylaminopropyl)carbodiimide hydrochloride (75 mg), and by a reaction in the same manner as in Example 4(ii), the title compound (63 mg) was obtained as a white powder.
$^1$H-NMR (DMSO-$d_6$) δ: 1.27 (3H, s), 1.34 (3H, s), 3.73-3.81 (2H, m), 4.49-4.68 (2H, m), 6.48 (1H, d, J = 2.5 Hz), 6.60 (1H, d, J = 7.7 Hz), 7.37 (1H, d, J = 8.7 Hz), 7.46-7.60 (2H, m), 7.72 (1H, dd, J = 2.5, 8.7 Hz), 7.90 (1H, d, J = 7.7 Hz), 7.98 (1H, d, J = 2.5 Hz), 8.19 (1H, br s), 8.32 (1H, s), 8.71 (1H, s), 9.22 (1H, d, J = 0.9 Hz) .

Example 29

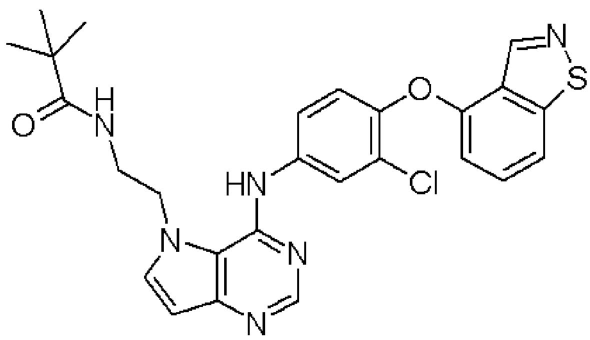

Production of N-[2-(4-{[4-(1,2-benzoisothiazol-4-yloxy)-3-chlorophenyl]amino}-5H-pyrrolo[3,2-d]pyrimidin-5-yl)ethyl]-2,2-dimethylpropanamide

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 2,2-dimethylpropanoic acid anhydride (55 mg), triethylamine (0.3 mL) and N,N-dimethylformamide (10 mL) was stirred at room temperature for 2 hr. Water was added to the reaction mixture, and the mixture was extracted with ethyl acetate. The organic layer was washed successively with water and saturated brine and dried over anhydrous magnesium sulfate. The solvent was evaporated under reduced pressure, and the obtained residue was subjected to silica gel column chromatography (eluent, methanol:ethyl acetate=0:100→10:90) and the object fraction was concentrated under reduced pressure. The residue was crystallized from ethyl acetate/diisopropyl ether to give the title compound (87 mg) as a white powder.
$^1$H-NMR (DMSO-$d_6$) δ: 1.01 (9H, s), 3.64-3.74 (2H, m), 4.51 (2H, t, J = 7.6 Hz), 6.49 (1H, d, J = 3.2 Hz), 6.60 (1H, d, J = 7.6 Hz), 7.37 (1H, d, J=8.9 Hz), 7.46-7.63 (2H, m), 7.75-7.94 (3H, m), 8.10 (1H, d, J = 2.5 Hz), 8.33 (1H, s), 8.90 (1H, s), 9.22 (1H, d, J = 0.9 Hz) .

Example 30

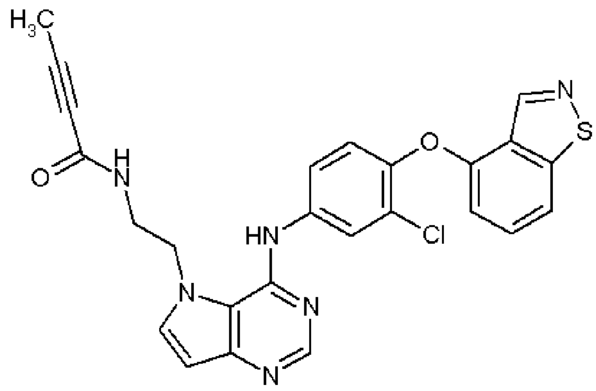

Production of N-[2-(4-{[4-(1,2-benzoisothiazol-4-yloxy)-3-chlorophenyl]amino}-5H-pyrrolo[3,2-d]pyrimidin-5-yl)ethyl] but-2-ynamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), but-2-ynoic acid (50 mg), N,N-dimethylformamide (15 mL), triethylamine (1.2 mL), 1-hydroxybenzotriazole (10 mg) and 1-ethyl-3-(3-dimethylaminopropyl)carbodiimide hydrochloride (320 mg), and by a reaction in the same manner as in Example 4(ii), the title compound (60 mg) was obtained as a pale-yellow powder. $^1$H-NMR (DMSO-$d_6$) δ: 1.90 (3H, s), 3.36-3.45 (2H, m), 4.55 (2H, t, J = 6.4 Hz), 6.51 (1H, d, J = 3.0 Hz), 6.60 (1H, d, J = 8.1 Hz), 7.37 (1H, d, J = 8.9 Hz), 7.45-7.63 (2H, m), 7.70 (1H, dd, J = 2.3, 8.9 Hz), 7.90 (1H, d, J = 8.1 Hz), 7.98 (1H, d, J = 2.3 Hz), 8.33 (1H, s), 8.59-8.80 (2H, m), 9.23 (1H, d, J = 0.9 Hz).

Example 31

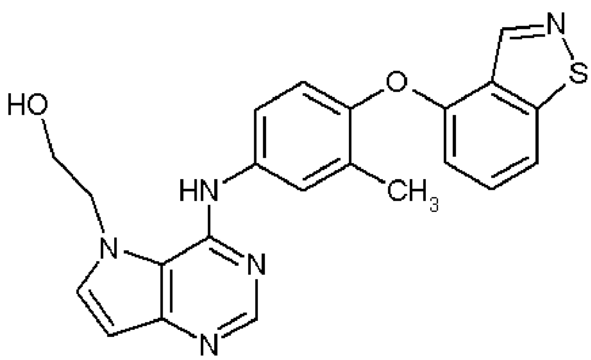

Production of 2-(4-{[4-(1,2-benzoisothiazol-4-yloxy)-3-methylphenyl]amino}-5H-pyrrolo[3,2-d]pyrimidin-5-yl)ethanol (i) Production of 4-(1,2-benzoisothiazol-4-yloxy)-3-methylaniline

To a solution of 1-fluoro-2-methyl-4-nitrobenzene (1.5 g) and 1,2-benzoisothiazol-4-ol (1.5 g) in N,N-dimethylformamide (50 mL) was added potassium carbonate (3.1 g), and the mixture was stirred at 60°C for 3 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=100:0→0:100). The obtained residue (1.4 g) was dissolved in ethanol (20 mL), reduced iron (10.0 g) and 1N hydrochloric acid (3 mL) were added, and the mixture was heated under reflux for 4 hr. The reaction mixture

was cooled to room temperature, 1N aqueous sodium hydroxide solution (10 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane:ethyl acetate=49: 1→1:1) to give the title compound (470 mg) as a pale-purple powder. $^1$H-NMR (DMSO-$d_6$) δ: 1.99 (3H, s), 5.06 (2H, s), 6.38-6.60 (3H, m), 6.84 (1H, d, J = 8.5 Hz), 7.45 (1H, t, J = 8.0 Hz), 7.78 (1H, d, J = 8.0 Hz), 9.20 (1H, d, J = 0.9 Hz).

(ii) Production of 2-(4-{[4-(1,2-benzoisothiazol-4-yloxy)-3-methylphenyl]amino}-5H-pyrrolo[3,2-d]pyrimidin-5-yl)ethanol

Using 2-(4-chloro-5H-pyrrolo[3,2-d]pyrimidin-5-yl)ethyl benzoate (120 mg), 4-(1,2-benzoisothiazol-4-yloxy)-3-methylaniline (130 mg), isopropyl alcohol (10 mL), ethanol (15 mL) and 1N aqueous sodium hydroxide solution (5.0 mL), and by a reaction in the same manner as in Example 3, the title compound (122 mg) was obtained as a white powder. $^1$H-NMR (DMSO-$d_6$) δ: 2.15 (3H, s), 3.88 (2H, t, J = 4.5 Hz), 4.52 (2H, t, J = 4.5 Hz), 6.40-6.62 (2H, m), 7.06-7.18 (1H, m), 7.39-7.68 (4H, m), 7.84 (1H, d, J = 8.1 Hz), 8.28 (1H, s), 9.22 (1H, d, J = 0.8 Hz), 9.73 (1H, s).
melting point: 229-231°C

Example 32

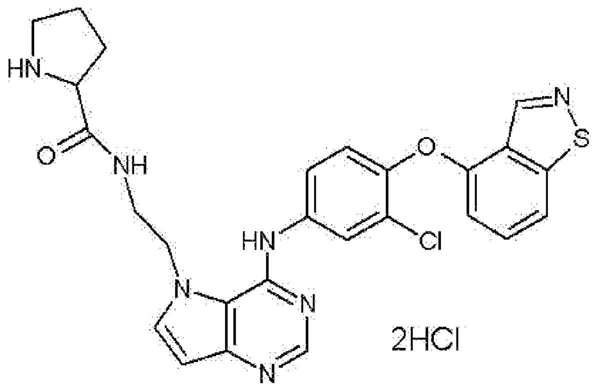

2HCl

Production of N-[2-(4-{[4-(1,2-benzoisothiazol-4-yloxy)-3-chlorophenyl]amino}-5H-pyrrolo[3,2-d]pyrimidin-5-yl)ethyl] prolinamide dihydrochloride

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (150 mg), 1-(tert-butoxycarbonyl)proline (72 mg), N,N-dimethylformamide (15 mL), triethylamine (1.2 mL), 1-hydroxybenzotriazole (20 mg) and 1-ethyl-3-(3-dimethylaminopropyl)carbodiimide hydrochloride (320 mg), and by a reaction in the same manner as in Example 4(ii), a compound was obtained and dissolved in ethanol (5 mL). 4N Hydrochloric acid/ethyl acetate (5 mL) was added, and the mixture was stirred at 80°C for 2 hr. After cooling to room temperature, the precipitated solid was collected by filtration to give the title compound (72 mg) as a white powder. $^1$H-NMR (DMSO-$d_6$) δ: 1.54-1.93 (3H, m), 2.06-2.25 (1H, m), 2.68-3.74 (4H, m), 3.96-4.10 (1H, m), 4.58-4.90 (2H, m), 6.57-6.74 (2H, m), 7.43 (1H, d, J = 8.9 Hz), 7.51-7.75 (2H, m), 7.84-8.01 (3H, m), 8.43 (1H, br s), 8.69 (1H, s), 8.80 (1H, t, J = 5.8 Hz), 9.19 (1H, d, J = 0.8 Hz), 9.54 (1H, br s), 9.95 (1H, br s).

Example 33

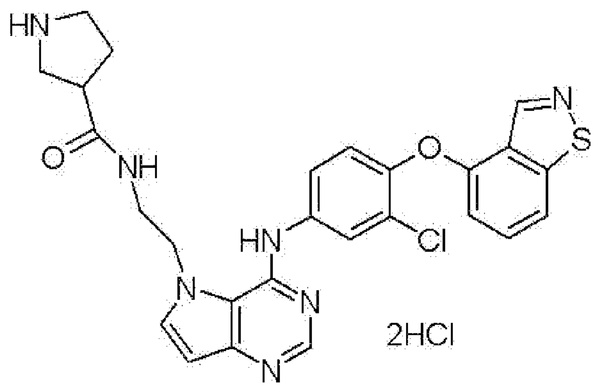

Production of N-[2-(4-{[4-(1,2-benzoisothiazol-4-yloxy)-3-chlorophenyl]amino}-5H-pyrrolo[3,2-d]pyrimidin-5-yl)ethyl] pyrrolidine-3-carboxamide dihydrochloride

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (150 mg), 1-(tert-butoxycarbonyl)pyrrolidine-3-carboxylic acid (40 mg), N,N-dimethylformamide (15 mL), triethylamine (1.2 mL), 1-hydroxybenzotriazole (16 mg) and 1-ethyl-3-(3-dimethylaminopropyl)carbodiimide hydrochloride (220 mg), and by a reaction in the same manner as in Example 4(ii), a compound was obtained and dissolved in ethanol (5 mL). 4N Hydrochloric acid/ethyl acetate (5 mL) was added, and the mixture was stirred at 80°C for 2 hr. After cooling to room temperature, the precipitated solid was collected by filtration to give the title compound (51 mg) as a white powder.
$^1$H-NMR (DMSO-$d_6$) δ: 1.63-2.15 (2H, m), 2.74 (1H, d, J = 4.7 Hz), 2.89-3.73 (6H, m), 4.68 (2H, br s), 6.55-6.75 (2H, m), 7.43 (1H, d, J = 8.7 Hz), 7.50-7.75 (2H, m), 7.82-8.02 (3H, m), 8.49 (1H, s), 8.67 (1H, s), 8.89 (2H, br s), 9.19 (1H, d, J = 0.9 Hz), 9.87 (1H, br s).
melting point: 147-149°C

Example 34

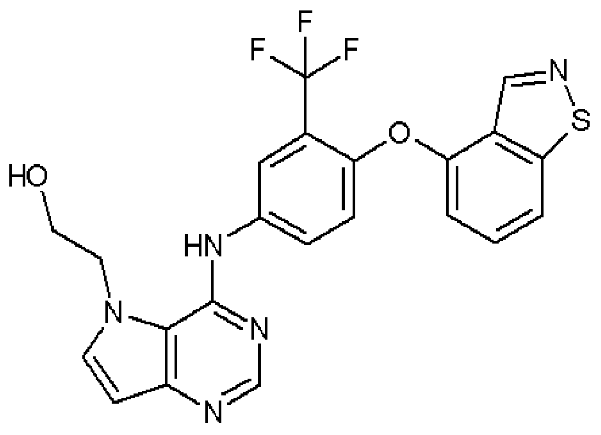

Production of 2-(4-{[4-(1,2-benzoisothiazol-4-yloxy)-3-(trifluoromethyl)phenyl]aminol-5H-pyrrolo[3,2-dlpyrimidin-5-yl) ethanol

(i) Production of 4-(1,2-benzoisothiazol-4-yloxy)-3-(trifluoromethyl)aniline

To a solution of 1-fluoro-4-nitro-2-(trifluoromethyl)benzene (3.0 g) and 1,2-benzoisothiazol-4-ol (3.0 g) in N,N-dimethylformamide (60 mL) was added potassium carbonate (6.2 g), and the mixture was stirred at room temperature for 5 hr. Water was added to the reaction mixture, and the mixture was extracted with ethyl acetate. The organic layer was washed with saturated brine and dried over anhydrous magnesium sulfate. Magnesium sulfate was filtered off, and

the filtrate was concentrated under reduced pressure. The obtained residue was separated and purified by silica gel chromatography (hexane:ethyl acetate=100:0→0:100), and the obtained residue was dissolved in ethanol (50 mL). Reduced iron (20.0 g) and 1N hydrochloric acid (5 mL) were added and the mixture was heated under reflux for 4 hr. The reaction mixture was cooled to room temperature, 1N aqueous sodium hydroxide solution (15 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane: ethyl acetate=49:1→1:1) to give the title compound (3.7 g) as a pale-green powder.
$^1$H-NMR (DMSO-$d_6$) δ: 5.60 (2H, s), 6.59 (1H, d, J = 8.0 Hz), 6.87 (1H, dd, J = 2.5, 8.7 Hz), 6.99 (1H, d, J = 2.5 Hz), 7.09 (1H, d, J = 8.7 Hz), 7.50 (1H, t, J = 8.0 Hz), 7.87 (1H, d, J = 8.0 Hz), 9.10 (1H, d, J = 0.9 Hz).

(ii) Production of 2-(4-{[4-(1,2-benzoisothiazol-4-yloxy)-3-(trifluoromethyl)phenyl]aminol-5H-pyrrolo[3,2-dlpyrimidin-5-yl) ethanol

Using 2-(4-chloro-5H-pyrrolo[3,2-d]pyrimidin-5-yl)ethyl benzoate (100 mg), 4-(1,2-benzoisothiazol-4-yloxy)-3-(trifluoromethyl)aniline (120 mg), isopropyl alcohol (10 mL), ethanol (15 mL) and 1N aqueous sodium hydroxide solution (3.0 mL), and by a reaction in the same manner as in Example 3, the title compound (70 mg) was obtained as a white powder.
$^1$H-NMR (DMSO-$d_6$) δ: 3.84-3.92 (2H, m), 4.45-4.61 (2H, m), 6.35 (1H, br s), 6.52 (1H, d, J = 3.0 Hz), 6.78 (1H, d, J = 7.9 Hz), 7.34 (1H, d, J = 9.1 Hz), 7.57 (1H, t, J = 7.9 Hz), 7.64-7.79 (1H, m), 7.87-8.00 (2H, m), 8.13 (1H, d, J = 3.0 Hz), 8.32 (1H, s), 9.10 (1H, d, J = 0.8 Hz), 9.98 (1H, s).
melting point: 212-214°C

Example 35

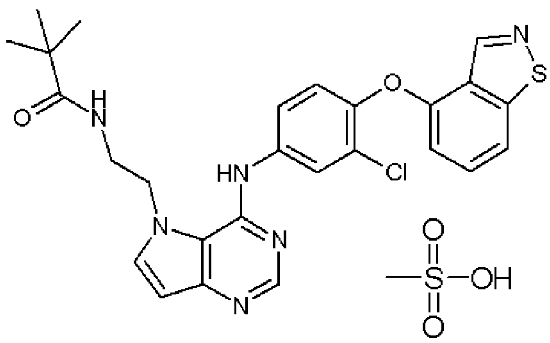

Production of N-[2-(4-{[4-(1,2-benzoisothiazol-4-yloxy)-3-chlorophenyl]amino}-5H-pyrrolo[3,2-d]pyrimidin-5-yl)ethyl]-2,2-dimethylpropanamide methanesulfonate

N-[2-(4-{[4-(1,2-Benzoisothiazol-4-yloxy)-3-chlorophenyl]amino}-5H-pyrrolo[3,2-d]pyrimidin-5-yl)ethyl]-2,2-dimethylpropanamide (1.5 g) was dissolved in ethyl acetate (100 mL), and methanesulfonic acid (300 mg) was added. Diethyl ether (5 mL) was added, and the precipitated powder was collected by filtration to give the title compound (1.3 g) as a white powder.
$^1$H-NMR (DMSO-$d_6$) δ: 1.02 (9H, s), 2.32 (3H, s), 3.43-3.52 (2H, m), 4.62-4.69 (2H, m), 6.68 (2H, dd, J = 2.2, 5.4 Hz), 7.47 (1H, d, J = 8.9 Hz), 7.58 (1H, t, J = 7.9 Hz), 7.73-8.10 (5H, m), 8.77 (1H, s), 9.22 (1H, d, J = 0.8 Hz), 10.13 (1H, br s) .

Example 36

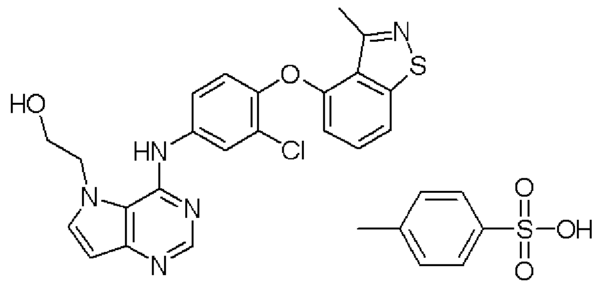

Production of 2-[4-({3-chloro-4-[(3-methyl-1,2-benzoisothiazol-4-yl)oxy]phenyl}amino)-5H-pyrrolo[3,2-d]pyrimidin-5-yl] ethanol p-toluenesulfonate

(i) Production of 1-[2-(benzylsulfanyl)-6-methoxyphenyl]ethanone

Benzylmercaptan (1.7 g) was dissolved in tetrahydrofuran (50 mL), lithium hexamethyldisilazide (12 mL) was added, and the mixture was stirred for 30 min. 1-(2-Fluoro-6-methoxyphenyl)ethanone (2.0 g) was added, and the mixture was stirred for 5 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=100:0→50:50) to give the title compound (2.6 g) as a pale-green powder.
$^1$H-NMR ($CDCl_3$) δ: 2.39 (3H, s), 3.81 (3H, s), 4.04 (2H, s), 6.80 (1H, d, J = 8.5 Hz), 6.93 (1H, d, J = 7.9 Hz), 7.17-7.32 (6H, m).

(ii) Production of 4-methoxy-3-methyl-1,2-benzoisothiazole

1-[2-(Benzylsulfanyl)-6-methoxyphenyl]ethanone (2.5 g) and thioanisole (2.5 g) were dissolved in acetonitrile (15 mL)/water (15 mL), hydroxylamine-O-sulfonic acid (1.8 g) was added, and the mixture was stirred for 2 hr. Saturated aqueous sodium hydrogen carbonate was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=100:0→50:50) to give the title compound (220 mg) as a white powder.
$^1$H-NMR (DMSO-$d_6$) δ: 2.78 (3H, s), 3.96 (3H, s), 6.94 (1H, d, J = 8.1 Hz), 7.50 (1H, t, J = 8.1 Hz), 7.63 (1H, d, J = 8.1 Hz).

(iii) Production of 3-chloro-4-[(3-methyl-1,2-benzoisothiazol-4-yl)oxy]aniline

A mixture of 4-methoxy-3-methyl-1,2-benzoisothiazole and pyridine hydrochloride was stirred at 200°C for 2 hr. The reaction mixture was cooled to room temperature, water and ethyl acetate were added, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=50:50→0:100). Using the obtained residue (64 mg), 2-chloro-1-fluoro-4-nitrobenzene (70 mg), N,N-dimethylformamide (5 mL), potassium carbonate (80 mg), ethanol (5 mL), reduced iron (200 mg) and 1N hydrochloric acid (0.5 mL), and by a reaction in the same manner as in Example 31(i), the title compound (65 mg) was obtained as a pale-purple powder.
$^1$H-NMR ($CDCl_3$) δ: 2.79 (3H, s), 3.73 (2H, br s), 6.44 (1H, d, J = 7.9 Hz), 6.63 (1H, dd, J = 2.7, 8.7 Hz), 6.83 (1H, d, J = 2.7 Hz), 6.99 (1H, d, J = 8.7 Hz), 7.27-7.33 (1H, m), 7.49 (1H, d, J = 7.9 Hz).

(iv) Production of 2-[4-({3-chloro-4-[(3-methyl-1,2-benzoisothiazol-4-yl)oxy]phenyl}amino)-5H-pyrrolo[3,2-d]pyrimidin-5-yl]ethanol p-toluenesulfonate

Using 2-(4-chloro-5H-pyrrolo[3,2-d]pyrimidin-5-yl)ethyl benzoate (52 mg), 3-chloro-4-[(3-methyl-1,2-benzoisothiazol-4-yl)oxy]aniline (50 mg), isopropyl alcohol (5 mL), ethanol (2 mL) and 1N aqueous sodium hydroxide solution (2.0 mL), and by a reaction in the same manner as in Example 3, the obtained residue was dissolved in methanol (5 mL). The solution was treated with p-toluenesulfonic acid monohydrate (15 mg), and the precipitated powder was collected by filtration to give the title compound (46 mg) as a white powder.

$^1$H-NMR (DMSO-$d_6$) δ: 2.28 (3H, s), 2.87 (3H, s), 3.91 (2H, t, J = 4.4 Hz), 4.64 (2H, br s), 6.37 (1H, br s), 6.52-6.73 (2H, m), 7.10 (2H, d, J = 7.9 Hz), 7.32-7.55 (3H, m), 7.64 (1H, dd, J = 2.5, 8.8 Hz), 7.78-7.94 (2H, m), 8.00 (1H, d, J = 2.5 Hz), 8.63 (1H, d, J = 5.3 Hz), 10.51 (1H, br s).

Example 37

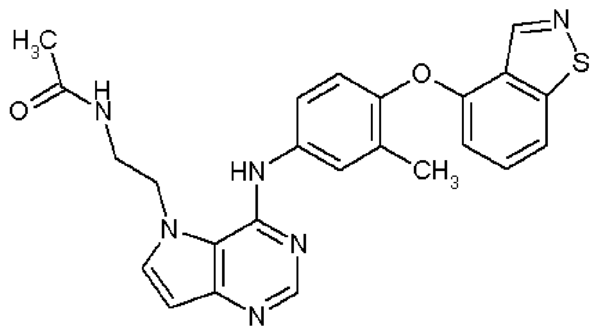

Production of N-[2-(4-{[4-(1,2-benzoisothiazol-4-yloxy)-3-methylphenyl]amino}-5H-pyrrolo[3,2-d]pyrimidin-5-yl)ethyl] acetamide

(i) Production of 5-(2-aminoethyl)-N-[4-(1,2-benzoisothiazol-4-yloxy)-3-methylphenyl]-5H-pyrrolo[3,2-d]pyrimidin-4-amine dihydrochloride

A mixture of tert-butyl [2-(4-chloro-5H-pyrrolo[3,2-d]pyrimidin-5-yl)ethyl]carbamate (1.1 g), 4-(1,2-benzoisothiazol-4-yloxy)-3-methylaniline (1.0 g) and isopropyl alcohol (25 mL) was stirred at 80°C for 3 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure. 4N hydrochloric acid/ethyl acetate (10 mL) and ethanol (15 mL) were added to the residue, and the mixture was stirred at 80°C for 4 hr. Ethyl acetate was added to the reaction mixture, and the precipitate was collected by filtration to give the title compound (1.2 g) as a brown powder.
$^1$H-NMR (DMSO-$d_6$) δ: 2.24 (3H, s), 3.25-3.34 (2H, m), 5.04 (2H, t, J = 6.1 Hz), 6.63 (1H, d, J = 7.5 Hz), 6.74 (1H, d, J = 3.2 Hz), 7.19 (1H, d, J = 8.5 Hz), 7.41-7.64 (3H, m), 7.93 (1H, d, J = 8.5 Hz), 8.08 (1H, d, J = 3.2 Hz), 8.37 (3H, br s), 8.71 (1H, s), 9.21 (1H, d, J = 0.9 Hz), 10.04 (1H, s).

(ii) Production of N-[2-(4-{[4-(1,2-benzoisothiazol-4-yloxy)-3-methylphenyllaminol-5H-pyrrolo[3,2-d]pyrimidin-5-yl)ethyl] acetamide

A mixture of 5-(2-aminoethyl)-N-[4-(1,2-benzoisothiazol-4-yloxy)-3-methylphenyl]-5H-pyrrolo[3,2-d]pyrimidin-4-amine dihydrochloride (250 mg), acetic acid (0.7 mL), triethylamine (1.5 mL), 1-hydroxybenzotriazole (20 mg), 1-ethyl-3-(3-dimethylaminopropyl)carbodiimide hydrochloride (400 mg) and N,N-dimethylformamide (25 mL) was stirred overnight at room temperature. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure and the residue was crystallized from ethyl acetate/diisopropyl ether to give the title compound (197 mg) as a white powder.
$^1$H-NMR (DMSO-$d_6$) δ: 1.81 (3H, s), 2.18 (3H, s), 3.39 (2H, t, J = 6.3 Hz), 4.52 (2H, t, J = 6.8 Hz), 6.48 (1H, d, J = 3.0 Hz), 6.52-6.62 (1H, m), 7.12 (1H, d, J = 8.7 Hz), 7.42-7.65 (3H, m), 7.70 (1H, d, J = 2.3 Hz), 7.87 (1H, d, J = 8.7 Hz), 8.23 (1H, t, J = 6.8 Hz), 8.29 (1H, s), 8.63 (1H, s), 9.24 (1H, d, J = 0.8 Hz).

Example 38

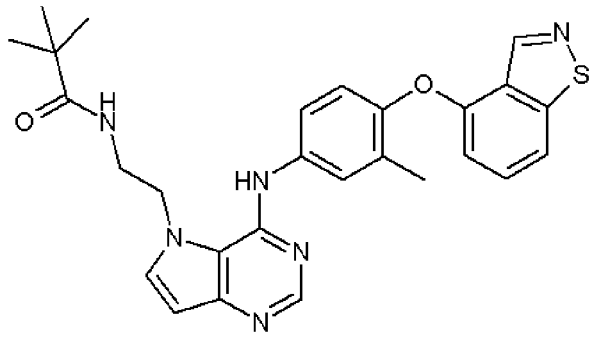

Production of N-[2-(4-{[4-(1,2-benzoisothiazol-4-yloxy)-3-methylphenyl]amino}-5H-pyrrolo[3,2-d]pyrimidin-5-yl)ethyl]-2,2-dimethylpropanamide

A mixture of 5-(2-aminoethyl)-N-[4-(1,2-benzoisothiazol-4-yloxy)-3-methylphenyl]-5H-pyrrolo[3,2-d]pyrimidin-4-amine dihydrochloride (350 mg), 2,2-dimethylpropanoic acid anhydride (0.75 mL), triethylamine (1.5 mL) and N,N-dimethylformamide (25 mL) was stirred at room temperature for 2 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was crystallized from ethyl acetate/diisopropyl ether to give the title compound (250 mg) as a white powder.
$^1$H-NMR (DMSO-$d_6$) δ: 1.04 (9H, s), 2.18 (3H, s), 3.37-3.48 (2H, m), 4.53 (2H, t, J = 6.5 Hz), 6.47 (1H, d, J = 3.0 Hz), 6.57 (1H, d, J = 7.7 Hz), 7.00-7.18 (1H, m), 7.43-7.60 (2H, m), 7.66-7.93 (4H, m), 8.30 (1H, s), 8.66 (1H, s), 9.24 (1H, d, J = 0.8 Hz).
melting point: 189-191°C

Example 39

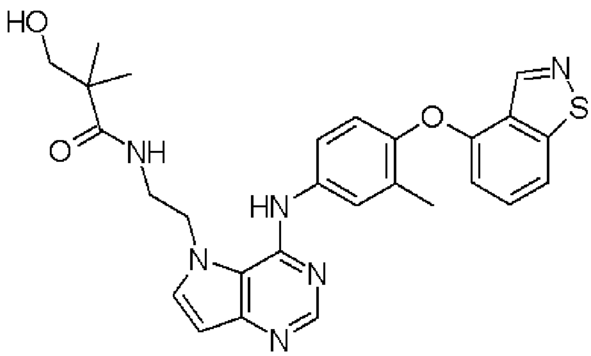

Production of N-[2-(4-{[4-(1,2-benzoisothiazol-4-yloxy)-3-methylphenyl]amino}-5H-pyrrolo[3,2-d]pyrimidin-5-yl)ethyl]-3-hydroxy-2,2-dimethylpropanamide

Using 5-(2-aminoethyl)-N-[4-(1,2-benzoisothiazol-4-yloxy)-3-methylphenyl]-5H-pyrrolo[3,2-d]pyrimidin-4-amine dihydrochloride (150 mg), 3-hydroxy-2,2-dimethylpropanoic acid (70 mg), N,N-dimethylformamide (15 mL), triethylamine (0.9 mL), 1-hydroxybenzotriazole (20 mg) and 1-ethyl-3-(3-dimethylaminopropyl)carbodiimide hydrochloride (300 mg), and by a reaction in the same manner as in Example 4(ii), the title compound (105 mg) was obtained as a pale-yellow powder.
$^1$H-NMR (DMSO-$d_6$) δ: 0.99 (6H, s), 2.18 (3H, s), 3.32 (2H, s), 3.38-3.48 (2H, m), 4.52 (2H, t, J = 6.7 Hz), 4.83 (1H, s),

6.46 (1H, d, J = 3.0 Hz), 6.54-6.61 (1H, m), 7.12 (1H, d, J = 9.4 Hz), 7.42-7.61 (2H, m), 7.67-7.93 (4H, m), 8.30 (1H, s), 8.71 (1H, s), 9.24 (1H, d, J = 0.9 Hz).

Example 40

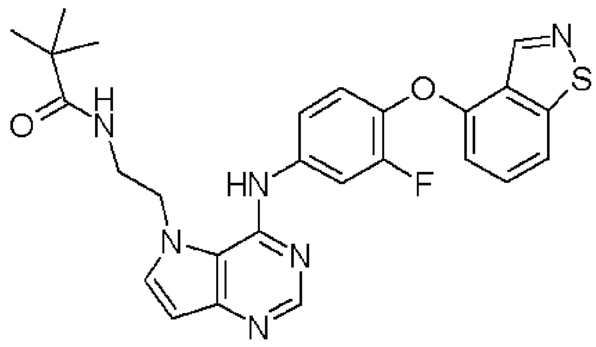

Production of N-[2-(4-{[4-(1,2-benzoisothiazol-4-yloxy)-3-fluorophenyl]amino}-5H-pyrrolo[3,2-d]pyrimidin-5-yl)ethyl]-2,2-dimethylpropanamide

A mixture of 5-(2-aminoethyl)-N-[4-(1,2-benzoisothiazol-4-yloxy)-3-fluorophenyl]-5H-pyrrolo[3,2-d]pyrimidin-4-amine dihydrochloride (300 mg), 2,2-dimethylpropanoic acid anhydride (0.6 mL), triethylamine (0.9 mL) and N,N-dimethylformamide (20 mL) was stirred at room temperature for 2 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure and the residue was crystallized from ethyl acetate/diisopropyl ether to give the title compound (230 mg) as a white powder.
$^1$H-NMR (DMSO-$d_6$) δ: 1.03 (9H, s), 3.34-3.47 (2H, m), 4.49-4.60 (2H, m), 6.50 (1H, d, J = 3.0 Hz), 6.69 (1H, d, J = 7.9 Hz), 7.40 (1H, t, J = 9.1 Hz), 7.49-7.64 (2H, m), 7.71 (1H, s), 7.84 (1H, br s), 7.92 (1H, d, J = 7.9 Hz), 7.99 (1H, dd, J = 2.4, 13.5 Hz), 8.37 (1H, s), 8.89 (1H, s), 9.26 (1H, d, J = 0.8 Hz).

Example 41

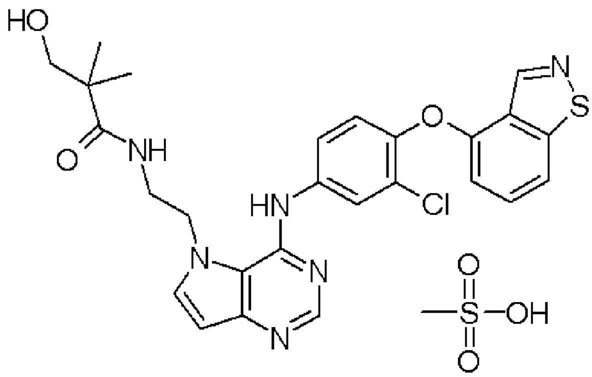

Production of N-[2-(4-{[4-(1,2-benzoisothiazol-4-yloxy)-3-chlorophenyl]amino}-5H-pyrrolo[3,2-d]pyrimidin-5-yl)ethyl]-3-hydroxy-2,2-dimethylpropanamide methanesulfonate

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (150 mg), 3-hydroxy-2,2-dimethylpropanoic acid (70 mg), N,N-dimethylformamide (25 mL), triethylamine (0.9 mL), 1-hydroxybenzotriazole (20 mg) and 1-ethyl-3-(3-dimethylaminopropyl)carbodiimide hydrochloride (320 mg), and by a reaction in the same manner as in Example 4(ii), a compound was obtained, which was treated with methanesulfonic acid (45 mg) and subjected to an operation similar to that in Example 7 to give the title compound (75 mg) as a white powder.
$^1$H-NMR (DMSO-$d_6$) δ: 0.97 (6H, s), 2.30 (3H, s), 3.30 (2H, s), 3.41-3.52 (2H, m), 4.56-4.69 (2H, m), 6.59-6.73 (2H, m), 7.46 (1H, d, J = 8.7 Hz), 7.58 (1H, t, J = 7.9 Hz), 7.73-8.00 (3H, m), 7.74-7.82 (1H, m), 8.05 (1H, d, J = 2.5 Hz), 8.74 (1H, s), 9.22 (1H, d, J = 0.8 Hz), 10.08 (1H, br s).
melting point: 219-221°C

Example 42

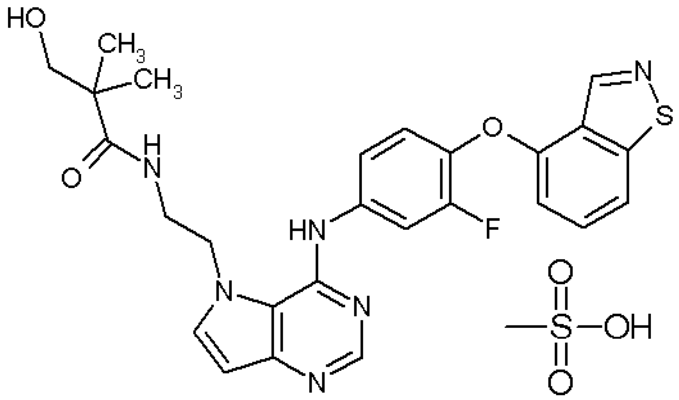

Production of N-[2-(4-{[4-(1,2-benzoisothiazol-4-yloxy)-3-fluorophenyl]amino}-5H-pyrrolo[3,2-d]pyrimidin-5-yl)ethyl]-3-hydroxy-2,2-dimethylpropanamide methanesulfonate

Using 5-(2-aminoethyl)-N-[4-(1,2-benzoisothiazol-4-yloxy)-3-fluorophenyl]-5H-pyrrolo[3,2-d]pyrimidin-4-amine dihydrochloride (200 mg), 3-hydroxy-2,2-dimethylpropanoic acid (70 mg), N,N-dimethylformamide (20 mL), triethylamine (1.0 mL), 1-hydroxybenzotriazole (20 mg) and 1-ethyl-3-(3-dimethylaminopropyl)carbodiimide hydrochloride (360 mg), and by a reaction in the same manner as in Example 4(ii), a compound was obtained, which was treated with methanesulfonic acid (47 mg) and subjected to an operation similar to that in Example 7 to give the title compound (95 mg) as a white powder.
$^1$H-NMR (DMSO-$d_6$) δ: 0.96 (6H, s), 2.30 (3H, s), 3.30 (2H, s), 3.43-3.54 (2H, m), 4.55-4.73 (2H, m), 6.67 (1H, d, J = 3.0 Hz), 6.76 (1H, d, J = 7.9 Hz), 7.42-7.68 (3H, m), 7.80-8.04 (4H, m), 8.76 (1H, s), 9.25 (1H, d, J = 0.9 Hz), 10.14 (1H, br s).

Example 43

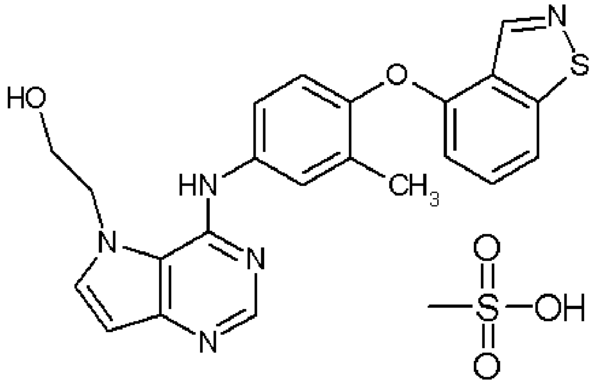

Production of 2-(4-{[4-(1,2-benzoisothiazol-4-yloxy)-3-methylphenyl]amino}-5H-pyrrolo[3,2-d]pyrimidin-5-yl)ethanol methanesulfonate

2-(4-{[4-(1,2-Benzoisothiazol-4-yloxy)-3-methylphenyl]amino}-5H-pyrrolo[3,2-d]pyrimidin-5-yl)ethanol (200 mg) was dissolved in ethyl acetate (5 mL)/ethanol (5 mL), and methanesulfonic acid (50 mg) was added. The precipitated crystals were collected by filtration to give the title compound (220 mg) as a white powder.
$^1$H-NMR (DMSO-$d_6$) δ: 2.22 (3H, s), 2.35 (3H, s), 3.89-3.95 (2H, m), 4.66 (2H, t, J = 4.3 Hz), 6.59 (1H, d, J = 7.6 Hz), 6.68 (1H, d, J = 3.2 Hz), 7.19 (1H, d, J = 8.3 Hz), 7.47-7.61 (3H, m), 7.89 (1H, d, J = 8.3 Hz), 7.96 (1H, d, J = 3.2 Hz), 8.69 (1H, s), 9.19 (1H, d, J = 0.9 Hz), 10.78 (1H, br s).

Example 44

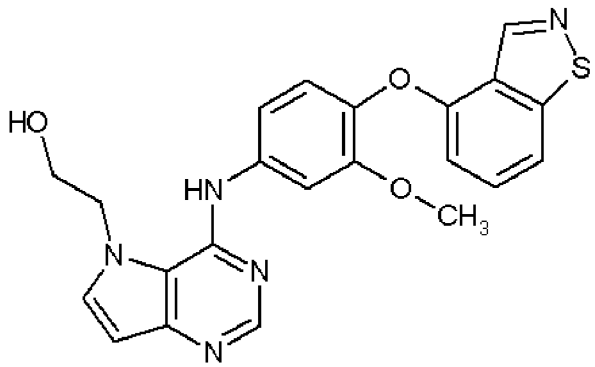

Production of 2-(4-{[4-(1,2-benzoisothiazol-4-yloxy)-3-methoxyphenyl]amino}-5H-pyrrolo[3,2-d]pyrimidin-5-yl)ethanol (i) Production of 4-(1,2-benzoisothiazol-4-yloxy)-3-methoxyaniline

To a solution of 1-bromo-2-methoxy-4-nitrobenzene (2.2 g) and 1,2-benzoisothiazol-4-ol (1.5 g) in N,N-dimethylformamide (20 mL) was added potassium carbonate (1.5 g), and the mixture was stirred at room temperature for 12 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=100:0→0:100) to give crude crystals (720 mg). The obtained crude crystals (300 mg) were dissolved in ethanol (20 mL), reduced iron (1.5 g) and 1N hydrochloric acid (1.0 mL) were added, and the mixture was heated under reflux at 80°C for 1 hr. The reaction mixture was cooled to room temperature, 1N aqueous sodium hydroxide solution (5 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane:ethyl acetate=49:1→1:1) to give the title compound (270 mg) as a pale-purple powder.
$^1$H-NMR (DMSO-$d_6$) δ: 3.62 (3H, s), 5.17 (2H, s), 6.20 (1H, dd, J = 2.5, 8.3 Hz), 6.38-6.46 (2H, m), 6.88 (1H, d, J = 8.3 Hz), 7.43 (1H, t, J = 7.9 Hz), 7.75 (1H, d, J = 8.3 Hz), 9.17 (1H, d, J = 0.9 Hz).

(ii) Production of 2-(4-{[4-(1,2-benzoisothiazol-4-yloxy)-3-methoxyphenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (110 mg), 4-(1,2-benzoisothiazol-4-yloxy)-3-methoxyaniline (100 mg) and isopropyl alcohol (7 mL) was stirred at 80°C overnight. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate: hexane=50:50→100:0→methanol:ethyl acetate=15:85), and the object fraction was concentrated under reduced pressure. Methanol (5 mL) and 1N aqueous sodium hydroxide solution (1.0 mL) were added to the residue, and the mixture was stirred overnight at room temperature. Water was added to the reaction mixture, and the mixture was extracted with

ethyl acetate. The organic layer was washed with saturated brine and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure and the residue was crystallized from ethyl acetate/diisopropyl ether to give the title compound (56 mg) as a white powder. $^1$H-NMR (DMSO-$d_6$) δ: 3.74 (3H, s), 3.90 (2H, t, J = 4.6 Hz), 4.54 (2H, t, J = 4.6 Hz), 6.32 (1H, br s), 6.47-6.54 (2H, m), 7.16-7.34 (2H, m), 7.47 (1H, t, J = 8.0 Hz), 7.60 (1H, d, J = 2.3 Hz), 7.65 (1H, d, J = 3.2 Hz), 7.82 (1H, d, J = 8.0 Hz), 8.32 (1H, s), 9.23 (1H, d, J = 0.8 Hz), 9.80 (1H, br s).

Example 45

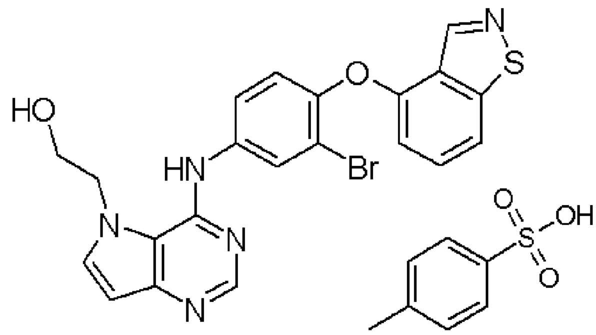

Production of 2-(4-{[4-(1,2-benzoisothiazol-4-yloxy)-3-bromophenyl]amino}-5H-pyrrolo[3,2-d]pyrimidin-5-yl)ethanol p-toluenesulfonate

(i) Production of 4-(1,2-benzoisothiazol-4-yloxy)-3-bromoaniline

To a solution of 2-bromo-1-fluoro-4-nitrobenzene (1.8 g) and 1,2-benzoisothiazol-4-ol (1.4 g) in N,N-dimethylformamide (25 mL) was added potassium carbonate (1.7 g), and the mixture was stirred at room temperature for 12 hr. Water was added to the reaction mixture, and the mixture was extracted with ethyl acetate. The organic layer was washed with saturated brine and dried over anhydrous magnesium sulfate. Magnesium sulfate was filtered off, and the filtrate was concentrated under reduced pressure. The obtained residue was separated and purified by silica gel chromatography (hexane:ethyl acetate=100:0→0:100) to give crude crystals (880 mg). The obtained crude crystals (500 mg) were dissolved in ethanol (30 mL), reduced iron (2.0 g) and 1N hydrochloric acid (2.0 mL) was added, and the mixture was heated under reflux at 80°C for 1 hr. The reaction mixture was cooled to room temperature, 1N aqueous sodium hydroxide solution (8 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane:ethyl acetate=100:0→50:50) to give the title compound (320 mg) as a pale-purple powder.
$^1$H-NMR (DMSO-$d_6$) δ: 5.43 (2H, s), 6.44 (1H, d, J = 8.0 Hz), 6.65 (1H, dd, J = 2.6, 8.7 Hz), 6.93 (1H, d, J = 2.6 Hz), 7.07 (1H, d, J = 8.7 Hz), 7.47 (1H, t, J = 8.0 Hz), 7.83 (1H, d, J = 8.0 Hz), 9.20 (1H, d, J = 0.9 Hz).

(ii) Production of 2-(4-{[4-(1,2-benzoisothiazol-4-yloxy)-3-bromophenyl]amino}-5H-pyrrolo[3,2-d]pyrimidin-5-yl)ethanol p-toluenesulfonate

A mixture of 2-(4-chloro-5H-pyrrolo[3,2-d]pyrimidin-5-yl)ethyl benzoate (94 mg), 4-(1,2-benzoisothiazol-4-yloxy)-3-bromoaniline (100 mg) and isopropyl alcohol (10 mL) was stirred at 80°C for 4 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate: hexane=50:50→100:0→methanol:ethyl acetate=15:85). The object fraction was concentrated under reduced pressure. Ethanol (5 mL) and 8N aqueous sodium hydroxide solution (3.0 mL) were added to the residue, and the mixture was stirred at room temperature for 3 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography

(eluent, methanol:ethyl acetate=0:100→10:90). The object fraction was concentrated under reduced pressure and the residue was dissolved in ethyl acetate. p-Toluenesulfonic acid monohydrate (8 mg) was added, and the precipitated crystals were collected by filtration to give the title compound (20 mg) as a white powder.
$^1$H-NMR (DMSO-$d_6$) δ: 2.28 (3H, s), 3.86-4.00 (2H, m), 4.55-4.70 (2H, m), 6.37 (1H, s), 6.49-6.65 (3H, m), 7.04-7.14 (2H, m), 7.38-7.60 (4H, m), 7.66-7.77 (1H, m), 7.84-7.99 (2H, m), 8.13 (1H, s), 8.61 (1H, br s), 9.23 (1H, d, J = 0.9 Hz).

Example 46

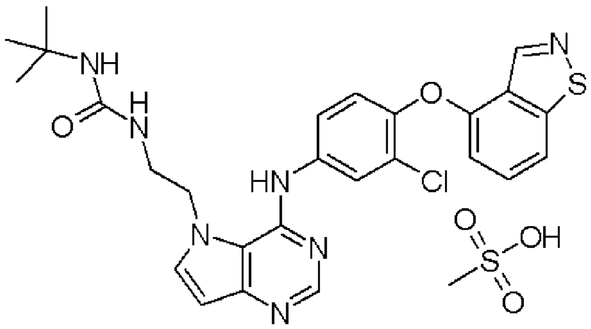

Production of 1-[2-(4-{[4-(1,2-benzoisothiazol-4-yloxy)-3-chlorophenyl]amino}-5H-pyrrolo[3,2-d]pyrimidin-5-yl)ethyl]-3-tert-butylurea methanesulfonate

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 2-isocyanato-2-methylpropane (0.4 mL), triethylamine (0.9 mL) and N,N-dimethylformamide (5 mL) was stirred at room temperature for 1 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure and the residue was dissolved in ethyl acetate. Methanesulfonic acid (25 mg) was added, and the precipitated powder was collected by filtration to give the title compound (76 mg) as a white powder.
$^1$H-NMR (DMSO-$d_6$) δ: 1.21 (9H, s), 2.30 (3H, s), 3.33-3.45 (2H, m), 4.44-4.62 (2H, m), 6.17 (1H, s), 6.24-6.34 (1H, m), 6.64 (2H, m), 7.44 (1H, d, J = 8.9 Hz), 7.52-7.63 (1H, m), 7.80-7.87 (1H, m), 7.96 (2H, s), 8.06 (1H, s), 8.73 (1H, s), 9.22 (1H, s), 10.69 (1H, br s).

Example 47

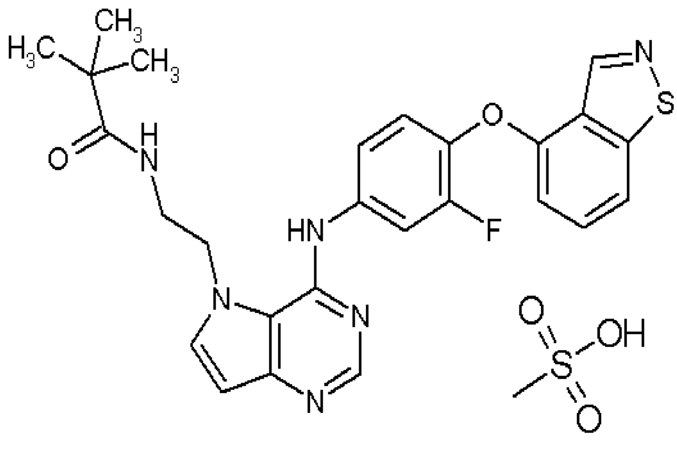

Production of N-[2-(4-{[4-(1,2-benzoisothiazol-4-yloxy)-3-fluorophenyl]amino}-5H-pyrrolo[3,2-d]pyrimidin-5-yl)ethyl]-2,2-dimethylpropanamide methanesulfonate

N-[2-(4-{[4-(1,2-Benzoisothiazol-4-yloxy)-3-fluorophenyl]amino}-5H-pyrrolo[3,2-d]pyrimidin-5-yl)ethyl]-2,2-dimethylpropanamide (100 mg) was dissolved in ethyl acetate (5 mL)/ethanol (4 mL), and methanesulfonic acid (45 mg) was added. The precipitated powder was collected by filtration to give the title compound (41 mg) as a white powder. $^1$H-NMR (DMSO-$d_6$) δ: 0.94-1.07 (9H, m), 2.29 (3H, s), 3.47 (2H, d, J = 6.0 Hz), 4.56-4.76 (2H, m), 6.55 (1H, s), 6.62-6.83 (2H, m), 7.11 (2H, d, J = 7.7 Hz), 7.41-7.67 (2H, m), 7.80-8.06 (2H, m), 8.78 (1H, s), 9.25 (1H, s), 10.14 (1H, s).

Example 48

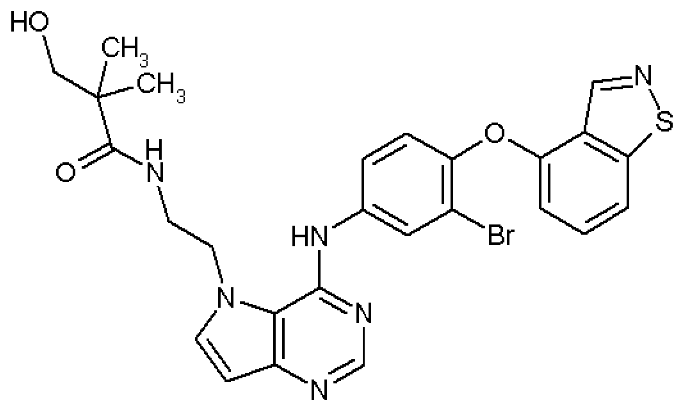

Production of N-[2-(4-{[4-(1,2-benzoisothiazol-4-yloxy)-3-bromophenyl]amino}-5H-pyrrolo[3,2-d]pyrimidin-5-yl)ethyl]-3-hydroxy-2,2-dimethylpropanamide

(i) Production of 5-(2-aminoethyl)-N-[4-(1,2-benzoisothiazol-4-yloxy)-3-bromophenyl]-5H-pyrrolo[3,2-d]pyrimidin-4-amine dihydrochloride

A mixture of tert-butyl [2-(4-chloro-5H-pyrrolo[3,2-d]pyrimidin-5-yl)ethyl]carbamate (343 mg), 4-(1,2-benzoisothiazol-4-yloxy)-3-bromoaniline (370 mg) and isopropyl alcohol (10 mL) was stirred at 80°C for 3 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol: ethyl acetate=0:100→10:90). The object fraction was concentrated under reduced pressure. 4N hydrochloric acid/ethyl acetate (4 mL) and ethanol (4 mL) were added to the residue, and the mixture was stirred at 80°C for 4 hr. Ethyl acetate was added to the reaction mixture, and the precipitate was collected by filtration to give the title compound (410 mg) as a brown powder.
$^1$H-NMR (DMSO-$d_6$) δ: 3.27-3.39 (2H, m), 4.96-5.12 (2H, m), 6.54 (1H, d, J = 7.7 Hz), 6.74-6.79 (1H, m), 7.01 (1H, s), 7.42-8.16 (6H, m), 8.30 (3H, br s), 8.78 (1H, s), 9.18 (1H, d, J = 0.8 Hz).

(ii) Production of N-[2-(4-{[4-(1,2-benzoisothiazol-4-yloxy)-3-bromophenyl]amino}-5H-pyrrolo[3,2-d]pyrimidin-5-yl)ethyl]-3-hydroxy-2,2-dimethylpropanamide

Using 5-(2-aminoethyl)-N-[4-(1,2-benzoisothiazol-4-yloxy)-3-bromophenyl]-5H-pyrrolo[3,2-d]pyrimidin-4-amine dihydrochloride (100 mg), 3-hydroxy-2,2-dimethylpropanoic acid (49 mg), N,N-dimethylformamide (10 mL), triethylamine (0.9 mL), 1-hydroxybenzotriazole (10 mg) and 1-ethyl-3-(3-dimethylaminopropyl)carbodiimide hydrochloride (240 mg), and by a reaction in the same manner as in Example 4(ii), the title compound (32 mg) was obtained as a pale-yellow powder. $^1$H-NMR (DMSO-$d_6$) δ: 0.99 (6H, s), 2.09 (2H, s), 3.40-3.46 (2H, m), 4.39-4.59 (2H, m), 4.78-4.89 (1H, m), 6.46-6.64 (2H, m), 7.37 (1H, d, J = 8.9 Hz), 7.49-7.66 (2H, m), 7.80-8.07 (3H, m), 8.28 (1H, d, J = 2.5 Hz), 8.35 (1H, s), 8.95 (1H, s), 9.25 (1H, d, J = 0.8 Hz).

Example 49

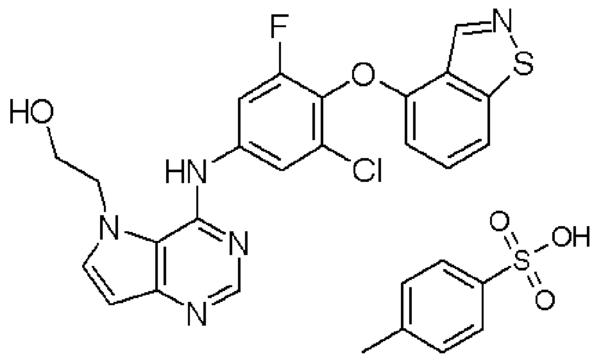

Production of 2-(4-{[4-(1,2-benzoisothiazol-4-yloxy)-3-chloro-5-fluorophenyl]amino}-5H-pyrrolo[3,2-d]pyrimidin-5-yl) ethanol p-toluenesulfonate

(i) Production of 4-(1,2-benzoisothiazol-4-yloxy)-3-chloro-5-fluoroaniline

To a solution of 1-chloro-2,3-difluoro-5-nitrobenzene (1.0 g) and 1,2-benzoisothiazol-4-ol (780 mg) in N,N-dimethylformamide (10 mL) was added potassium carbonate (1.4 g), and the mixture was stirred at room temperature for 12 hr. Water was added to the reaction mixture, and the precipitated solid was collected by filtration. The obtained solid (1.3 g) was dissolved in ethanol (30 mL), reduced iron (3.0 g) and 1N hydrochloric acid (3.0 mL) were added, and the mixture was heated under reflux at 80°C for 1 hr. The reaction mixture was cooled to room temperature, 1N aqueous sodium hydroxide solution (8 mL) was added, and the reaction mixture was filtered through celite. The obtained filtrate was diluted with ethyl acetate, washed with water and saturated brine, and dried over magnesium sulfate. Magnesium sulfate was filtered off, and the filtrate was concentrated under reduced pressure. The obtained residue was separated and purified by silica gel chromatography (hexane:ethyl acetate=49:1→1:1) to give the title compound (850 mg) as a pale-purple powder.
$^1$H-NMR (DMSO-$d_6$) δ: 5.78 (2H, s), 6.48-6.67 (3H, m), 7.49 (1H, t, J = 8.0 Hz), 7.88 (1H, d, J = 8.0 Hz), 9.27 (1H, d, J = 0.8 Hz).

(ii) Production of 2-(4-{[4-(1,2-benzoisothiazol-4-yloxy)-3-chloro-5-fluorophenyl]amino}-5H-pyrrolo[3,2-d]pyrimidin-5-yl) ethanol p-toluenesulfonate

A mixture of 2-(4-chloro-5H-pyrrolo[3,2-d]pyrimidin-5-yl)ethyl benzoate (205 mg), 4-(1,2-benzoisothiazol-4-yloxy)-3-chloro-5-fluoroaniline (200 mg) and isopropyl alcohol (5 mL) was stirred at 80°C for 5 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=50:50→100:0→methanol:ethyl acetate=15:85), and the object fraction was concentrated under reduced pressure. Methanol (20 mL) and 8N aqueous sodium hydroxide solution (3.0 mL) were added to the residue, and the mixture was stirred at room temperature for 5 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was dissolved in ethyl acetate, p-toluenesulfonic acid monohydrate (47 mg) was added, and the precipitated powder was collected by filtration to give the title compound (45 mg) as a white powder.
$^1$H-NMR (DMSO-$d_6$) δ:2.29 (3H, s), 3.85-3.94 (2H, m), 4.50-4.59 (2H, m), 6.54 (1H, d, J = 3.0 Hz), 6.61 (1H, d, J = 8.0 Hz), 7.12 (2H, d, J = 8.0 Hz), 7.48-7.51 (2H, m), 7.52 (1H, t, J = 8.0 Hz), 7.64-7.77 (2H, m), 7.85-7.96 (2H, m), 8.39 (1H, s), 9.31 (1H, s), 10.11 (1H, br s).

Example 50

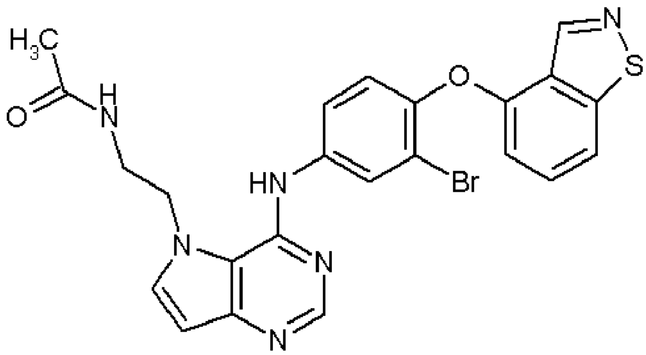

Production of N-[2-(4-{[4-(1,2-benzoisothiazol-4-yloxy)-3-bromophenyl]amino}-5H-pyrrolo[3,2-d]pyrimidin-5-yl)ethyl] acetamide

A mixture of 5-(2-aminoethyl)-N-[4-(1,2-benzoisothiazol-4-yloxy)-3-bromophenyl]-5H-pyrrolo[3,2-d]pyrimidin-4-amine dihydrochloride (150 mg), acetic acid (45 mg), triethylamine (0.9 mL), 1-hydroxybenzotriazole (10 mg), 1-ethyl-3-(3-dimethylaminopropyl)carbodiimide hydrochloride (300 mg) and N,N-dimethylformamide (10 mL) was stirred overnight at room temperature. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was crystallized from ethyl acetate/diisopropyl ether to give the title compound (125 mg) as a white powder.
$^1$H-NMR (DMSO-$d_6$) δ: 1.80 (3H, s), 3.34-3.44 (2H, m), 4.52 (2H, t, J = 6.9 Hz), 6.43-6.65 (2H, m), 7.37 (1H, d, J = 8.7 Hz), 7.54 (1H, t, J = 7.9 Hz), 7.65 (1H, s), 7.79-7.98 (2H, m), 8.17-8.29 (2H, m), 8.35 (1H, s), 8.82 (1H, s), 9.24 (1H, d, J = 0.8 Hz).
melting point: 132-135°C

Example 51

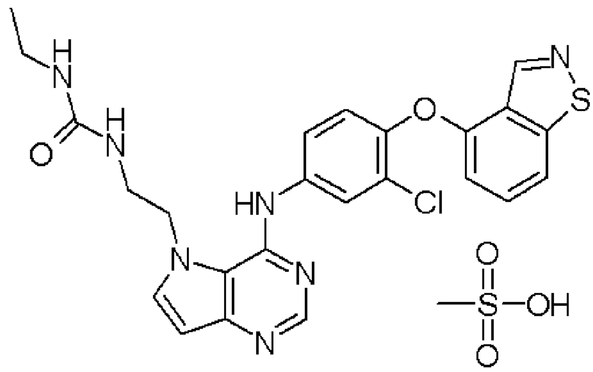

Production of 1-[2-(4-{[4-(1,2-benzoisothiazol-4-yloxy)-3-chlorophenyl]amino}-5H-pyrrolo[3,2-d]pyrimidin-5-yl)ethyl]-3-ethylurea methanesulfonate

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (200 mg), isocyanatoethane (0.7 mL), triethylamine (1.5 mL) and N,N-dimethylformamide (20

mL) was stirred at room temperature for 2 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was dissolved in ethyl acetate, methanesulfonic acid (60 mg) was added, and the precipitated powder was collected by filtration to give the title compound (150 mg) as a white powder.
$^1$H-NMR (DMSO-$d_6$) δ: 0.97 (3H, t, J = 6.9 Hz), 2.31 (3H, s), 3.00 (2H, dd, J = 5.6, 6.9 Hz), 3.40-3.54 (2H, m), 4.50-4.67 (2H, m), 6.32 (1H, s), 6.46 (1H, br s), 6.60-6.75 (2H, m), 7.44 (1H, d, J = 8.7 Hz), 7.58 (1H, t, J = 8.0 Hz), 7.81 (1H, dd, J = 2.5, 8.7 Hz), 7.98 (2H, dd, J = 2.5, 5.7 Hz), 8.07 (1H, d, J = 2.5 Hz), 8.73 (1H, s), 9.22 (1H, d, J = 0.9 Hz), 10.52 (1H, br s).
melting point: 204-206°C

Example 52

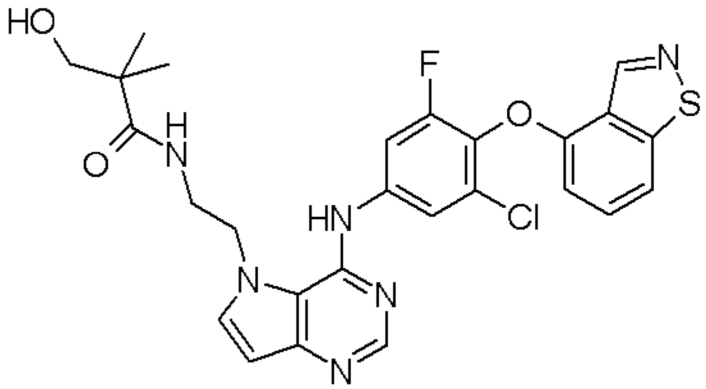

Production of N-[2-(4-{[4-(1,2-benzoisothiazol-4-yloxy)-3-chloro-5-fluorophenyl]amino}-5H-pyrrolo[3,2-d]pyrimidin-5-yl) ethyl]-3-hydroxy-2,2-dimethylpropanamide

A mixture of tert-butyl [2-(4-chloro-5H-pyrrolo[3,2-d]pyrimidin-5-yl)ethyl]carbamate (340 mg), 4-(1,2-benzoisothiazol-4-yloxy)-3-chloro-5-fluoroaniline (360 mg) and isopropyl alcohol (10 mL) was stirred at 80°C for 3 hr. Aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. 4N Hydrochloric acid/ethyl acetate (4 mL) and ethanol (4 mL) were added to the residue, and the mixture was stirred at 80°C for 4 hr. Ethyl acetate was added to the reaction mixture, and the precipitate was collected by filtration. Using the obtained solid (150 mg), 3-hydroxy-2,2-dimethylpropanoic acid (80 mg), N,N-dimethylformamide (20 mL), triethylamine (1.2 mL), 1-hydroxybenzotriazole (10 mg) and 1-ethyl-3-(3-dimethylaminopropyl)carbodiimide hydrochloride (320 mg), and by a reaction in the same manner as in Example 4(ii), the title compound (80 mg) was obtained as a pale-yellow powder.
$^1$H-NMR (DMSO-$d_6$) δ: 1.01 (6H, s), 2.50 (2H, s), 3.22-3.36 (2H, m), 4.48-4.58 (2H, m), 4.89 (1H, s), 6.54 (1H, d, J = 3.0 Hz), 6.63 (1H, d, J = 7.9 Hz), 7.53 (1H, t, J = 7.9 Hz), 7.68 (1H, d, J = 3.0 Hz), 7.88-8.01 (2H, m), 8.05-8.22 (2H, m), 8.44 (1H, s), 9.18 (1H, s), 9.34 (1H, s).
melting point: 175-177°C

Example 53

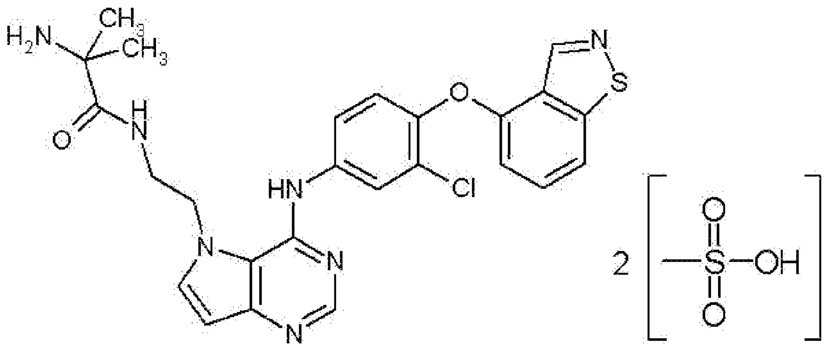

Production of N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide dimethanesulfonate

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (150 mg), N-(tert-butoxycarbonyl)-2-methylalanine (50 mg), N,N-dimethylformamide (10 mL), triethylamine (0.5 mL), 1-hydroxybenzotriazole (10 mg) and 1-ethyl-3-(3-dimethylaminopropyl)carbodiimide hydrochloride (300 mg), and by a reaction in the same manner as in Example 4(ii), the obtained residue was dissolved in ethanol (4 mL). 4N Hydrochloric acid/ethyl acetate (4 mL) was added, and the mixture was stirred at 80°C for 1 hr. 8N Aqueous sodium hydroxide solution (10 mL) was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→40:60), and the object fraction was concentrated under reduced pressure. The obtained residue was washed with water and diethyl ether, and dried under reduced pressure. The obtained compound was dissolved in ethyl acetate, methanesulfonic acid (25 mg) was added, and the precipitated solid was collected by filtration to give the title compound (103 mg) as a white powder.
$^1$H-NMR (DMSO-$d_6$) δ: 1.31 (6H, s), 2.34 (6H, s), 3.56-3.67 (2H, m), 4.71-4.86 (2H, m), 6.61-6.80 (2H, m), 7.47 (1H, d, J = 8.7 Hz), 7.52-7.73 (2H, m), 7.84-8.09 (5H, m), 8.36 (1H, s), 8.78 (1H, s), 9.22 (1H, d, J = 0.9 Hz), 9.89 (1H, br s).

Example 54

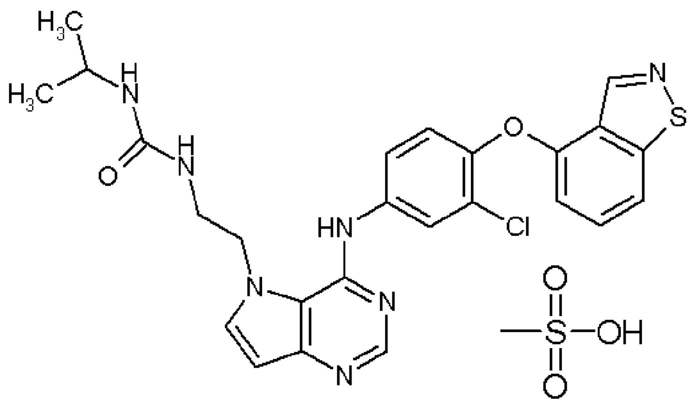

Production of 1-[2-(4-{[4-(1,2-benzoisothiazol-4-yloxy)-3-chlorophenyl]amino}-5H-pyrrolo[3,2-d]pyrimidin-5-yl)ethyl]-3-(1-methylethyl)urea methanesulfonate

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (200 mg), 2-isocyanatopropane (0.9 mL), triethylamine (1.5 mL) and N,N-dimethylformamide (20 mL) was stirred at room temperature for 2 hr. Water was added to the reaction mixture, and the mixture was extracted with ethyl acetate. The organic layer was washed successively with water and saturated brine, and dried over anhydrous

magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was dissolved in ethyl acetate, methanesulfonic acid (60 mg) was added, and the precipitated powder was collected by filtration to give the title compound (165 mg) as a white powder.
$^1$H-NMR (DMSO-$d_6$) δ: 1.02 (6H, d, J = 6.4 Hz), 2.31 (3H, s), 3.38-3.43 (2H, m), 3.57-3.74 (1H, m), 4.49-4.65 (2H, m), 6.22 (1H, d, J = 8.0 Hz), 6.28-6.36 (1H, m), 6.61-6.74 (2H, m), 7.44 (1H, d, J = 8.9 Hz), 7.57 (1H, t, J = 8.0 Hz), 7.82 (1H, dd, J = 2.5, 8.9 Hz), 7.90-8.02 (2H, m), 8.07 (1H, d, J = 2.5 Hz), 8.73 (1H, s), 9.22 (1H, d, J = 0.9 Hz), 10.52 (1H, br s). melting point: 189-190°C

Example 55

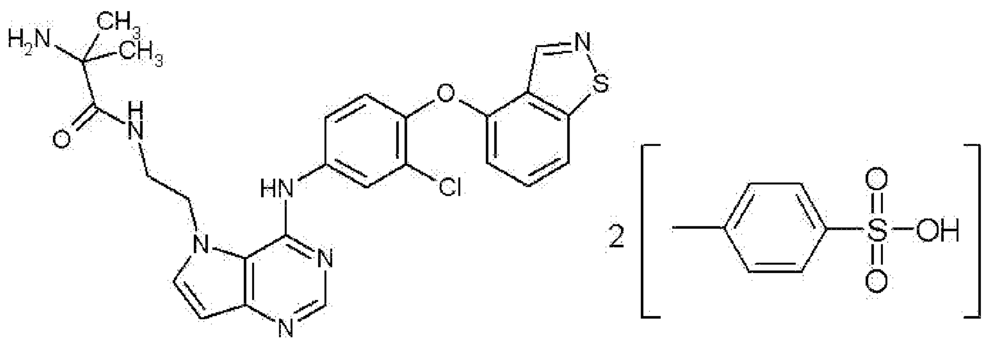

Production of N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide di-p-toluenesulfonate

N-[2-(4-{[4-(1,2-Benzoisothiazol-4-yloxy)-3-chlorophenyl]amino}-5H-pyrrolo[3,2-d]pyrimidin-5-yl)ethyl]-2-methylalaninamide (100 mg) prepared in the same manner as in Example 53 was dissolved in ethanol (1 mL), and p-toluenesulfonic acid monohydrate (60 mg) was added. Ethyl acetate (2 mL) was added, and the precipitated powder was collected by filtration to give the title compound (61 mg) as a white powder.
$^1$H-NMR (DMSO-$d_6$) δ: 1.30 (6H, s), 2.28 (6H, s), 3.54-3.67 (2H, m), 4.65-4.78 (2H, m), 6.57-6.73 (2H, m), 7.11 (4H, d, J = 7.9 Hz), 7.34-7.51 (4H, m), 7.52-7.69 (2H, m), 7.74-8.13 (6H, m), 8.31 (1H, s), 8.74 (1H, s), 9.21 (1H, d, J = 0.9 Hz), 9.74 (1H, br s).

Example 56

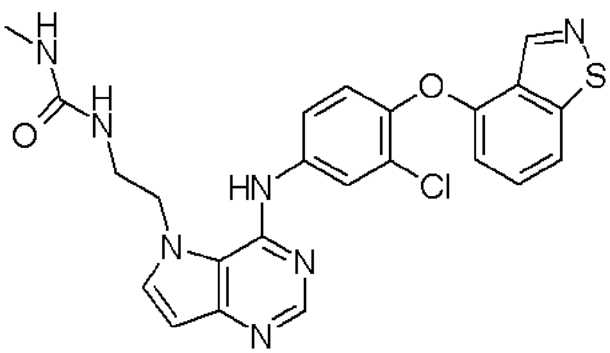

Production of 1-[2-(4-{[4-(1,2-benzoisothiazol-4-yloxy)-3-chlorophenyl]amino}-5H-pyrrolo[3,2-d]pyrimidin-5-yl)ethyl]-3-methylurea

A mixture of 5-(2-aminoethyl)-N-[4-(1,2-benzoisothiazol-4-yloxy)-3-chlorophenyl]-5H-pyrrolo[3,2-d]pyrimidin-4-amine dihydrochloride (110 mg), isocyanatomethane (200 mg), triethylamine (1.5 mL) and N,N-dimethylformamide (20 mL) was stirred at room temperature for 2 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→20:80), and the object fraction was concentrated under reduced pressure. The residue was crystallized from ethyl acetate/diisopropyl ether to give the title compound (76 mg) as a white powder.
$^1$H-NMR (DMSO-$d_6$) δ: 2.58 (3H, d, J = 4.5 Hz), 3.31-3.40 (2H, m), 4.48 (2H, t, J = 7.4 Hz), 6.14 (1H, d, J = 4.5 Hz), 6.44 (1H, br s), 6.52 (1H, d, J = 3.0 Hz), 6.60 (1H, d, J = 7.6 Hz), 7.38 (1H, d, J = 8.9 Hz), 7.53 (1H, t, J = 8.0 Hz), 7.66 (1H, d, J = 3.0 Hz), 7.86-8.02 (2H, m), 8.20 (1H, d, J = 2.6 Hz), 8.35 (1H, s), 9.15 (1H, s), 9.26 (1H, d, J = 0.8 Hz).
melting point: 241-243°C

Example 57

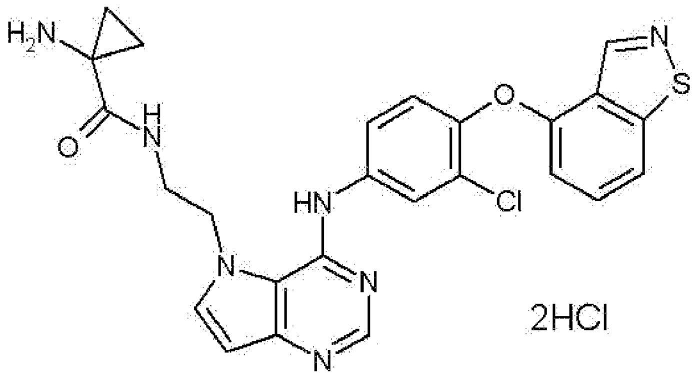

Production of 1-amino-N-[2-(4-{[4-(1,2-benzoisothiazol-4-yloxy)-3-chlorophenyl]aminol-5H-pyrrolo[3,2-dlpyrimidin-5-yl) ethyl]cyclopropanecarboxamide dihydrochloride

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (100 mg), 1-[(tert-butoxycarbonyl)amino]cyclopropanecarboxylic acid (47 mg), N,N-dimethylformamide (10 mL), triethylamine (0.9 mL), 1-hydroxybenzotriazole (10 mg) and 1-ethyl-3-(3-dimethylaminopropyl)carbodiimide hydrochloride (220 mg), and by a reaction in the same manner as in Example 4(ii), the obtained residue was dissolved in methanol (10 mL). 4N Hydrochloric acid/ethyl acetate (5 mL) was added, and the mixture was heated under reflux for 2 hr. The reaction mixture was cooled to room temperature, and the precipitated solid was collected by filtration to give the title compound (75 mg) as a white powder.
$^1$H-NMR (DMSO-$d_6$) δ: 1.06-1.30 (4H, m), 3.46-3.60 (2H, m), 4.67-4.88 (2H, m), 6.61-6.74 (2H, m), 7.44 (1H, d, J = 8.7 Hz), 7.58 (1H, t, J = 7.9 Hz), 7.65-7.76 (1H, m), 7.88 (1H, s), 7.95-8.01 (2H, m), 8.09 (1H, s), 8.54 (3H, br s), 8.71 (1H, s), 9.22 (1H, d, J = 0.8 Hz), 9.89 (1H, br s).

Example 58

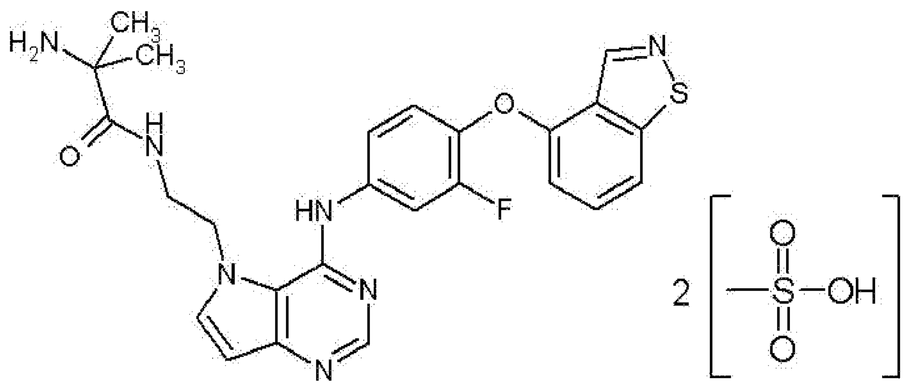

Production of N-[2-(4-{[4-(1,2-benzoisothiazol-4-yloxy)-3-fluorophenyl]amino}-5H-pyrrolo[3,2-d]pyrimidin-5-yl)ethyl]-2-methylalaninamide dimethanesulfonate

Using 5-(2-aminoethyl)-N-[4-(1,2-benzoisothiazol-4-yloxy)-3-fluorophenyl]-5H-pyrrolo[3,2-d]pyrimidin-4-amine dihydrochloride (150 mg), N-(tert-butoxycarbonyl)-2-methylalanine (100 mg), N,N-dimethylformamide (20 mL), triethylamine (0.9 mL), 1-hydroxybenzotriazole (20 mg) and 1-ethyl-3-(3-dimethylaminopropyl)carbodiimide hydrochloride (320 mg), and by a reaction in the same manner as in Example 4(ii), a residue was obtained. The residue was dissolved in ethanol (5 mL), 4N hydrochloric acid/ethyl acetate (2 mL) was added, and the mixture was stirred at 50°C for 10 hr. 1N Aqueous sodium hydroxide solution (10 mL) was added to the reaction mixture, and the mixture was extracted with ethyl acetate. The organic layer was washed successively with water and saturated brine, and dried over anhydrous magnesium sulfate. The solvent was evaporated under reduced pressure, and the obtained residue was dissolved in ethanol (2 mL). A solution (5 mL) of methanesulfonic acid (23 mg) in ethyl acetate was added, and the precipitated solid was collected by filtration to give the title compound (112 mg) as a white powder.
$^1$H-NMR (DMSO-$d_6$) δ: 1.31 (6H, s), 2.31 (6H, s), 3.55-3.65 (2H, m), 4.64-4.83 (2H, m), 6.67 (1H, d, J = 3.0 Hz), 6.76 (1H, d, J = 7.7 Hz), 7.41-7.65 (3H, m), 7.74-7.89 (2H, m), 7.94-8.07 (3H, m), 8.35 (1H, s), 8.74 (1H, s), 9.24 (1H, d, J = 0.9 Hz), 9.78 (1H, br s).

Example 59

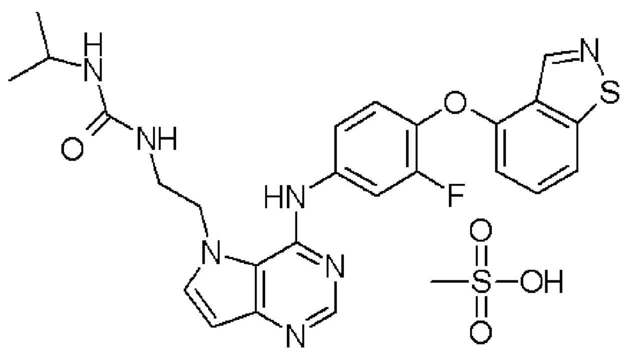

Production of 1-[2-(4-{[4-(1,2-benzoisothiazol-4-yloxy)-3-fluorophenyl]amino}-5H-pyrrolo[3,2-d]pyrimidin-5-yl)ethyl]-3-(1-methylethyl)urea methanesulfonate

A mixture of 5-(2-aminoethyl)-N-[4-(1,2-benzoisothiazol-4-yloxy)-3-fluorophenyl]-5H-pyrrolo[3,2-d]pyrimidin-4-amine dihydrochloride (100 mg), 2-isocyanatopropane (40 mg), triethylamine (0.2 mL) and N,N-dimethylformamide (15 mL) was stirred at room temperature for 1 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90), and the object fraction was concentrated under reduced pressure. The residue was dissolved in ethyl acetate, methanesulfonic acid (23 mg) was added,

and the precipitated powder was collected by filtration to give the title compound (118 mg) as a white powder. $^1$H-NMR (DMSO-$d_6$) δ: 1.01 (6H, d, J = 6.4 Hz), 2.30 (3H, s), 3.34-3.48 (2H, m), 3.52-3.70 (1H, m), 4.59 (2H, t, J = 7.5 Hz), 6.22 (1H, d, J = 7.7 Hz), 6.33 (1H, br s), 6.68 (1H, d, J = 3.0 Hz), 6.73 (1H, d, J = 7.7 Hz), 7.41-7.61 (2H, m), 7.63-7.72 (1H, m), 7.90 (1H, dd, J = 2.5, 12.7 Hz), 7.98 (2H, dd, J = 2.5, 5.7 Hz), 8.74 (1H, s), 9.25 (1H, d, J = 0.9 Hz), 10.53 (1H, br s).

Example 60

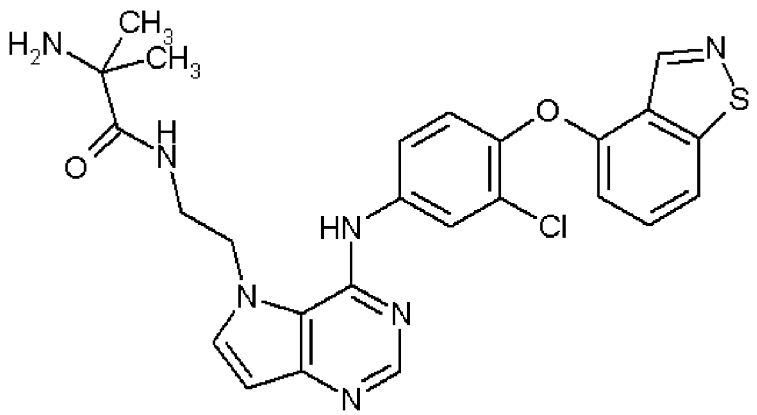

Production of N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (2.0 g), N-(tert-butoxycarbonyl)-2-methylalanine (1.0 g), N,N-dimethylformamide (200 mL), triethylamine (4.5 mL), 1-hydroxybenzotriazole (400 mg) and 1-ethyl-3-(3-dimethylaminopropyl)carbodiimide hydrochloride (3.2 g), and by a reaction in the same manner as in Example 4(ii), a residue was obtained. The obtained residue was dissolved in ethanol (100 mL), 4N hydrochloric acid/ethyl acetate (50 mL) was added, and the mixture was stirred at 70°C for 10 hr. 1N Aqueous sodium hydroxide solution (400 mL) was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to basic silica gel column chromatography (eluent, methanol:ethyl acetate=0:100→10:90), and the object fraction was concentrated under reduced pressure. The obtained residue was crystallized from ethyl acetate/diisopropyl ether to give the title compound (1.3 g) as a white powder.
$^1$H-NMR (DMSO-$d_6$) δ: 1.12 (6H, s), 1.85 (2H, br s), 3.44 (2H, t, J = 6.6 Hz), 4.55 (2H, t, J = 6.6 Hz), 6.50 (1H, d, J = 3.0 Hz), 6.61 (1H, d, J = 7.6 Hz), 7.39 (1H, d, J = 8.0 Hz), 7.54 (1H, t, J = 8.0 Hz), 7.61 (1H, d, J = 3.0 Hz), 7.84-7.91 (1H, m), 7.92 (1H, d, J = 8.0 Hz), 8.12 (1H, br s), 8.22 (1H, br s), 8.35 (1H, s), 8.90 (1H, br s), 9.26 (1H, d, J = 0.8 Hz).
melting point: 198°C

Example 61

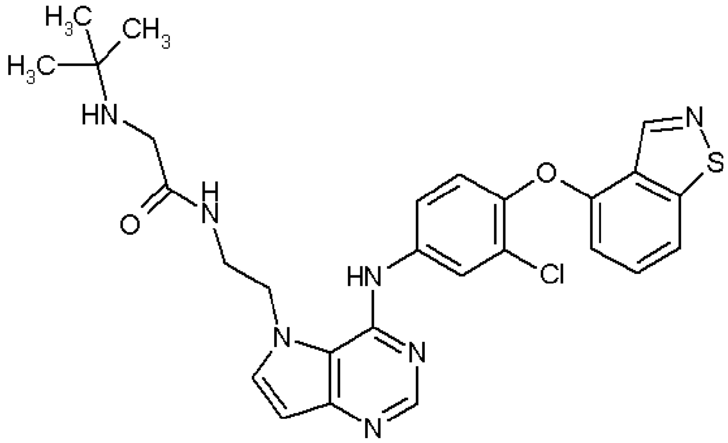

Production of N-[2-(4-{[4-(1,2-benzoisothiazol-4-yloxy)-3-chlorophenyl]amino}-5H-pyrrolo[3,2-d]pyrimidin-5-yl) ethyl]-$N^2$-tert-butylglycinamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (200 mg), N-tert-butylglycine (220 mg), N,N-dimethylformamide (25 mL), triethylamine (1.2 mL), 1-hydroxybenzotriazole (10 mg) and 1-ethyl-3-(3-dimethylaminopropyl)carbodiimide hydrochloride (370 mg), and by a reaction in the same manner as in Example 4(ii), the title compound (120 mg) was obtained as a white powder.
$^1$H-NMR (DMSO-$d_6$) δ: 0.95 (9H, s), 1.99 (1H, br s), 3.01 (2H, s), 3.48 (2H, br s), 4.55 (2H, br s), 6.52 (1H, d, J = 8.9 Hz), 6.60 (1H, d, J = 8.0 Hz), 7.39 (1H, d, J = 8.9 Hz), 7.54 (1H, t, J = 8.0 Hz), 7.63 (1H, br s), 7.83 (1H, br s), 7.92 (1H, d, J = 8.0 Hz), 8.11 (2H, br s), 8.35 (1H, s), 8.82 (1H, br s), 9.25 (1H, d, J = 0.8 Hz).
melting point: 178-180°C

Example 62

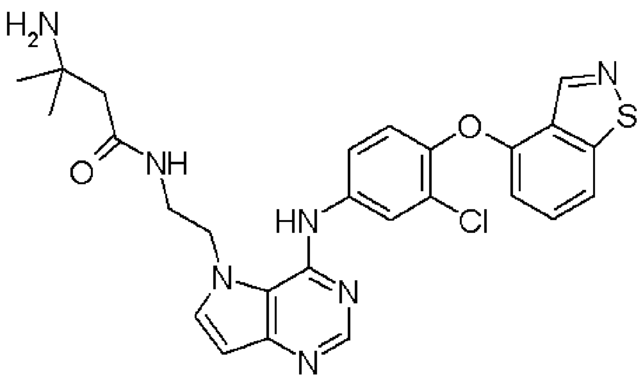

Production of 3-amino-N-[2-(4-{[4-(1,2-benzoisothiazol-4-yloxy)-3-chlorophenyl]amino}-5H-pyrrolo[3,2-d]pyrimidin-5-yl) ethyl]-3-methylbutanamide

Using 5-(2-aminoethyl)-N-[4-(1,2-benzoisothiazol-4-yloxy)-3-chlorophenyl]-5H-pyrrolo[3,2-d]pyrimidin-4-amine dihydrochloride (150 mg), 3-[(tert-butoxycarbonyl)amino]-3-methylbutanoic acid (100 mg), N,N-dimethylformamide (10 mL), triethylamine (1.7 mL), 1-hydroxybenzotriazole (20 mg) and 1-ethyl-3-(3-dimethylaminopropyl)carbodiimide hydrochloride (260 mg), and by a reaction in the same manner as in Example 4(ii), a residue was obtained. The obtained residue was dissolved in ethanol (10 mL), 4N hydrochloric acid/ethyl acetate (5 mL) was added, and the mixture was stirred at 70°C for 10 hr. 1N Aqueous sodium hydroxide solution (20 mL) was added to the reaction mixture, and the mixture was extracted with ethyl acetate. The organic layer was washed successively with water and saturated brine, and dried over anhydrous magnesium sulfate. The solvent was evaporated under reduced pressure and the obtained

residue was subjected to basic silica gel column chromatography (eluent, methanol:ethyl acetate=0:100→10:90), and the object fraction was concentrated under reduced pressure. The obtained residue was crystallized from ethyl acetate/diisopropyl ether to give the title compound (70 mg) as a white powder.
$^1$H-NMR (DMSO-$d_6$) δ: 1.04 (6H, s), 1.81 (2H, br s), 2.12 (2H, s), 3.37-3.50 (2H, m), 4.45-4.62 (2H, m), 6.50 (1H, d, J = 3.0 Hz), 6.58-6.63 (1H, m), 7.38 (1H, d, J = 8.9 Hz), 7.53 (1H, t, J = 8.0 Hz), 7.66 (1H, d, J = 3.0 Hz), 7.83-7.91 (1H, m), 7.90-7.96 (1H, m), 8.11 (1H, br s), 8.35 (1H, s), 8.59 (1H, br s), 8.96 (1H, br s), 9.25 (1H, d, J = 0.9 Hz).

Example 63

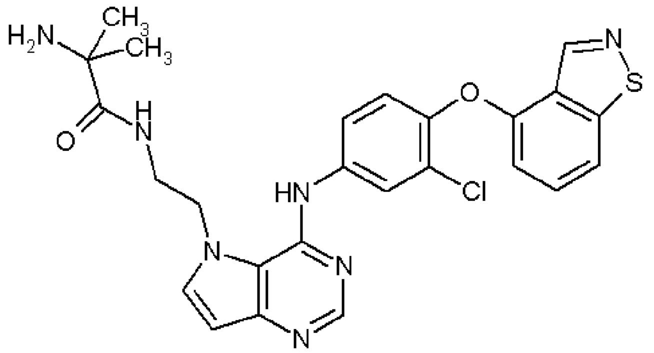

Production of N-[2-(4-{[4-(1,2-benzoisothiazol-4-yloxy)-3-chlorophenyl]amino}-5H-pyrrolo[3,2-d]pyrimidin-5-yl)ethyl]-2-methylalaninamide

(i) Production of tert-butyl (2-{[2-(4-{[4-(1,2-benzoisothiazol-4-yloxy)-3-chlorophenyl]amino}-5H-pyrrolo[3,2-d]pyrimidin-5-yl)ethyl]amino}-1,1-dimethyl-2-oxoethyl)carbamate

To a solution of 5-(2-aminoethyl)-N-[4-(1,2-benzoisothiazol-4-yloxy)-3-chlorophenyl]-5H-pyrrolo[3,2-d]pyrimidin-4-amine dihydrochloride (94 g), N-(tert-butoxycarbonyl)-2-methylalanine (56 g) and 1-hydroxybenzotriazole (25 g) in acetonitrile (1.40 L) were added 1-ethyl-3-(3-dimethylaminopropyl)carbodiimide hydrochloride (53 g) and triethylamine (93 g) under ice-cooling, and the mixture was stirred at room temperature for 5 hr. Water (1.0 L) and ethyl acetate (1.0 L) were added to reaction system, and the organic layer was washed with saturated brine (250 mL) and dried over magnesium sulfate. The mixture was concentrated under reduced pressure, and the residue was separated and purified by silica gel column chromatography (silica gel 1500 g, eluent, ethyl acetate:methanol=1:0→92:8). The solvent was evaporated and dried under reduced pressure to give the title compound (115 g) as a white powder.
$^1$H-NMR (DMSO-$d_6$) δ: 1.26 (6H, s), 1.34 (9H, s), 3.33-3.42 (2H, m), 4.49 (2H, br s), 6.48 (1H, d, J = 2.8 Hz), 6.61 (1H, d, J = 8.0 Hz), 6.98 (1H, br s), 7.38 (1H, d, J = 9.0 Hz), 7.54 (1H, t, J = 8.0 Hz), 7.62 (1H, d, J = 3.2 Hz), 7.90-8.14 (4H, m), 8.37 (1H, s), 8.90 (1H, s), 9.26 (1H, s).

(ii) Production of N-[2-(4-{[4-(1,2-benzoisothiazol-4-yloxy)-3-chlorophenyllaminol-5H-pyrrolo[3,2-d]pyrimidin-5-yl)ethyl]-2-methylalaninamide

To a solution of tert-butyl (2-{[2-(4-{[4-(1,2-benzoisothiazol-4-yloxy)-3-chlorophenyl]amino}-5H-pyrrolo[3,2-d]pyrimidin-5-yl)ethyl]amino}-1,1-dimethyl-2-oxoethyl)carbamate (115 g) obtained above in methanol (600 mL) was slowly added concentrated hydrochloric acid (400 mL) at an inside temperature of 0°C, and the mixture was stirred at room temperature for 4 hr. 8N Aqueous NaOH solution (600 mL), 4N aqueous NaOH solution (400 mL) and ethyl acetate (1.0L) were added under ice-cooling. The organic layer was washed with saturated brine (250 mL), dried over magnesium sulfate and concentrated under reduced pressure. The residue was separated and purified by basic silica gel column chromatography (basic silica gel 800 g, eluent, ethyl acetate:methanol=1:0→95:5). The solvent was evaporated, and the residue was suspended in ethyl acetate (300 mL). Diisopropyl ether (500 mL) was added, and the crystals were collected by filtration. 5% Water-containing ethanol (930 mL) was added to the obtained crude crystals (92.7 g), and the mixture was heated under reflux for 1 hr. The obtained solution was filtered hot, and the filtrate was stirred at an inside temperature of 60°C. Heptane (1.12 L) was added dropwise to this solution while maintaining at an inside temperature of 60°C, and the mixture was stirred at 60°C for 1 hr. The mixture was allowed to cool, and stirred at room temperature

for 1 hr. The precipitated crystals were collected by filtration and washed with a mixed solvent (150 mL) of heptane/ethanol=1/1 to give the title compound (74 g) as colorless crystals.
$^1$H-NMR (DMSO-$d_6$) δ: 1.12 (6H, s), 1.85 (2H, br s), 3.44 (2H, t, J = 6.6 Hz), 4.55 (2H, t, J = 6.6 Hz), 6.50 (1H, d, J = 3.0 Hz), 6.61 (1H, d, J = 7.6 Hz), 7.39 (1H, d, J = 8.0 Hz), 7.54 (1H, t, J = 8.0 Hz), 7.61 (1H, d, J = 3.0 Hz), 7.84-7.91 (1H, m), 7.92 (1H, d, J = 8.0 Hz), 8.12 (1H, br s), 8.22 (1H, br s), 8.35 (1H, s), 8.90 (1H, br s), 9.26 (1H, d, J = 0.8 Hz).
melting point: 198°C

**Formulation Example 1** (amount per tablet)

| | | |
|---|---|---|
| (1) | compound obtained in Example 7 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound obtained in Example 7, 60.0 mg of lactose and 35.0 mg of corn starch is granulated through a 1 mm-mesh sieve using 0.03 ml of a 10% by weight aqueous solution of gelatin (3.0 mg of gelatin), after which the granules are dried at 40°C and filtered again. The obtained granules are mixed with 2.0 mg of magnesium stearate and compressed. The obtained core tablets are coated with a sugar coat comprising a suspension of sucrose, titanium dioxide, talc and gum arabic and polished with beeswax to yield sugar-coated tablets.

**Formulation Example 2** (dose per tablet)

| | | |
|---|---|---|
| (1) | compound obtained in Example 7 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Example 7 and 3.0 mg of magnesium stearate are granulated using 0.07 ml of an aqueous solution of soluble starch (7.0 mg of soluble starch), after which these granules are dried and mixed with 70.0 mg of lactose and 50.0 mg of corn starch. This mixture is compressed to yield tablets.

**Experimental Example 1A** Cloning of human HER2 gene and preparation of recombinant baculovirus

Human HER2 gene was cloned by RT-PCR using total RNA prepared from MCF7 cells as a template. The primer used for RT-PCR was prepared in consideration of the information of nucleotide sequence (Genbank Accession No. M11730) of HER2 gene by adding a nucleotide sequence encoding DYKDDDD peptide and a restriction enzyme recognition sequence to a nucleotide sequence (corresponding to 2176-3918 of Genbank Accession No. M11730 as a base, 676-1255 amino acids of Genbank Accession No. NP_004439 as a protein) encoding the HER2 intracellular domain region such that the protein contains an N-terminal DYKDDDD peptide tag. The primer nucleotide sequence is shown below.

HER2-U:5'-AATTAAGTCGACATGGACTACAAAGACGATGACGACAAGCGACGGCAGCAGAAGATCCGGAA GTAC-3'(SEQ ID NO:1)
and
HER2-L: 5'-AATTAAGCATGCTCACACTGGCACGTCCAGACCCAGGTACTC-3' (SEQ ID NO:2)

The RT reaction was conducted using SuperScript First-Strand Synthesis System for RT-PCR (Invitrogen) and the PCR reaction was conducted using a KOD-plus kit (TOYOBO). The obtained PCR product was electrophoresed on agarose gel (1%), the DNA fragment amplified by PCR was recovered from the gel, and then digested with restriction enzymes Sal I and Sph I. The DNA treated with the restriction enzymes was electrophoresed on agarose gel (1%), and

the obtained DNA fragment was recovered and ligated to plasmid pFASTBAC1 (Invitrogen) digested with restriction enzymes Sal I and Sph I to give expression plasmid pFB-HER2. The nucleotide sequence of the insertion fragment was confirmed and found to be identical with the nucleotide sequence (2176-3918 of Genbank Accession M11730) of HER2 intracellular domain. Furthermore, using BAC-TO-BAC Baculovirus Expression System (Invitrogen), recombinant baculovirus BAC-HER2 was prepared.

**Experimental Example 1B** Preparation of HER2 intracellular domain protein

SF-21 cells were sown at $1\times10^6$ cells/mL in Sf-900II SFM medium (1 L, Invitrogen) containing 10% fetal bovine serum (trace), 50 mg/L gentamicin (Invitrogen) and 0.1% Pluronic F-68 (Invitrogen), and shaking culture was performed using a 2 L volume Erlenmeyer flask at 27°C, 100 rpm. After culturing for 24 hr, recombinant baculovirus BAC-HER2 (13.4 mL) was added, and the mixture was further cultured for 3 days. The culture medium was centrifuged at 2,000 rpm for 5 min to give virus-infected cells. The infected cells were washed with a phosphate buffered saline (Invitrogen), centrifuged under the same conditions, and the cells were preserved at -80°C. The cryopreserved cells were thawed in ice, suspended in buffer A (50 mM Tris buffer (30 mL, pH 7.4) containing 20% glycerol, 0.15 M NaCl) supplemented with Complete Protease Inhibitor (Boehringer), and ruptured 3 times with a Polytron homogenizer (Kinematica) at 20,000 rpm for 30 sec. The ruptured solution was clarified by centrifugation at 40,000 rpm for 30 min, and filtered with a 0.45 μm filter. The filtrate was passed through a column packed with Anti-FLAG M2 Affinity Gel (4 mL, Sigma-Aldrich) at a flow rate of about 0.5 mL/min. The column was washed with buffer A, and eluted with buffer A containing 100 μg/mL of FLAG peptide. The eluent was concentrated with Vivaspin 20 (Vivascience) having a molecular weight cut off of 30K. The concentrate was purified by gel filtration using Hi Load 16/60 Superdex 200 pg (GE Healthcare Bioscience) equilibrated with buffer A. The fractions containing HER2 intracellular domain were collected and cryopreserved at -80°C.

**Experimental Example 1C** Determination of HER2 kinase inhibitory activity

HER2 kinase reaction was performed using a 96 well plate. As a buffer for kinase reaction, a buffer having a composition of 50 mM Tris-HCl (pH 7.5), 5 mM $MnCl_2$, 2 mM dithiothreitol, 0.01% Tween-20 was used. The compound of the present invention was dissolved in dimethyl sulfoxide (DMSO), and diluted with the kinase reaction buffer so that DMSO concentration would be 0.1% during kinase reaction. This compound solution (10 μL) was mixed with a kinase reaction buffer (20 μL) containing HER2 intracellular domain (0.625 μg/ mL) obtained in Experimental Example 1B and polypeptide substrate poly-Glu:Tyr (4:1) (Sigma Ltd., 12.5 μg/ mL), and the mixture was stood at room temperature for 5 min. Then, a kinase reaction buffer (20 μL) containing 125 μM ATP and 45 μCi/mL [$\gamma$-$^{32}$P]ATP was added, and the kinase reaction was initiated with 50 μL of the final reaction mixture. A kinase reaction was performed at room temperature for 10 min, and the kinase reaction was quenched by addition of 20% TCA solution (50 μL). The mixture after completion of the reaction was stood at room temperature for 30 min, and acid insoluble fractions in the mixture after completion of the reaction were collected in a 96 well GF/C filter plate (PerkinElmer) using a cell harvester (PerkinElmer). Thereafter, the filter containing the acid insoluble fractions was washed with 3% phosphoric acid solution. After washing, the filter plate was dried at 45°C for 60 min, and 25 μL of MicroScinti 0 (PerkinElmer) was added. The radioactivity was measured using TopCount (PerkinElmer). HER2 kinase inhibitory rate (%) of the test compound was calculated by the following formula:

$$\text{Inhibitory rate (\%)} = (1 - (\text{count of test compound} - \text{blank}) \div (\text{control} - \text{blank})) \times 100$$

The count of the solution reacted without addition of the compound was used as a "control", and the count of the solution without the compound and HER2 intracellular domain was used as a "blank". The results of the inhibitory rate of the compounds are shown in Table 1.

From the foregoing, it was shown that the compounds of the present invention strongly inhibited the activity of HER2 kinase.

Table 1

| Example No. (compound No.) | inhibitory rate (%) at 1.0 μL |
|---|---|
| 2 | 100 |

(continued)

| Example No. (compound No.) | inhibitory rate (%) at 1.0 μL |
|---|---|
| 4 | 100 |
| 13 | 100 |
| 20 | 98 |
| 31 | 100 |
| 44 | 98 |
| 52 | 99 |
| 60 | 100 |

**Experimental Example 2A** Cloning of human EGFR gene and preparation of recombinant baculovirus

Human EGFR gene was cloned by RT-PCR using total RNA prepared from A431 cells as a template. The primer used for RT-PCR was prepared in consideration of the information of nucleotide sequence (Genbank Accession No. X00588) of EGFR gene by adding a nucleotide sequence encoding DYKDDDD peptide and a restriction enzyme recognition sequence to a nucleotide sequence (corresponding to 2191-3819 of Genbank Accession No. X00588 as a base, 669-1210 amino acids of Genbank Accession No. NP_005219 as a protein) encoding the EGFR intracellular domain region such that the protein contains an N-terminal DYKDDDD peptide tag. The primer nucleotide sequence is shown below.

EGFR-U:5'-AATTAAGTCGACATGGACTACAAAGACGATGACGACCGAAGGCGCCACATCGTTCGGAAGCG CACG-3'(SEQ ID NO: 3)
and
EGFR-L:5'-AATTAAGCATGCTCATGCTCCAATAAATTCACTGCTTTGTGG-3' (SEQ ID NO: 4)

The RT reaction was conducted using SuperScript First-Strand Synthesis System for RT-PCR (Invitrogen) and the PCR reaction was conducted using a KOD-plus kit (TOYOBO). The obtained PCR product was electrophoresed on agarose gel (1%), the DNA fragment amplified by PCR was recovered from the gel, and then digested with restriction enzymes Sal I and Sph I. The DNA treated with the restriction enzymes was electrophoresed on agarose gel (1%), and the obtained DNA fragment was recovered and ligated to plasmid pFASTBAC1 (Invitrogen) digested with restriction enzymes Sal I and Sph I to give expression plasmid pFB-EGFR. The nucleotide sequence of the insertion fragment was confirmed and found to be identical with the nucleotide sequence (2191-3819 of Genbank Accession X00588) of EGFR intracellular domain. Furthermore, using BAC-TO-BAC Baculovirus Expression System (Invitrogen), recombinant baculovirus BAC-EGFR was prepared.

**Experimental Example 2B** Preparation of human EGFR intracellular domain protein

SF-21 cells were sown at $1\times10^6$ cells/mL in Sf-900II SFM medium (1 L, Invitrogen) containing 10% fetal bovine serum (trace), 50 mg/L gentamicin (Invitrogen) and 0.1% Pluronic F-68 (Invitrogen), and shaking culture was performed using a 2 L volume Erlenmeyer flask at 27°C, 100 rpm. After culturing for 24 hrs, recombinant baculovirus BAC-EGFR (13.4 mL) was added, and the mixture was further cultured for 3 days. The culture medium was centrifuged at 2,000 rpm for 5 min to give virus-infected cells. The infected cells were washed with a phosphate buffered saline (Invitrogen), centrifuged under the same conditions, and the cells were preserved at -80°C. The cryopreserved cells were thawed in ice, suspended in buffer A (50 mM Tris buffer (30 mL, pH 7.4) containing 20% glycerol, 0.15 M NaCl) supplemented with Complete Protease Inhibitor (Boehringer), and ruptured 3 times with a Polytron homogenizer (Kinematica) at 20,000 rpm for 30 sec. The ruptured solution was clarified by centrifugation at 40,000 rpm for 30 min, and filtered with a 0.45 μm filter. The filtrate was passed through a column packed with Anti-FLAG M2 Affinity Gel (4 mL, Sigma-Aldrich) at a flow rate of about 0.5 mL/min. The column was washed with buffer A, and eluted with buffer A containing 100 μg/mL of FLAG peptide. The eluent was concentrated with Vivaspin 20 (Vivascience) having a molecular weight cut off of 30K. The concentrate was purified by gel filtration using Hi Load 16/60 Superdex 200 pg (GE Healthcare Bioscience) equilibrated with buffer A. The fractions containing EGFR intracellular domain were collected and cryopreserved at -80°C.

**Experimental Example 2C** Determination of EGFR kinase inhibitory activity

EGFR kinase reaction was performed using a 96 well plate. As a buffer for kinase reaction, a buffer having a composition of 50 mM Tris-HCl (pH 7.5), 5 mM $MnCl_2$, 2 mM dithiothreitol, 0.01% Tween-20 was used. The compound of the present invention was dissolved in dimethyl sulfoxide (DMSO), and diluted with the kinase reaction buffer so that DMSO concentration would be 0.1% during kinase reaction. This compound solution (10 μL) was mixed with a kinase reaction buffer (20 μL) containing EGFR intracellular domain (0.625 μg/ mL) obtained in Experimental Example 2B and polypeptide substrate poly-Glu:Tyr (4:1) (Sigma Ltd., 12.5 μg/ mL), and the mixture was stood at room temperature for 5 min. Then, a kinase reaction buffer (20 μL) containing 125 μM ATP and 45 μCi/mL [$\gamma$-$^{32}$P] ATP was added, and the kinase reaction was initiated with 50 μL of the final reaction mixture. A kinase reaction was performed at room temperature for 10 min, and the kinase reaction was quenched by addition of 20% TCA solution (50 μL). The mixture after completion of the reaction was stood at room temperature for 30 min, and acid insoluble fractions in the mixture after completion of the reaction were collected in a 96 well GF/C filter plate (PerkinElmer) using a cell harvester (PerkinElmer). Thereafter, the filter containing the acid insoluble fractions was washed with 3% phosphoric acid solution. After washing, the filter plate was dried at 45°C for 60 min, and 25 μL of MicroScinti 0 (PerkinElmer) was added. The radioactivity was measured using TopCount (PerkinElmer). EGFR kinase inhibitory rate (%) of the test compound was calculated by the following formula:

Inhibitory rate (%)=(1-(count of test compound - blank)÷(control - blank))×100

The count of the solution reacted without addition of the compound was used as a "control", and the count of the solution without the compound and EGFR intracellular domain was used as a "blank". The results of the inhibitory rate of the compounds are shown in Table 2.
From the foregoing, it was shown that the compounds of the present invention strongly inhibited the activity of EGFR kinase.
Table 2

| Example No. (compound No.) | inhibitory rate (%) at 1.0 μL |
|---|---|
| 1 | 99 |
| 10 | 98 |
| 12 | 95 |
| 24 | 100 |
| 45 | 99 |
| 55 | 99 |
| 62 | 99 |

**Experimental Example 3** Inhibitory action on breast cancer cell BT-474 proliferation in vitro

A suspension of human breast cancer cell BT-474 (100 μl (6,000 cells)) were plated in a 96-well microplate and cultured in an incubator (37°C, 5% carbon dioxide). On the following day, 100 μl of a solution of each test compound previously diluted serially in 2-fold, was added, and the cells were cultured for 5 days. After the culture medium containing the test compound was removed, the cells were washed and fixed with 50% trichloroacetic acid, after which a 0.4% (w/v) SRB dye solution (dissolved in 1% acetic acid) was added to stain as well as fix the cell protein (Skehan et al., Journal of the National Cancer Institute, Vol. 82, pp. 1107-1112, 1990). After washing with a 1% acetic acid solution, 100 μl of extract solution (10 mM, Tris buffer) was added to extract the dye and the absorbance was measured at a wavelength of 550 nm to quantify the amount of cells as protein content. Taking as 100% the protein content for the control group, which received no test compound solution, the ratio of the residual protein content for each treatment group was determined, and the compound concentration required to achieve 50% suppression of the residual cell content relative to the control ($IC_{50}$ value) was calculated. The results are shown in Table 3.
Table 3

| Example No. (compound No.) | $IC_{50}$ (nM) |
|---|---|
| 3 | <100 |
| 7 | <100 |
| 11 | <100 |
| 39 | <100 |
| 53 | <100 |
| 54 | <100 |

**Industrial Applicability**

According to the present invention, pyrrolo[3,2-d]pyrimidine compound, a production method thereof and use thereof are provided. These fused pyrimidine compounds have a superior tyrosine kinase inhibitory action, are highly safe, and are sufficiently satisfactory as pharmaceutical products.

This application is based on a patent application No. 2008-063363 filed in Japan, the contents of which are incorporated in full herein by this reference.

## Claims

**1.** A compound represented by the formula:

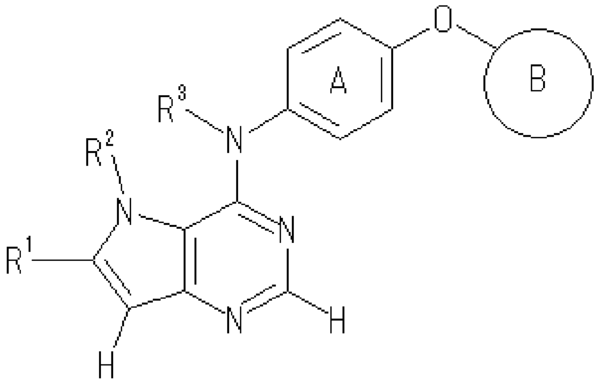

(I)

wherein

ring A is an optionally substituted benzene ring;
ring B is an optionally substituted benzoisothiazole ring;
$R^1$ is a hydrogen atom, a halogen atom, or an optionally substituted group bonded via a carbon atom, a nitrogen atom or an oxygen atom;
$R^2$ is a hydrogen atom, or an optionally substituted group bonded via a carbon atom or a sulfur atom;
$R^3$ is a hydrogen atom or an optionally substituted aliphatic hydrocarbon group; or
$R^1$ and $R^2$, or $R^2$ and $R^3$ are optionally bonded to each other to form an optionally substituted ring structure; or
$R^3$ is optionally bonded to the carbon atom on ring A to form an optionally substituted ring structure;
or a salt thereof.

**2.** The compound of claim 1, wherein $R^1$ is a hydrogen atom or a halogen atom.

**3.** The compound of claim 1, wherein $R^2$ is a hydrogen atom or an optionally substituted alkyl group.

**4.** The compound of claim 1, wherein $R^3$ is a hydrogen atom.

**5.** The compound of claim 1, wherein ring A is a benzene ring optionally substituted by 1 or 2 substituents selected from the group consisting of (1) a halogen atom, (2) $C_{1-4}$ alkyl optionally having 1 to 3 halogen atoms and (3) $C_{1-4}$ alkoxy.

**6.** The compound of claim 1, wherein ring B is a benzoisothiazole ring optionally having one $C_{1-6}$ alkyl.

**7.** The compound of claim 1, wherein $R^1$ is a hydrogen atom or a chlorine atom;

$R^2$ is a $C_{1-6}$ alkyl group optionally having one substituent selected from

(1) hydroxy,
(2) $C_{1-6}$ alkyl-carbonylamino optionally having 1 to 3 substituents selected from (1') halogen atom, (2') amino, (3') $C_{1-6}$ alkylamino, (4') hydroxy and (5') $C_{1-6}$ alkylsulfonyl,
(3) $C_{3-6}$ cycloalkyl-carbonylamino optionally having one substituent selected from (1') cyano, (2') amino and (3') hydroxy,
(4) $C_{2-6}$ alkynyl-carbonylamino,
(5) heterocyclyl-carbonylamino,
(6) $C_{1-6}$ alkyl-aminocarbonyl-amino,
(7) $C_{1-6}$ alkyl-carbonyloxy optionally having one carboxy and
(8) $C_{1-6}$ alkylsulfonyl;

$R^3$ is a hydrogen atom;
ring A is a benzene ring optionally substituted by 1 or 2 substituents selected from the group consisting of a fluorine atom, a chlorine atom, a bromine atom, methyl, trifluoromethyl and methoxy; and
ring B is a benzoisothiazole ring optionally having one methyl, which is bonded to oxygen atom at the 4- or 6-position,

**8.** 2-(4-{[4-(1,2-Benzoisothiazol-4-yloxy)-3-chlorophenyl]amino}-5H-pyrrolo[3,2-d]pyrimidin-5-yl)ethanol or a salt thereof.

**9.** N-[2-(4-{[4-(1,2-Benzoisothiazol-4-yloxy)-3-chlorophenyl]amino}-5H-pyrrolo[3,2-d]pyrimidin-5-yl)ethyl]acetamide or a salt thereof.

**10.** N-[2-(4-{[4-(1,2-Benzoisothiazol-4-yloxy)-3-chlorophenyl]amino}-5H-pyrrolo[3,2-d]pyrimidin-5-yl)ethyl]pyrrolidine-3-carboxamide or a salt thereof.

**11.** N-[2-(4-{[4-(1,2-Benzoisothiazol-4-yloxy)-3-methylphenyl]amino}-5H-pyrrolo[3,2-d]pyrimidin-5-yl)ethyl]-2,2-dimethylpropanamide or a salt thereof.

**12.** N-[2-(4-{[4-(1,2-Benzoisothiazol-4-yloxy)-3-chlorophenyl]amino}-5H-pyrrolo[3,2-d]pyrimidin-5-yl)ethyl]-3-hydroxy-2,2-dimethylpropanamide or a salt thereof.

**13.** 1-[2-(4-{[4-(1,2-Benzoisothiazol-4-yloxy)-3-chlorophenyl]amino}-5H-pyrrolo[3,2-d]pyrimidin-5-yl)ethyl]-3-ethylurea or a salt thereof.

**14.** 1-[2-(4-{[4-(1,2-Benzoisothiazol-4-yloxy)-3-chlorophenyl]amino}-5H-pyrrolo[3,2-d]pyrimidin-5-yl)ethyl]-3-methylurea or a salt thereof.

**15.** N-[2-(4-{[4-(1,2-Benzoisothiazol-4-yloxy)-3-chlorophenyl]amino}-5H-pyrrolo[3,2-d]pyrimidin-5-yl)ethyl]-2-methylalaninamide or a salt thereof.

**16.** N-[2-(4-{[4-(1,2-Benzoisothiazol-4-yloxy)-3-chlorophenyl]amino}-5H-pyrrolo[3,2-d]pyrimidin-5-yl)ethyl]-$N^2$-tert-butylglycinamide or a salt thereof.

**17.** A prodrug of the compound of claim 1.

**18.** A pharmaceutical agent comprising the compound of claim 1 or a salt thereof, or a prodrug thereof.

**19.** The pharmaceutical agent of claim 18, which is a tyrosine kinase inhibitor.

**20.** The pharmaceutical agent of claim 18, which is an agent for the prophylaxis or treatment of cancer.

**21.** The pharmaceutical agent of claim 18, which is an agent for the prophylaxis or treatment of breast cancer, ovarian cancer, colorectal cancer, small intestinal cancer, gastric cancer, esophagus cancer, prostate cancer, lung cancer, pancreatic cancer or kidney cancer.

**22.** A method for the prophylaxis or treatment of cancer in a mammal, which comprises administering an effective amount of the compound of claim 1 or a salt thereof, or a prodrug thereof, to the mammal.

**23.** Use of the compound of claim 1 or a salt thereof, or a prodrug thereof, for the production of an agent for the prophylaxis or treatment of cancer.

**INTERNATIONAL SEARCH REPORT**

International application No.
PCT/JP2009/054603

A. CLASSIFICATION OF SUBJECT MATTER
*C07D487/04*(2006.01)i, *A61K31/519*(2006.01)i, *A61P9/00*(2006.01)i, *A61P9/10*(2006.01)i, *A61P31/12*(2006.01)i, *A61P31/18*(2006.01)i, *A61P35/00*(2006.01)i, *A61P35/02*(2006.01)i, *A61P43/00*(2006.01)i, *C07D487/16*(2006.01)i

According to International Patent Classification (IPC) or to both national classification and IPC

B. FIELDS SEARCHED

Minimum documentation searched (classification system followed by classification symbols)
C07D487/04, A61K31/519, A61P9/00, A61P9/10, A61P31/12, A61P31/18, A61P35/00, A61P35/02, A61P43/00, C07D487/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2005/118588 A1 (Takeda Chemical Industries, Ltd.), 15 December, 2005 (15.12.05), Examples; pages 97 to 98 & JP 2008-247907 A & US 2007/0244132 A1 & US 2009/0018335 A1 & US 2009/0029973 A1 & EP 1752457 A1 & KR 2007026695 A & CN 1993362 A | 1-21,23 |
| A | WO 2007/064045 A1 (TAKEDA PHARMACEUTICAL CO., LTD.), 07 June, 2007 (07.06.07), Full text & EP 1957495 A1 | 1-21,23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 April, 2009 (22.04.09) | 12 May, 2009 (12.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No. PCT/JP2009/054603

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2008/072634 A1 (Takeda Chemical Industries, Ltd.), 19 June, 2008 (19.06.08), Full text (Family: none) | 1-21,23 |

Form PCT/ISA/210 (continuation of second sheet) (April 2007)

**INTERNATIONAL SEARCH REPORT**

International application No.
PCT/JP2009/054603

**Box No. II Observations where certain claims were found unsearchable (Continuation of item 2 of first sheet)**

This international search report has not been established in respect of certain claims under Article 17(2)(a) for the following reasons:

1. [X] Claims Nos.: 22
because they relate to subject matter not required to be searched by this Authority, namely:
It pertains to methods for treatment of the human body by therapy and thus relates to a subject matter which this International Searching Authority is not required, under the provisions of Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9713771 A
- WO 9802437 A
- WO 0044728 A
- WO 0202552 A
- WO 0198277 A
- WO 03049740 A
- WO 03050108 A
- WO 03053446 A
- WO 9803648 A
- WO 0177107 A
- WO 03031442 A
- WO 2005118588 A
- WO 9640142 A
- WO 9823613 A
- JP 2008063363 A


### Non-patent literature cited in the description


- *Khim.-Farm. Zh.,* 1982, vol. 16, 1338-1343
- *Collect. Czech. Chem. Commun.,* 2003, vol. 68, 779-791
- *Khim. -Farm.Zh.,* 1982, vol. 16, 1338-1343
- IYAKUHIN no KAIHATSU. Design of Molecules. HIROKAWA SHOTEN, 1990, vol. 7, 163-198
- **Skehan et al.** *Journal of the National Cancer Institute,* 1990, vol. 82, 1107-1112

SEQUENCE LISTING

<110> Takeda Pharmaceutical Company Limited

<120> FUSED HETEROCYCLIC COMPOUND

<130> 091345

<150> JP2008-063363
<151> 2008-03-12

<160> 4

<170> PatentIn version 3.5

<210> 1
<211> 66
<212> DNA
<213> Artificial Sequence

<220>
<223> primer

<400> 1
aattaagtcg acatggacta caaagacgat gacgacaagc gacggcagca gaagatccgg 60

aagtac 66

<210> 2
<211> 42
<212> DNA
<213> Artificial Sequence

<220>
<223> primer

<400> 2
aattaagcat gctcacactg gcacgtccag acccaggtac tc 42

<210> 3
<211> 66
<212> DNA
<213> Artificial Sequence

<220>
<223> primer

<400> 3
aattaagtcg acatggacta caaagacgat gacgaccgaa ggcgccacat cgttcggaag 60

cgcacg 66

<210> 4
<211> 42
<212> DNA
<213> Artificial Sequence

<220>
<223> primer

<400> 4
aattaagcat gctcatgctc caataaattc actgctttgt gg 42